(19) Europäisches Patentamt
European Patent Office
Office européen des brevets

(11) EP 2 626 066 A1

(12) EUROPEAN PATENT APPLICATION

(43) Date of publication:
**14.08.2013 Bulletin 2013/33**

(21) Application number: **12154880.4**

(22) Date of filing: **10.02.2012**

(51) Int Cl.:
***A61K 31/16*** *(2006.01)*
***A61K 31/517*** *(2006.01)*
***A61P 35/00*** *(2006.01)*
***A61K 45/06*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Max-Planck-Gesellschaft zur Förderung der Wissenschaften e.V.
80539 München (DE)**

(72) Inventor: **The designation of the inventor has not yet been filed**

(74) Representative: **Vossius & Partner
Siebertstrasse 4
81675 München (DE)**

(54) **Combination therapy comprising selective VEGFR-2 inhibitors and MEK inhibitors**

(57) The present invention relates to a combination therapy comprising selective inhibitors of vascular endothelial growth factor receptor-2 (VEGFR-2) tyrosine kinase and inhibitors of MAP kinase kinase (MEK). Accordingly, a pharmaceutical composition is provided that comprises a selective inhibitor of vascular endothelial growth factor receptor-2 (VEGFR-2) tyrosine kinase or a pharmaceutically acceptable salt, solvate and/or hydrate thereof, and an inhibitor or a pharmaceutically acceptable salt, solvate and/or hydrate thereof, for use in medicine, especially in the treatment of cancer, like lung cancer, such as non-small cell lung cancer, lung adenocarcinoma or small cell lung cancer. Also a method for identifying patients that are particularly susceptible to such a combination therapy is subject of the present invention. The method comprises the evaluation of the expression level of VEGFR-2 and/or VEGF. An increase in said expression level indicates susceptibility to the combination therapy.

EP 2 626 066 A1

## Description

The present invention relates to a combination therapy comprising selective inhibitors of vascular endothelial growth factor receptor-2 (VEGFR-2) tyrosine kinase and inhibitors of MAP kinase kinase (MEK). Accordingly, a pharmaceutical composition is provided that comprises a selective inhibitor of vascular endothelial growth factor receptor-2 (VEGFR-2) tyrosine kinase or a pharmaceutically acceptable salt, solvate and/or hydrate thereof, and an inhibitor or a pharmaceutically acceptable salt, solvate and/or hydrate thereof, for use in medicine, especially in the treatment of cancer, like lung cancer, such as non-small cell lung cancer, lung adenocarcinoma or small cell lung cancer. Also a method for identifying patients that are particularly susceptible to such a combination therapy is subject of the present invention. The method comprises the evaluation of the expression level of VEGFR-2 and/or VEGF. An increase in said expression level indicates susceptibility to the combination therapy.

Several receptor tyrosine kinases have been implicated in tumor angiogenesis, such as VEGFR2, Flt1/VEGFR1, VEGFR3 or PDGFR. When activated through ligation of the respective ligand, they regulate related, yet distinct processes involved in neoangiogenesis. Endothelial cell migration and proliferation of blood vessels is primarily regulated by VEGFA binding to VEGFR2[1]. In the canonical model, tumor cells express VEGF binding to VEGFR2 on endothelial cells under hypoxic conditions[2], thus stimulating tumor vessel formation. This change from pre-vascular hyperplasia to highly vascularized tumors has been referred to as the angiogenic switch[1]. In recent years, several studies have reported expression of VEGFRI/ Flt1 as well as of VEGFR2 on tumor cells[3-5]. An intriguing finding related to tumor-cell expression of Flt1 was the observation that VEGF secreted by tumor cells triggers proliferation of tumor cells by binding Fltl[6]. A combination therapy comprising the non-selective VEGFR inhibitor AZD2171 and MEK inhibitor AZD6244 is disclosed in WO 2008/125820, US 2010/0130519 and Takahashi (2012) Clin Cancer Res, doi:10.1158/1078-0432.CCR-11-2324. These documents show an inhibition or slow down of tumor growth using the therein disclosed combination therapy, i.e. the tumor continues growing despite the therein disclosed combination therapy. In contrast thereto, it was found herein that the use of a selective inhibitor of VEGFR-2 tyrosine kinase combined with an inhibitor of MEK kinase results, unexpectedly, in a shrinkage of tumors, i.e. tumor growth is not merely slowed down or stopped but it is effectively reversed; see Example 2 and Figures 10 to 12.

Thus, the technical problem underlying the present invention is the provision of means and methods for effective medical intervention in cancer.

The technical problem is solved by provision of the embodiments characterized in the claims.

Accordingly, the present invention relates to a pharmaceutical composition comprising a selective inhibitor of vascular endothelial growth factor receptor-2 (VEGFR-2) tyrosine kinase or a pharmaceutically acceptable salt, solvate and/or hydrate thereof, and an inhibitor of MAP kinase kinase (MEK) or a pharmaceutically acceptable salt, solvate and/or hydrate thereof, for use in the treatment of cancer. The terms "inhibitor" and "antagonist" can be used interchangeably herein.

As documented in the appended examples the herein provided combination therapy using a selective inhibitor of VEGFR2 and a MEK inhibitor is highly effective in cancer treatmnt, as it leads to an unexpected and strong reduction of tumor volume. As shown in Example 2 and in Figure 10A, test animals ("H441" and "H1975") treated with the herein provided combination therapy using a selective inhibitor of VEGFR2 (Vandetanib) and a MEK inhibitor (PD0325901) showed a drastic tumor shrinkage: the tumor almost completely disappeared after 3 to 4 weeks of treatment (tumor volume after treatment compared to initial tumor volume at start of the treatment). In contrast, monotherapy with the selective VEGFR-2 inhibitor Vandetanib and the MEK inhibitor (PD0325901), respectively, did not reduce the tumor volume. This demonstrates the surprisingly high treatment efficacy of the herein provided combination therapy. The animal models ("H441" and "H1975") used in the combination therapy of Example 2 (the results thereof are shown in Figure 10A ) have a high VEGF and VEGFR2 expression level in the tumor, i.e. the tumor/cancer is characterized by a high VEGF and VEGFR2 expression level. In Figure 10B the results of combination therapy using a selective inhibitor of VEGFR2 (Vandetanib) and a MEK inhibitor (PD0325901) in a further animal model ("H 1650") are shown: this animal model is characterized by a very low VEGF and VEGFR2 expression level. While being as effective as monotherapy with the MEK inhibitor, the combination therapy did not result in a tumor shrinkage in this animal model.

Thus, it was found herein that the efficacy of the combination therapy depends on the expression level of VEGFR2/VEGF in the tumor; the combination therapy is particularly effective in a high VEGFR2/VEGF expressing setting, like in subject/patients having a high VEGFR2/VEGF expression (or, more precisely, patients/subjects having or suffering from tumors characterized by or associated with a high VEGFR2 and/or VEGF expression). Such a high expression is, for example, observed in solid cancer/solid tumors, like lung cancer (e.g. non-small cell lung cancer, small cell lung cancer or lung adenocarcinoma). For example, it is thought that the tumor shrinkage will be observed in subjects/ patients having a higher VEGFR2 and/or VEGF expression (e.g. at least 1.5 fold higher) compared to a control as defined herein (like the "H1650" control). Explanations and methods for determining whether the VEGFR2 and/or VEGF expression is high in a sample are described in detail herein further below. As noted above, the therapy using the non-selective VEGFR inhibitor AZD2171 and the MEK inhibitor AZD6244 disclosed in WO 2008/125820 did not show any reduction

in tumor volume: the tumor continued growing even though the growth was slowed down compared to the non-treated control. Therefore, the herein finding in the present invention was unexpected.

As used herein, the term "inhibitor of vascular endothelial growth factor receptor-2 tyrosine kinase" (or short "inhibitor of VEGFR-2 tyrosine kinase" or even shorter "inhibitor of VEGFR-2"; these terms are used interchangeably herein) refers to any compound capable of downregulating, decreasing, suppressing or otherwise regulating the amount and/or activity of the VEGFR-2 tyrosine kinase. The inhibitors to be used in accordance with the present invention are, however, selective inhibitors of VEGFR-2 tyrosine kinase; the term "selective" will be defined further below. Inhibition of this kinase can be achieved by any of a variety of mechanisms known in the art, including, but not limited to binding directly to the kinase polypeptide, denaturing or otherwise inactivating the kinase, or inhibiting the expression of the gene (e.g., inhibition of the transcription to mRNA, translation to a nascent polypeptide, and/or final polypeptide modifications to a mature protein), which encodes the kinase. Generally, the kinase inhibitors may be proteins, polypeptides, nucleic acids, small molecules, or other chemical moieties. As used herein the term "inhibiting" or "inhibition" refers to the ability of a compound to downregulate, decrease, reduce, suppress, inactivate, or inhibit at least partially the activity of the VEGFR-2 tyrosine kinase or the expression of said kinase. A "VEGFR-2 tyrosine kinase inhibitor" or "inhibitor of CDK9 VEGFR-2 tyrosine kinase" may, for example, interfere with transcription of a VEGFR-2 gene, processing (e.g. splicing, export from the nucleus and the like) of the gene product (e.g. unspliced or partially spliced mRNA) and/or translation of the gene product (e.g. mature mRNA). The VEGFR-2 tyrosine kinase inhibitor may also interfere with further modification (like phosphorylation) of the polypeptide/protein encoded by the VEGFR-2 gene and thus completely or partially inhibit the activity of the VEGFR-2 tyrosine kinase. Furthermore, the VEGFR-2 tyrosine kinase inhibitor may interfere with interactions of the VEGFR-2 tyrosine kinase with other proteins.

As mentioned, the prior art (see WO 2008/125820 or US 2010/0130519 or Takahashi, loc. cit.) has used the non-selective VEGFR inhibitor AZD2171. According to WO 2008/125820 the $IC_{50}$ values of AZD2171 for inhibition of isolated KDR (VEGFR-2), Flt-1 (VEGFR-1) and Flt-4 (VEGFR-3) tyrosine kinase activities in an enzyme assay were < 2 nM, 5 $\pm$2 nM and $\leq$ 3nM, respectively. This shows that AZD2171 inhibits KDR (VEGFR-2), F1t-1 (VEGFR-1) and Flt-4 (VEGFR-3) tyrosine kinases. In contrast, the VEGFR-2 inhibitors advantageously used in accordance with the present invention, selectively inhibit VEGFR-2, i.e. they primarily inhibit VEGFR-2 and do substantially not inhibit other proteins, in particular kinases like Fit-1 (VEGFR-1) and/or Flt-4 (VEGFR-3). In other words, the selective inhibitors show a stronger VEGFR-2 inhibition than VEGFR-1 and/or VEGFR-3 inhibition. Preferably, the selective inhibitors show at least a 3 fold, 4 fold, 5 fold, 6 fold, 7 fold, 8 fold, 9 fold, 10 fold, 15 fold, 20 fold, 25 fold, 30 fold,35 fold or 40 fold (or higher) stronger VEGFR-2 inhibition than VEGFR-1 inhibition, wherein the higher values are preferred. The selective inhibitors may show an up to 100 fold stronger VEGFR-2 inhibition than VEGFR-1 inhibition. The selective inhibitors may, in addition, or in the alternative, show at least a 2 fold, 2.1 fold, 2.2 fold, 2.3 fold, 2.4 fold, 2.5 fold, 2.6 fold or 2.7 fold (or higher) stronger VEGFR-2 inhibition than VEGFR-3 inhibition. The selective inhibitors may show an up to 5 fold stronger VEGFR-2 inhibition than VEGFR-3 inhibition. The $IC_{50}$ values of the exemplary selective VEGFR-2 inhibitor vandetanib (or Zactima or ZD6474) for inhibition of isolated KDR (VEGFR-2), Flt-1 (VEGFR-1) and Flt-4 (VEGFR-3) tyrosine kinase activities in an enzyme assay are 40 nM $\pm$ 10 nM, 1600 $\pm$ 400 nM and 110 nM $\pm$ 20 nM, respectively; see Wedge (2002) Cancer Res 62, 4645-4655 and Ryan (2005) British Journal of Cancer 92, S6-S 13.

Selectivity expresses the biologic fact that at a given compound concentration enzymes (or proteins) are affected to different degrees. In the case of enzymes selective inhibition can be defined as preferred inhibition by a compound at a given concentration. Or in other words, an enzyme is selectively inhibited over another enzyme when there is a concentration which results in inhibition of the first enzyme whereas the second enzyme is not affected. To compare compound effects on different enzymes it is crucial to employ similar assay formats, such as the Lance assay or the $^{33}$PanQinase® as described in more detail below. Appropriate assays are also described at page 80 to 83 of WO 00/47212 which is incorporated herein by reference in its entirety.

The Lance assay has been described for example in Moshinsky et al.; 2003 (A widely applicable, high-throughput TR-FRET assay for the measurement of kinase autophosphorylation: VEGFR-2 as a prototype. Moshinsky DJ, Ruslim L, Blake RA, Tang F. J Biomol Screen. 2003 Aug;8(4):447-52). The Lance Ultra KinaSelect Assay may be used to determine half maximal inhibitory concentration ($IC_{50}$) of inhibitor compounds and CDK/Cyclin complexes. The principle behind this enzymatic assay is based on the phosphorylation of the Ulight-Peptide Substrat. It is detected by using a specific EU-labeled anti-phospho peptide antibody. The binding of the Eu labeled anti-phospho peptide antibody to the phosphorylated ULight labeled peptide gives rise to a FRET-signal. Binding of an inhibitor to the kinase prevents phosphorylation of the Ulight-MBP Substrat, resulting in a loss of FRET. Based on these results, the IC50 value can be determined.

The Lance assay may be performed as follows:

Typically such experiments are started by generation of compounds which are serially diluted in multi titer plates in dimethylsulfoxide (DMSO). In the next step, working solutions for the enzymes, the substrates (protein and ATP separately) are generated in enzyme buffer. The preparation of the assay plate (definition of positive and negative control, reference inhibitors, test compounds and the pipetting of all solutions and compounds except the ATP

working solution) is done within the next step. Finally the reaction is started by the addition of the ATP working solution. All pipetting steps can be done manually or by the help of robotics. Within the incubation of 1 h at room temperature the enzyme catalyzes the generation of phosphorylated substrate. This reaction is more ore less inhibited by the added compounds. Finally, to stop the reaction and to detect phosphorylated substrate detection buffer (see material and methods) is added followed by another incubation of 1 h. The data is evaluated by measuring the FRET-Signal. Data is processed by subtraction of the backgroung signal (negative control) from all investigated activities. These activities are set into relation to the positive control. Altogether this is shown by the following equation:

$$\text{resulting activity (\%)} = 100 \text{ X } [(\text{signal of compound} - \text{signal of negative control}) / (\text{signal of positive control} - \text{signal of negative control})]$$

Further analysis steps include the determination of IC50 values by using the activities of a dose response experiment and an algorithm (equation #205 in Excel fit) for calculation.

A similar experimental procedure is performed when the resulting activity within $^{33}$PanQinase® assay is done. In advance buffers are prepared but in this case the pipetting sequence is first ATP solution diluted with assay buffer, DMSO or compound solution. The reaction (1h at 30°C) is started by addition of a substrate-kinase mix. During the incubation the kinase phosphorylates the substrate (different for each kinase). Due to the fact that the ATP solution contains $^{33}$P-labelled ATP the substrate proteins are labeled with $^{33}$P. The reaction is stopped by addition of excess $H_3PO_4$. If the reaction is performed in plates binding substrate proteins, said plates are washed to reduce unspecific signals (mainly not used ATP). The incorporation of $^{33}$P into substarte proteins is a direct measure of activity of the respective kinase. Therefore, the incorporated radioactivity is detected by scintillation counting. Data is evaluated, processed and analyzed as described for the LANCE assays.

It is preferred herein that the ratio of $IC_{50}$ values of selective VEGFR2-inhibitors determined according to an appropriate assay (e.g. the VEGFR2 Lance assay) and IC50 values of selective VEGFR2-inhibitors determined according to an appropriate comparative assay (e.g. the VEGFR Lance assay and/or other assays as described herein or known in the art) is about 1:10 or lower. More preferred is a ratio of below 1:15, 1:20, 1:30, 1:35 or 1:40, 1:50, 1:60, 1:70, 1:80, 1:90 or 1:100 or even lower.

In one aspectof the present invention, the selective inhibitor of VEGFR-2 tyrosine kinase inhibits in addition epidermal growth factor receptor (EGFR) tyrosine kinase. An exemplary inhibitor that inhibits selectively VEGFR-2 tyrosine kinase and EGFR tyrosine kinase is Vandetanib (this inhibitor is described herein further below in more detail).

The following exemplary selective inhibitors of VEGFR-2 tyrosine kinase may be used in accordance with the present invention:

N-(4-bromo-2-fluorophenyl)-6-methoxy-7-[(1-methylpiperidin-4-yl)methoxy]quinazolin-4-amine (Vandetanib/Zactima&ZD6474) as shown in formula (I) below or a pharmaceutically acceptable salt, solvate or hydrate thereof:

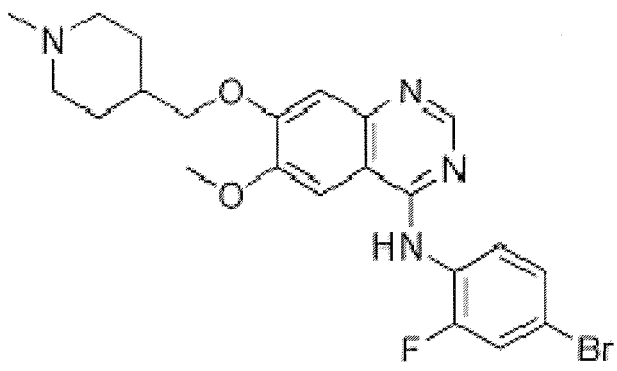

(I);

Cabozantinib (BMS-907351) as shown in formula (II) below or a pharmaceutically acceptable salt, solvate or hydrate thereof ($C_{28}H_{24}FN_3O_5$ (commercially available from Selleckchem, Houston, Tx 77054 USA)):

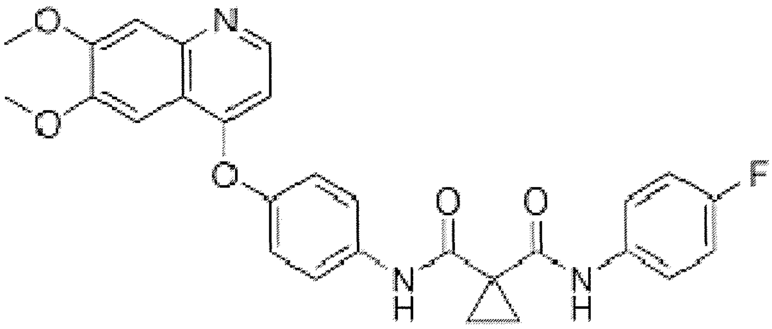

(II);

Apatinib (YN968D1) as shown in formula (III) below or a pharmaceutically acceptable salt, solvate or hydrate thereof:

$C_{25}H_{27}N_5O_4S$ (commercially available from Selleckchem, Houston, Tx 77054 USA)

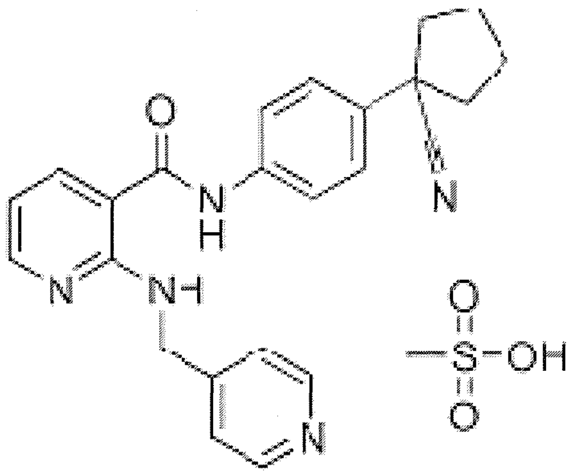

(III);

BMS 794833 MET/R2 as shown in formula (IV) below or a pharmaceutically acceptable salt, solvate or hydrate thereof ($C_{23}H_{15}ClF_2N_4O_3$ (commercially available from Selleckchem, Houston, Tx 77054 USA)):

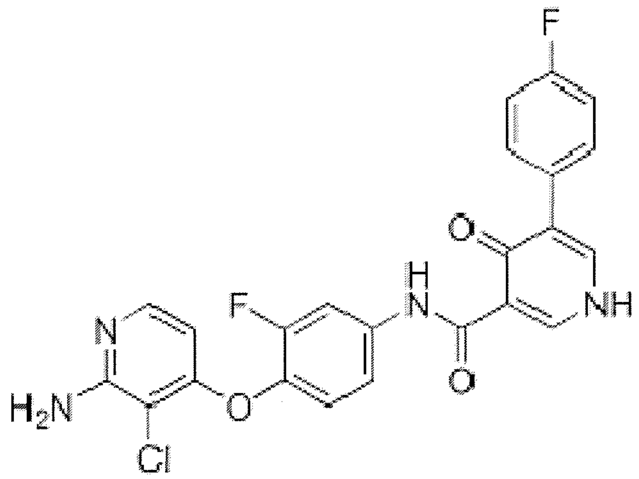

(IV);

Ki8751 as shown in formula (V) below or a pharmaceutically acceptable salt, solvate or hydrate thereof (commercially available from Selleckchem, Houston, Tx 77054 USA):

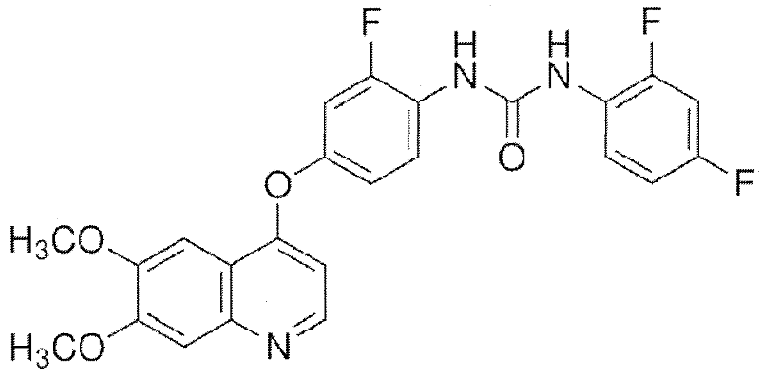

(V); or

OSI-930 as shown in formula (VI) below or a pharmaceutically acceptable salt, solvate or hydrate thereof ($C_{22}H_{16}F_3N_3O_2S$ (commercially available from Selleckchem, Houston, Tx 77054 USA)):

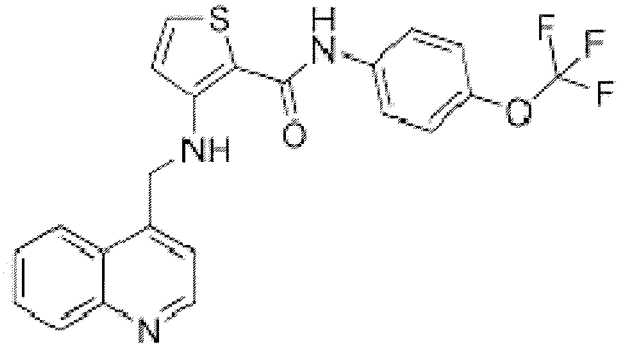

(VI).

The use of N-(4-bromo-2-fluorophenyl)-6-methoxy-7-[(1-methylpiperidin-4-yl)methoxy]quinazolin-4-amine (Vandetanib/Zactima/ZD6474) as shown in formula (I) or a pharmaceutically acceptable salt, solvate or hydrate thereof is preferred in the present invention.

The above compounds are commercially available (as mentioned above and below) and are well known in the art.

In accordance with the invention, a combination therapy is provided, wherein one or more of the herein above defined and described selective inhibitors of VEGFR2 tyrosine kinase is/are used in combination with one or more of MAP kinase kinase (MEK) inhibitors. MEK inhibitors are described in detail herein below. Generally, any kind of MEK inhibitor can be used herein; preferably, the MEK inhibitor inhibits MEK1 or MEK1 and MEK5; for example it may selectively inhibit MEK1 or it may inhibit MEK1 and, in addition, MEK5. One exemplary inhibitor to be used in this context is PD0325901.

The term "inhibitor of MAP kinase kinase" (or "MAP kinase kinase inhibitor") as used herein (or short "MEK inhibitor" or "inhibitor of MEK"; these terms are used interchangeably herein) refers to any compound capable of downregulating, decreasing, suppressing or otherwise regulating the amount and/or activity of a MAP kinase kinase, especially of MEK1/MEK2 and/or MEK5. Inhibition of this kinase can be achieved by any of a variety of mechanisms known in the art, including, but not limited to binding directly to the kinase polypeptide, denaturing or otherwise inactivating the kinase, or inhibiting the expression of the gene (e.g., inhibition of the transcription to mRNA, translation to a nascent polypeptide, and/or final polypeptide modifications to a mature protein), which encodes the kinase. Generally, the kinase inhibitors may be proteins, polypeptides, nucleic acids, small molecules, or other chemical moieties. As used herein the term "inhibiting" or "inhibition" refers to the ability of a compound to downregulate, decrease, reduce, suppress, inactivate, or inhibit at least partially the activity of the MEK or the expression of said kinase. A "MEK inhibitor" may, for example, interfere with transcription of a MEK gene, processing (e.g. splicing, export from the nucleus and the like) of the gene product (e.g. unspliced or partially spliced mRNA) and/or translation of the gene product (e.g. mature mRNA). The MEK inhibitor may also interfere with further modification (like phosphorylation) of the polypeptide/protein encoded by the MEK gene and thus completely or partially inhibit the activity of MEK. Furthermore, the MEK inhibitor may interfere with interactions of MEK with other proteins.

The following exemplary MEK inhibitors may be used in accordance with the present invention:

N-[(2R)-2,3-dihydroxypropoxy]-3,4-difluoro-2-[(2-fluoro-4-iodophenyl)amino]-benzamide (PD0325901) as shown in formula (VII) below or a pharmaceutically acceptable salt, solvate or hydrate thereof (commercially available from Selleckchem, Houston, Tx 77054 USA):

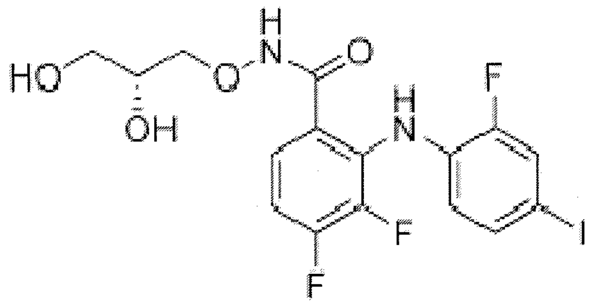

(VII);

AS703026 as shown in formula (VIII) below or a pharmaceutically acceptable salt, solvate or hydrate thereof (commercially available from Selleckchem, Houston, Tx 77054 USA)

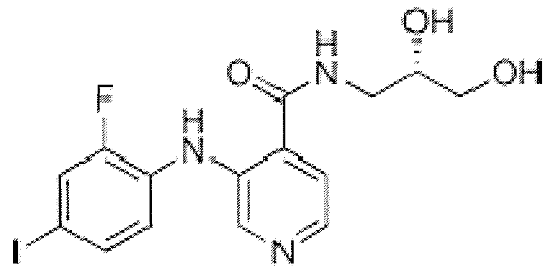

(VIII);

AZD6244 (Selumetinib) as shown in formula (IX) below or a pharmaceutically acceptable salt, solvate or hydrate thereof (commercially available from Selleckchem, Houston, Tx 77054 USA)

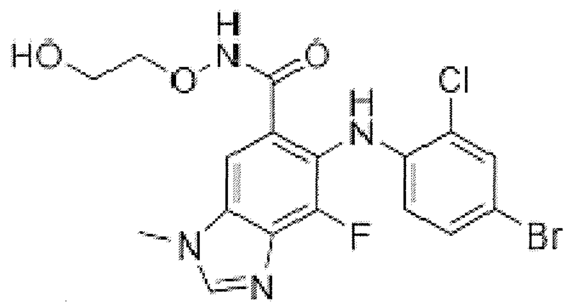

(IX);

AZD8330 as shown in formula (X) below or a pharmaceutically acceptable salt, solvate or hydrate thereof (commercially available from Selleckchem, Houston, Tx 77054 USA)

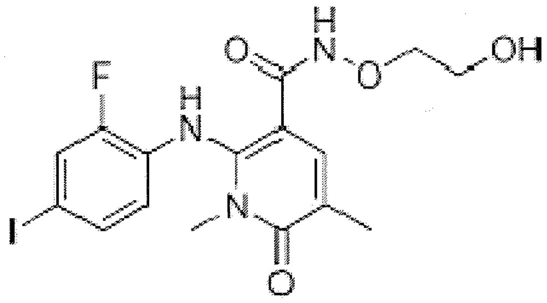

(X);

PD318088 as shown in formula (XI) below or a pharmaceutically acceptable salt, solvate or hydrate thereof (commercially available from Selleckchem, Houston, Tx 77054 USA),

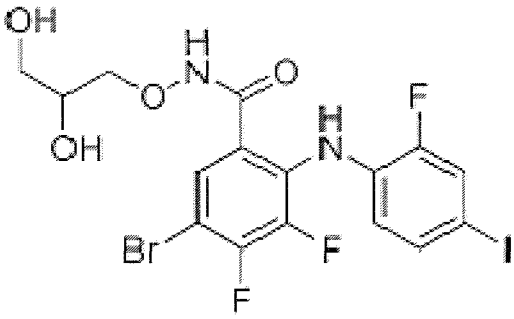

(XI);

PD98059 as shown in formula (XII) below or a pharmaceutically acceptable salt, solvate or hydrate thereof (commercially available from Selleckchem, Houston, Tx 77054 USA),

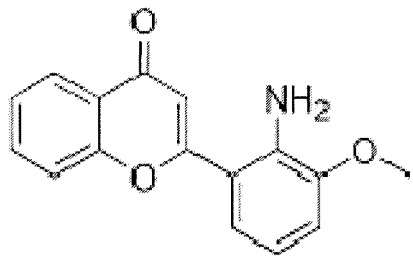

(XII);

TAK-733 as shown in formula (XIII) below or a pharmaceutically acceptable salt, solvate or hydrate thereof (commercially available from Selleckchem, Houston, Tx 77054 USA),

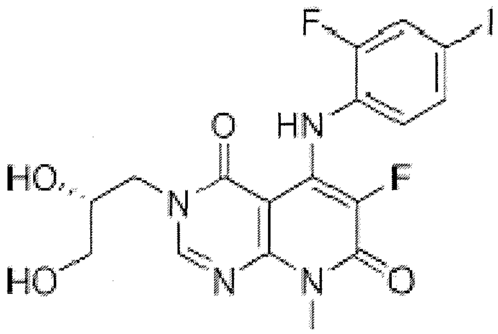

(XIII); or

U0126 as shown in formula (XIV) below or a pharmaceutically acceptable salt, solvate or hydrate thereof (commercially available from Selleckchem, Houston, Tx 77054 USA)

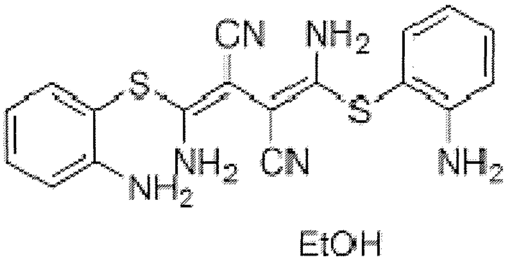

(XIV).

The above compounds are commercially available (see above and below), and are well known in the art.

Particularly preferred herein is the use of N-[(2R)-2,3-dihydroxypropoxy]-3,4-difluoro-2-[(2-fluoro-4-iodophenyl) amino]-benzamide (PD0325901) as shown in formula (VII) or a pharmaceutically acceptable salt, solvate or hydrate thereof. Very preferred is the use of PD0325901 as defined above in combination therapy with N-(4-bromo-2-fluorophenyl)-6-methoxy-7-[(1-methylpiperidin-4-yl)methoxy]quinazolin-4-amine (Vandetanib/Zactima/ZD6474) as shown in formula (I) or a pharmaceutically acceptable salt, solvate or hydrate thereof. Accordingly, in a very preferred embodiment of the present invention a composition is provided comprising or consisting of N-[(2R)-2,3-dihydroxypropoxy]-3,4-difluoro-2-[(2-fluoro-4-iodophenyl)amino]-benzamide (PD0325901) as shown in formula (VII) (or a pharmaceutically acceptable salt, solvate or hydrate thereof) and N-(4-bromo-2-fluorophenyl)-6-methoxy-7-[(1-methylpiperidin-4-yl)methoxy]quinazolin-4-amine (Vandetanib/Zactima/ZD6474) as shown in formula (I) (or a pharmaceutically acceptable salt, solvate or hydrate thereof). Said composition is preferably for use in the treatment of cancer, like lung cancer, such as non-small cell lung cancer, small cell lung cancer or lung adenocarcinoma. Preferably, said composition is a pharmaceutical composition optionally comprising pharmaceutically acceptable excipients and/or carriers.

As mentioned, the gist of the present invention lies in the provision of a combination therapy of one or more of the selective inhibitors of VEGFR-2 tyrosine kinase as defined herein (or a pharmaceutically acceptable salt, solvate and/or hydrate thereof) and one or more of the herein defined inhibitors of MEK (or a pharmaceutically acceptable salt, solvate and/or hydrate thereof). The term "combination therapy" as used herein refers in particular to the administration of a composition (preferably a pharmaceutical composition) to a subject or patient in need thereof, wherein the composition comprises one or more of the herein defined selective inhibitors of vascular endothelial growth factor receptor-2 (VEGFR-2) tyrosine kinase or a pharmaceutically acceptable salt, solvate and/or hydrate thereof, and one or more of the herein defined inhibitors of MAP kinase kinase (MEK) or a pharmaceutically acceptable salt, solvate and/or hydrate thereof. Such a "combination therapy" is preferably for use in the treatment of cancer. A particularly preferred "combination therapy" of the present invention is the administration of a composition (preferably a pharmaceutical composition) to a patient in need thereof, wherein the composition comprises or consists of N-[(2R)-2,3-dihydroxypropoxy]-3,4-difluoro-2-[(2-fluoro-4-iodophenyl)amino]-benzamide (PD0325901) as shown in formula (VII) (or a pharmaceutically acceptable salt, solvate or hydrate thereof) and N-(4-bromo-2-fluorophenyl)-6-methoxy-7-[(1-methylpiperidin-4-yl)methoxy]quinazolin-4-amine (Vandetanib/Zactima/ZD6474) as shown in formula (I) (or a pharmaceutically acceptable salt, solvate or hydrate thereof) and, optionally, pharmaceutically acceptable excipients and/or carriers.

In accordance with the above, compositions are provided comprising or consisting of one or more of the following exemplary selective inhibitors of vascular endothelial growth factor receptor-2 (VEGFR-2) tyrosine kinase or a pharmaceutically acceptable salt, solvate and/or hydrate thereof:

N-(4-bromo-2-fluorophenyl)-6-methoxy-7-[(1-methylpiperidin-4-yl)methoxy]quinazolin-4-amine (Vandetanib/Zactima&ZD6474) as shown in formula (I) or a pharmaceutically acceptable salt, solvate or hydrate thereof;
Cabozantinib (BMS-907351) as shown in formula (II) or a pharmaceutically acceptable salt, solvate or hydrate thereof;
Apatinib (YN968D1) as shown in formula (III) or a pharmaceutically acceptable salt, solvate or hydrate thereof;
BMS 794833 as shown in formula (IV) or a pharmaceutically acceptable salt, solvate or hydrate thereof;
Ki8751 as shown in formula (V) or a pharmaceutically acceptable salt, solvate or hydrate thereof; and/or
OSI-930 as shown in formula (VI) or a pharmaceutically acceptable salt, solvate or hydrate thereof;

and one or more of the following inhibitors of MAP kinase kinase (MEK) or a pharmaceutically acceptable salt, solvate and/or hydrate thereof:

N-[(2R)-2,3-dihydroxypropoxy]-3,4-difluoro-2-[(2-fluoro-4-iodophenyl)amino]-benzamide (PD0325901) as shown in formula (VII) or a pharmaceutically acceptable salt, solvate or hydrate thereof;
AS703026 as shown in formula (VIII) or a pharmaceutically acceptable salt, solvate or hydrate thereof;
AZD6244 (Selumetinib) as shown in formula (IX) or a pharmaceutically acceptable salt, solvate or hydrate thereof;
AZD8330 as shown in formula (X) or a pharmaceutically acceptable salt, solvate or hydrate thereof;
PD318088 as shown in formula (XI) or a pharmaceutically acceptable salt, solvate or hydrate thereof;
PD98059 as shown in formula (XII) or a pharmaceutically acceptable salt, solvate or hydrate thereof;
TAK-733 as shown in formula (XIII) or a pharmaceutically acceptable salt, solvate or hydrate thereof; and/or
U0126 as shown in formula (XIV) or a pharmaceutically acceptable salt, solvate or hydrate thereof.

Preferably, these and other compositions provided herein are for use in medicine. More preferably, the compositions are pharmaceutical composition, optionally comprising pharmaceutically acceptable excipients and/or carriers. Even more preferably, the pharmaceutical compositions are for use in the treatment of cancer, like lung cancer, such as non-small cell lung cancer, small cell lung cancer or lung adenocarcinoma.

Exemplary, non-limiting compositions to be used in accordance with the present invention are, accordingly, compositions comprising N-(4-bromo-2-fluorophenyl)-6-methoxy-7-[(1-methylpiperidin-4-yl)methoxy]quinazolin-4-amine (Vandetanib/Zactima&ZD6474) as shown in formula (1) or a pharmaceutically acceptable salt, solvate or hydrate thereof and one of N-[(2R)-2,3-dihydroxypropoxy]-3,4-difluoro-2-[(2-fluoro-4-iodophenyl)amino]-benzamide (PD0325901) as shown in formula (VII) or a pharmaceutically acceptable salt, solvate or hydrate thereof; AS703026 as shown in formula (VIII) or a pharmaceutically acceptable salt, solvate or hydrate thereof, AZD6244 (Selumetinib) as shown in formula (IX) below or a pharmaceutically acceptable salt, solvate or hydrate thereof, AZD8330 as shown in formula (X) below or a pharmaceutically acceptable salt, solvate or hydrate thereof, PD318088 as shown in formula (XI) below or a pharmaceutically acceptable salt, solvate or hydrate thereof, PD98059 as shown in formula (XII) below or a pharmaceutically acceptable salt, solvate or hydrate thereof, TAK-733 as shown in formula (XIII) below or a pharmaceutically acceptable salt, solvate or hydrate thereof or U0126 as shown in formula (XIV) below or a pharmaceutically acceptable salt, solvate or hydrate thereof.

Other compositions comprise Cabozantinib (BMS-907351) as shown in formula (II) or a pharmaceutically acceptable salt, solvate or hydrate thereof and one of N-[(2R)-2,3-dihydroxypropoxy]-3,4-difluoro-2-[(2-fluoro-4-iodophenyl) amino]-benzamide (PD0325901) as shown in formula (VII) or a pharmaceutically acceptable salt, solvate or hydrate thereof; AS703026 as shown in formula (VIII) or a pharmaceutically acceptable salt, solvate or hydrate thereof, AZD6244 (Selumetinib) as shown in formula (IX) below or a pharmaceutically acceptable salt, solvate or hydrate thereof, AZD8330 as shown in formula (X) below or a pharmaceutically acceptable salt, solvate or hydrate thereof, PD318088 as shown in formula (XI) below or a pharmaceutically acceptable salt, solvate or hydrate thereof, PD98059 as shown in formula (XII) below or a pharmaceutically acceptable salt, solvate or hydrate thereof, TAK-733 as shown in formula (XIII) below or a pharmaceutically acceptable salt, solvate or hydrate thereof or U0126 as shown in formula (XIV) below or a pharmaceutically acceptable salt, solvate or hydrate thereof.

Other compositions comprise Apatinib (YN968D1) as shown in formula (III) or a pharmaceutically acceptable salt, solvate or hydrate thereof and one of N-[(2R)-2,3-dihydroxypropoxy]-3,4-difluoro-2-[(2-fluoro-4-iodophenyl)amino]-benzamide (PD0325901) as shown in formula (VII) or a pharmaceutically acceptable salt, solvate or hydrate thereof; AS703026 as shown in formula (VIII) or a pharmaceutically acceptable salt, solvate or hydrate thereof, AZD6244 (Selumetinib) as shown in formula (IX) below or a pharmaceutically acceptable salt, solvate or hydrate thereof, AZD8330

as shown in formula (X) below or a pharmaceutically acceptable salt, solvate or hydrate thereof, PD318088 as shown in formula (XI) below or a pharmaceutically acceptable salt, solvate or hydrate thereof, PD98059 as shown in formula (XII) below or a pharmaceutically acceptable salt, solvate or hydrate thereof, TAK-733 as shown in formula (XIII) below or a pharmaceutically acceptable salt, solvate or hydrate thereof or U0126 as shown in formula (XIV) below or a pharmaceutically acceptable salt, solvate or hydrate thereof.

Other compositions comprise BMS 794833 as shown in formula (IV) or a pharmaceutically acceptable salt, solvate or hydrate thereof and one of N-[(2R)-2,3-dihydroxypropoxy]-3,4-difluoro-2-[(2-fluoro-4-iodophenyl)amino]-benzamide (PD0325901) as shown in formula (VII) or a pharmaceutically acceptable salt, solvate or hydrate thereof; AS703026 as shown in formula (VIII) or a pharmaceutically acceptable salt, solvate or hydrate thereof, AZD6244 (Selumetinib) as shown in formula (IX) below or a pharmaceutically acceptable salt, solvate or hydrate thereof, AZD8330 as shown in formula (X) below or a pharmaceutically acceptable salt, solvate or hydrate thereof, PD318088 as shown in formula (XI) below or a pharmaceutically acceptable salt, solvate or hydrate thereof, PD98059 as shown in formula (XII) below or a pharmaceutically acceptable salt, solvate or hydrate thereof, TAK-733 as shown in formula (XIII) below or a pharmaceutically acceptable salt, solvate or hydrate thereof or U0126 as shown in formula (XIV) below or a pharmaceutically acceptable salt, solvate or hydrate thereof.

Other compositions comprise Ki8751 as shown in formula (V) or a pharmaceutically acceptable salt, solvate or hydrate thereof and one of N-[(2R)-2,3-dihydroxypropoxy]-3,4-difluoro-2-[(2-fluoro-4-iodophenyl)amino]-benzamide (PD0325901) as shown in formula (VII) or a pharmaceutically acceptable salt, solvate or hydrate thereof; AS703026 as shown in formula (VIII) or a pharmaceutically acceptable salt, solvate or hydrate thereof, AZD6244 (Selumetinib) as shown in formula (IX) below or a pharmaceutically acceptable salt, solvate or hydrate thereof, AZD8330 as shown in formula (X) below or a pharmaceutically acceptable salt, solvate or hydrate thereof, PD318088 as shown in formula (XI) below or a pharmaceutically acceptable salt, solvate or hydrate thereof, PD98059 as shown in formula (XII) below or a pharmaceutically acceptable salt, solvate or hydrate thereof, TAK-733 as shown in formula (XIII) below or a pharmaceutically acceptable salt, solvate or hydrate thereof or U0126 as shown in formula (XIV) below or a pharmaceutically acceptable salt, solvate or hydrate thereof.

Other compositions comprise OSI-930 as shown in formula (VI) or a pharmaceutically acceptable salt, solvate or hydrate thereof and one of N-[(2R)-2,3-dihydroxypropoxy]-3,4-difluoro-2-[(2-fluoro-4-iodophenyl)amino]-benzanide (PD0325901) as shown in formula (VII) or a pharmaceutically acceptable salt, solvate or hydrate thereof; AS703026 as shown in formula (VIII) or a pharmaceutically acceptable salt, solvate or hydrate thereof, AZD6244 (Selumetinib) as shown in formula (IX) below or a pharmaceutically acceptable salt, solvate or hydrate thereof, AZD8330 as shown in formula (X) below or a pharmaceutically acceptable salt, solvate or hydrate thereof, PD318088 as shown in formula (XI) below or a pharmaceutically acceptable salt, solvate or hydrate thereof, PD98059 as shown in formula (XII) below or a pharmaceutically acceptable salt, solvate or hydrate thereof, TAK-733 as shown in formula (XIII) below or a pharmaceutically acceptable salt, solvate or hydrate thereof or U0126 as shown in formula (XIV) below or a pharmaceutically acceptable salt, solvate or hydrate thereof.

Other compositions comprise N-[(2R)-2,3-dihydroxypropoxy]-3,4-difluoro-2-[(2-fluoro-4-iodophenyl)amino]-benzamide (PD0325901) as shown in formula (VII) or a pharmaceutically acceptable salt, solvate or hydrate thereof and one of N-(4-bromo-2-fluorophenyl)-6-methoxy-7-[(1-methylpiperidin-4-yl)methoxy]quinazolin-4-amine (Vandetanib/Zactima&ZD6474) as shown in formula (I) or a pharmaceutically acceptable salt, solvate or hydrate thereof, Cabozantinib (BMS-907351) as shown in formula (II) or a pharmaceutically acceptable salt, solvate or hydrate thereof, Apatinib (YN968D1) as shown in formula (III) or a pharmaceutically acceptable salt, solvate or hydrate thereof, BMS 794833 as shown in formula (IV) or a pharmaceutically acceptable salt, solvate or hydrate thereof, Ki8751 as shown in formula (V) or a pharmaceutically acceptable salt, solvate or hydrate thereof; or OSI-930 as shown in formula (VI) or a pharmaceutically acceptable salt, solvate or hydrate thereof.

Other compositions comprise AS703026 as shown in formula (VIII) or a pharmaceutically acceptable salt, solvate or hydrate thereof and and one of N-(4-bromo-2-fluorophenyl)-6-methoxy-7-[(1-methylpiperidin-4-yl)methoxy]quinazolin-4-amine (Vandetanib/Zactima&ZD6474) as shown in formula (I) or a pharmaceutically acceptable salt, solvate or hydrate thereof, Cabozantinib (BMS-907351) as shown in formula (II) or a pharmaceutically acceptable salt, solvate or hydrate thereof, Apatinib (YN968D1) as shown in formula (III) or a pharmaceutically acceptable salt, solvate or hydrate thereof, BMS 794833 as shown in formula (IV) or a pharmaceutically acceptable salt, solvate or hydrate thereof, Ki8751 as shown in formula (V) or a pharmaceutically acceptable salt, solvate or hydrate thereof; or OSI-930 as shown in formula (VI) or a pharmaceutically acceptable salt, solvate or hydrate thereof.

Other compositions comprise AZD6244 (Selumetinib) as shown in formula (IX) below or a pharmaceutically acceptable salt, solvate or hydrate thereof and one of N-(4-bromo-2-fluorophenyl)-6-methoxy-7-[(1-methylpiperidin-4-yl)methoxy]quinazolin-4-amine (Vandetanib/Zactima&ZD6474) as shown in formula (I) or a pharmaceutically acceptable salt, solvate or hydrate thereof, Cabozantinib (BMS-907351) as shown in formula (II) or a pharmaceutically acceptable salt, solvate or hydrate thereof, Apatinib (YN968D1) as shown in formula (III) or a pharmaceutically acceptable salt, solvate or hydrate thereof, BMS 794833 as shown in formula (IV) or a pharmaceutically acceptable salt, solvate or

hydrate thereof, Ki8751 as shown in formula (V) or a pharmaceutically acceptable salt, solvate or hydrate thereof; or OSI-930 as shown in formula (VI) or a pharmaceutically acceptable salt, solvate or hydrate thereof.

Other compositions comprise AZD8330 as shown in formula (X) below or a pharmaceutically acceptable salt, solvate or hydrate thereof and one of N-(4-bromo-2-fluorophenyl)-6-methoxy-7-[(1-methylpiperidin-4-yl)methoxy]quinazolin-4-amine (Vandetanib/Zactima&ZD6474) as shown in formula (I) or a pharmaceutically acceptable salt, solvate or hydrate thereof, Cabozantinib (BMS-907351) as shown in formula (II) or a pharmaceutically acceptable salt, solvate or hydrate thereof, Apatinib (YN968D1) as shown in formula (III) or a pharmaceutically acceptable salt, solvate or hydrate thereof, BMS 794833 as shown in formula (IV) or a pharmaceutically acceptable salt, solvate or hydrate thereof, Ki8751 as shown in formula (V) or a pharmaceutically acceptable salt, solvate or hydrate thereof; or OSI-930 as shown in formula (VI) or a pharmaceutically acceptable salt, solvate or hydrate thereof.

Other compositions comprise PD318088 as shown in formula (XI) below or a pharmaceutically acceptable salt, solvate or hydrate thereof and one of N-(4-bromo-2-fluorophenyl)-6-methoxy-7-[(1-methylpiperidin-4-y1)methoxy]quinazolin-4-amine (Vandetanib/Zactima&ZD6474) as shown in formula (I) or a pharmaceutically acceptable salt, solvate or hydrate thereof, Cabozantinib (BMS-907351) as shown in formula (II) or a pharmaceutically acceptable salt, solvate or hydrate thereof, Apatinib (YN968D1) as shown in formula (III) or a pharmaceutically acceptable salt, solvate or hydrate thereof, BMS 794833 as shown in formula (IV) or a pharmaceutically acceptable salt, solvate or hydrate thereof, Ki8751 as shown in formula (V) or a pharmaceutically acceptable salt, solvate or hydrate thereof; or OSI-930 as shown in formula (VI) or a pharmaceutically acceptable salt, solvate or hydrate thereof.

Other compositions comprise PD98059 as shown in formula (XII) below or a pharmaceutically acceptable salt, solvate or hydrate thereof and one of N-(4-bromo-2-fluorophenyl)-6-methoxy-7-[(1-methylpiperidin-4-yl)methoxy]quinazolin-4-amine (Vandetanib/Zactima&ZD6474) as shown in formula (I) or a pharmaceutically acceptable salt, solvate or hydrate thereof, Cabozantinib (BMS-907351) as shown in formula (II) or a pharmaceutically acceptable salt, solvate or hydrate thereof, Apatinib (YN968D1) as shown in formula (III) or a pharmaceutically acceptable salt, solvate or hydrate thereof, BMS 794833 as shown in formula (IV) or a pharmaceutically acceptable salt, solvate or hydrate thereof, Ki8751 as shown in formula (V) or a pharmaceutically acceptable salt, solvate or hydrate thereof; or OSI-930 as shown in formula (VI) or a pharmaceutically acceptable salt, solvate or hydrate thereof.

Other compositions comprise TAK-733 as shown in formula (XIII) below or a pharmaceutically acceptable salt, solvate or hydrate thereof and one of N-(4-bromo-2-fluorophenyl)-6-methoxy-7-[(1-methylpiperidin-4-yl)methoxy]quinazolin-4-amine (Vandetanib/Zactima&ZD6474) as shown in formula (I) or a pharmaceutically acceptable salt, solvate or hydrate thereof, Cabozantinib (BMS-907351) as shown in formula (II) or a pharmaceutically acceptable salt, solvate or hydrate thereof, Apatinib (YN968D1) as shown in formula (III) or a pharmaceutically acceptable salt, solvate or hydrate thereof, BMS 794833 as shown in formula (IV) or a pharmaceutically acceptable salt, solvate or hydrate thereof, Ki8751 as shown in formula (V) or a pharmaceutically acceptable salt, solvate or hydrate thereof; or OSI-930 as shown in formula (VI) or a pharmaceutically acceptable salt, solvate or hydrate thereof.

Other compositions comprise U0126 as shown in formula (XIV) below or a pharmaceutically acceptable salt, solvate or hydrate thereof. and one of N-(4-bromo-2-fluorophenyl)-6-methoxy-7-[(1-methylpiperidin-4-yl)methoxy]quinazolin-4-amine (Vandetanib/Zactima&ZD6474) as shown in formula (I) or a pharmaceutically acceptable salt, solvate or hydrate thereof, Cabozantinib (BMS-907351) as shown in formula (II) or a pharmaceutically acceptable salt, solvate or hydrate thereof, Apatinib (YN968D1) as shown in formula (III) or a pharmaceutically acceptable salt, solvate or hydrate thereof, BMS 794833 as shown in formula (IV) or a pharmaceutically acceptable salt, solvate or hydrate thereof, Ki8751 as shown in formula (V) or a pharmaceutically acceptable salt, solvate or hydrate thereof; or OSI-930 as shown in formula (VI) or a pharmaceutically acceptable salt, solvate or hydrate thereof.

In an alternative aspect, the present invention relates to the use of a selective inhibitor of vascular endothelial growth factor receptor-2 (VEGFR-2) tyrosine kinase or a pharmaceutically acceptable salt, solvate and/or hydrate thereof, and of an inhibitor of MAP kinase kinase (MEK) or a pharmaceutically acceptable salt, solvate and/or hydrate thereof, for the manufacture of a pharmaceutical composition for the treatment of cancer. In a further aspect, the present invention relates to a method for treating cancer comprising administering an effective amount of a selective inhibitor of vascular endothelial growth factor receptor-2 (VEGFR-2) tyrosine kinase or a pharmaceutically acceptable salt, solvate and/or hydrate thereof, and an inhibitor of MAP kinase kinase (MEK) or a pharmaceutically acceptable salt, solvate and/or hydrate thereof to a subject in need thereof. Preferably, the subject is a human. All definitions and explanations given herein above in context of combination therapy and corresponding compositions (in particular pharmaceutical compositions, especially those of use in the treatment of cancer) apply, mutatis mutandis, in this context.

Preferably, the cancer to be treated is characterized by or associated with VEGF and/or VEGFR2 expression, in particular VEGF and/or VEGFR2 overexpression (i.e. the expression level of VEGF and/or VEGFR2 in a tumor/cancer sample is (preferably statistically significantly higher) compared to a control sample, such as a "non-responder" or "non susceptible" control (e.g. cell line H1650 as used in Example 2). As explained above, it has been found that subjects/ individuals/patients suffering from a tumor/cancer which is characterized by or associated with VEGF and/or VEGFR2 expression are highly responsive/susceptible to the herein provided combination therapy. In these subjects, the combination therapy shows a more than additive effect, especially a reduction in the tumor volume, compared with the respective monotherapies of only a selective inhibitor of VEGFR-2 tyrosine kinase as defined herein or of only an inhibitor of MAP kinase kinase (MEK). Preferably, the expression level of VEGF and/or VEGFR2 is at least 1.1-fold, 1.2-fold, 1.3-fold, 1.4-fold, more preferably at least 1.5-fold, 1.6 -fold, 1.7 -fold, 1.8-fold, 1.9-fold, 2.0-fold, 2.1-fold, 2.2-fold, 2.3-fold, 2.4-fold, even more preferably at least 2.5-fold, 2.6-fold, 2.7-fold 2.8-fold, 2.9-fold, at least 3.0-fold, 3.1-fold, 3.2-fold, 3.3-fold, 3.4-fold, 3.5-fold, 4.0-fold, and yet more preferably at least 5-fold, 5.5-fold, 6-fold, or even more, like 7-fold, 8-fold, 9-fold, 10-fold, 11-fold, 12-fold, 13-fold, 14-fold, 15-fold or more increased in comparison to the control.

As used in context of the methods of the present invention, a non-limiting example of a "control" is preferably a "non-responder" or "non susceptible" control, for example a sample/cell/tissue obtained from one or more healthy subjects or one or more patients that suffer from a cancer/tumor and are known to be not responsive to cancer treatment, particularly to the herein described one or more selective inhibitors of VEGFR-2 tyrosine kinase and the one ore more inhibitors of MAP kinase kinase (MEK) (combination therapy). Another example for a "non-responder" control is a cell line/sample/cell/tissue that shows no response to cancer treatment, particularly to the herein described one or more selective inhibitors of VEGFR-2 tyrosine kinase as defined herein and the one ore more inhibitors of MAP kinase kinase (MEK) (combination therapy) in an ex-vivo test. One example for such a "non-responder" control is cell line H1650 as used in Example 2 and shown in Fig. 10. Another non-limiting example of a "control" is an "internal standard", for example a mixture of purified or synthetically produced proteins and/or peptides, where the amounts of each protein/peptide is gauged by using the "non-responder"/ "non susceptible" control described above. In particular, this mixture can contain the the VEGFR2 and/or VEGF protein as desribed and defined herein.

A further non-limiting example of a "control" may be a "healthy" control, for example a sample/cell/tissue obtained from a healthy subject or patient that is not suffering from a cancer/tumor or a cell obtained from such a subject. In accordance with the above, the reference or control status e.g. of VEGFR2 and/or VEGF is that determined in (a sample of) the corresponding healthy control subject/patient, i.e. it is the "normal" status of e.g. VEGFR2 and/or VEGF. The control may also be a sample/cell/tissue obtained from the individual or patient suspected of suffering from the cancer provided that the sample/cell/tissue does not contain tumor or cancer cells. In a further alternative, the "control" may be a sample/cell/tissue obtained from an individual or patient suffering from the cancer, that has been obtained prior to the development or diagnosis of said cancer.

Exemplary tumors which are characterized by or associated with VEGF and/or VEGFR2 expression as described above, are solid cancers / solid tumors, like lung cancer, such as non-small cell lung cancer, lung adenocarcinoma or small cell lung cancer, which are preferably to be treated in accordance with the present invention..

Other solid cancers/tumors to be treated may be cancers/tumors that are characterized by or associated with VEGF and/or VEGFR2 expression as described above and that express for example, VEGFR2 and/or VEGF on tumor cells, such as tyroid cancer (such as medullary thyroid cancer), pancreatic cancer, colorectal cancer, colon cancer, rectum cancer, breast cancer, head and neck cancer, ovarian cancer, endometrial cancer, gastrointestinal cancer (including gastric and esophageal cancer), renal cell cancer, urinary tract carcinomas, prostate cancer, lymphomas, melanomas, brain tumors/brain cancer, pediatric tumors and sarcomas, esophagus cancer, stomach cancer, liver cancer, biliary tract cancer, kidney cancer, cervix cancer, overy cancer, uterus cancer, skin cancer (including melanoma, uveal melanoma, and squamous cell carcinoma), gallbladder cancer, pancreas cancer, genital cancer (including vaginal carcinoma and penile carcinoma), embryonal carcinomas, hepatocellular carcinoma, neuroendocrine carcinomas, and/or cancer of unknown primary (CUP syndrome). The cancer to be treated may also include haematological malignancies such as leukemias, multiple myeloma and/or lymphoma.

The inhibitors may be administered by any one of oral route, parenteral route, intravenous route, subcutaneous route, intranasal route or transdermal route. Preferably, the inhibitors are to be administered in a neoadjuvant or adjuvant setting.

The terms "treatment", "treating" and the like are used herein to generally mean obtaining a desired pharmacological and/or physiological effect. The effect may be prophylactic in terms of completely or partially preventing a disease or symptom thereof and/or may be therapeutic in terms of partially or completely curing a disease and/or adverse effect attributed to the disease. The term "treatment" as used herein covers any treatment of a disease in a subject and includes: (a) preventing a disease related in a subject which may be predisposed to the disease; (b) inhibiting the disease, i.e. arresting its development; or (c) relieving the disease, i.e. causing regression of the disease.

A "patient" or "subject" for the purposes of the present invention includes both humans and other animals, particularly mammals, and other organisms. Thus, the methods are applicable to both human therapy and veterinary applications. Preferably, the patient is a mammal, and in the most preferably the patient is human.

The pharmaceutical composition will be formulated and dosed in a fashion consistent with good medical practice, taking into account the clinical condition of the individual patient, the site of delivery of the pharmaceutical composition, the method of administration, the scheduling of administration, and other factors known to practitioners. The "effective amount" of the pharmaceutical composition for purposes herein is thus determined by such considerations.

The skilled person knows that the effective amount of one of the herein described inhibitors in a pharmaceutical

composition administered to an individual will, inter alia, depend on the nature of the compound. For example, if said inhibitor is a selective VEGFR2-inhibitor, such as Vandetanib, the total (pharmaceutically) effective amount of the inhibitor in the pharmaceutical composition administered orally per dose will be in the range of about 50 mg inhibitor per day to 600 mg inhibitor per day of patient, although, as noted above, this will be subject to therapeutic discretion. More preferably, this dose is at least 50 mg inhibitor per day, and most preferably for humans between about 50mg and 300 mg protein per day. If given continuously, the inhibitor is typically administered at a dose rate of about 50 mg per day to about 300 mg per day. An intravenous bag solution may also be employed. The length of treatment needed to observe changes and the interval following treatment for responses to occur appears to vary depending on the desired effect. The particular amounts may be determined by conventional tests which are well known to the person skilled in the art.
For example, if said inhibitor is a MEK- inhibitor, such as PD0325901, the total (pharmaceutically) effective amount of the inhibitor in the pharmaceutical composition administered orally] per dose will be in the range of about 1 mg inhibitor twice daily (BID) to 30 mg inhibitor BID, although, as noted above, this will be subject to therapeutic discretion. More preferably, this dose is at least 2 mg inhibitor BID, and most preferably for humans between about 2mg to 15mg BID. If given continuously, the inhibitor is typically administered at a dose rate of about 2 mg BID to about 15 mg BID. An intravenous bag solution may also be employed. The length of treatment needed to observe changes and the interval following treatment for responses to occur appears to vary depending on the desired effect. The particular amounts may be determined by conventional tests which are well known to the person skilled in the art.

Preferably, the herein employed MEK inhibitors are to be administered twice daily (bis in die/BID), for example once in the morning (e.g. 8 a.m.) and once in the evening (e.g. 8 p.m.). Pharmaceutical compositions of the invention may be administered orally, rectally, parenterally, intracisternally, intravaginally, intraperitoneally, topically (as by powders, ointments, drops or transdermal patch), bucally, or as an oral or nasal spray. Pharmaceutical compositions of the invention preferably comprise a pharmaceutically acceptable carrier. By "pharmaceutically acceptable carrier" is meant a non-toxic solid, semisolid or liquid filler, diluent, encapsulating material or formulation auxiliary of any type. The term "parenteral" as used herein refers to modes of administration which include intravenous, intramuscular, intraperitoneal, intrasternal, subcutaneous and intraarticular injection and infusion. The administration of the herein provided compositions may, inter alia, comprise an administration twice daily, every day, every other day, every third day, every forth day, every fifth day, once a week, once every second week, once every third week, once every month, etc.

The pharmaceutical composition is also suitably administered by sustained release systems. Suitable examples of sustained-release compositions include semi-permeable polymer matrices in the form of shaped articles, e.g., films, or mirocapsules. Sustained-release matrices include polylactides (U.S. Pat. No. 3,773,919, EP 58,481), copolymers of L-glutamic acid and gamma-ethyl-L-glutamate (Sidman, U. et al., Biopolymers 22:547-556 (1983)), poly (2-hydroxyethyl methacrylate) (R. Langer et al., J. Biomed. Mater. Res. 15:167-277 (1981), and R. Langer, Chem. Tech. 12:98-105 (1982)), ethylene vinyl acetate (R. Langer et al., Id.) or poly-D-(-)-3-hydroxybutyric acid (EP 133,988). Sustained release pharmaceutical composition also include liposomally entrapped compound. Liposomes containing the pharmaceutical composition are prepared by methods known per se: DE 3,218,121; Epstein et al., Proc. Natl. Acad. Sci. (USA) 82: 3688-3692 (1985); Hwang et al., Proc. Natl. Acad. Sci. (USA) 77:4030-4034 (1980); EP 52,322; EP 36,676; EP 88,046; EP 143,949; EP 142,641; Japanese Pat. Appl. 83-118008; U.S. Pat. Nos. 4,485,045 and 4,544,545; and EP 102,324. Ordinarily, the liposomes are of the small (about 200-800 Angstroms) unilamellar type in which the lipid content is greater than about 30 mol. percent cholesterol, the selected proportion being adjusted for the optimal therapy.

For parenteral administration, the pharmaceutical composition is formulated generally by mixing it at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation.

Generally, the formulations are prepared by contacting the components of the pharmaceutical composition uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non aqueous vehicles such as fixed oils and ethyl oleate are also useful herein, as well as liposomes. The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) (poly)peptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, manose, or dextrins; chelating agents such as EDTA; sugar alcohols such as mannitol or sorbitol; counterions such as sodium; and/or nonionic surfactants such as polysorbates, poloxamers, or PEG.

The components of the pharmaceutical composition to be used for therapeutic administration must be sterile. Sterility is readily accomplished by filtration through sterile filtration membranes (e.g., 0.2 micron membranes). Therapeutic components of the pharmaceutical composition generally are placed into a container having a sterile access port, for example, an intravenous solution bag or vial having a stopper pierceable by a hypodermic injection needle.

The components of the pharmaceutical composition ordinarily will be stored in unit or multidose containers, for example, sealed ampoules or vials, as an aqueous solution or as a lyophilized formulation for reconstitution. As an example of a lyophilized formulation, 10-ml vials are filled with 5 ml of sterile-filtered 1 % (w/v) aqueous solution, and the resulting mixture is lyophilized. The infusion solution is prepared by reconstituting the lyophilized compound(s) using bacteriostatic Water-for-Injection.

A combination therapy as provided and described herein may be performed by way of the simultaneous, sequential or separate administration of the individual components of said treatment. For example, one or more of the selective inhibitors of vascular endothelial growth factor receptor-2 (VEGFR-2) tyrosine kinase as defined herein (or a pharmaceutically acceptable salt, solvate and/or hydrate thereof) may be administered simultaneously with one or more of the herein defined inhibitors of MAP kinase kinase (MEK) (or a pharmaceutically acceptable salt, solvate and/or hydrate thereof). Also sequential administration of the inhibitors to be used in accordance with the present invention is envisaged herein. The herein defined inhibitors may also be administered separately. For example, one or more of the selective inhibitors of VEGFR-2 tyrosine kinase as defined herein (or a pharmaceutically acceptable salt, solvate and/or hydrate thereof) may be administered in a first step followed by administration in a second step with one or more of the herein defined inhibitors of MEK (or a pharmaceutically acceptable salt, solvate and/or hydrate thereof) and vice versa.

A combination therapy as provided herein can be applied as a sole therapy. It may, however, also be applied with one or more additional therapies (i.e. in cotherapy with), for example, conventional therapies like surgery, radiotherapy and/or one ore more an additional chemotherapeutic agents.

Surgery may comprise the step of partial or complete tumour resection, prior to, during or after the administration of the herein provided combination therapy of one or more of the selective inhibitors of VEGFR-2 tyrosine kinase as defined herein (or a pharmaceutically acceptable salt, solvate and/or hydrate thereof) and one or more of the herein defined inhibitors of MEK (or a pharmaceutically acceptable salt, solvate and/or hydrate thereof). The herein provided inhibitors may be administered in a neoadjuvant or adjuvant setting (in particular neoadjuvant or adjuvant treatment of cancer).

Accordingly, the herein provided combination therapy of one or more of the selective inhibitors of VEGFR-2 tyrosine kinase as defined herein (or a pharmaceutically acceptable salt, solvate and/or hydrate thereof) and one or more of the herein defined inhibitors of MEK (or a pharmaceutically acceptable salt, solvate and/or hydrate thereof) may be administered to a patient in need of such a treatment during or after a surgical intervention/resection of the cancerous tissue. Therefore, the present invention is useful in neoadjuvant therapy, i.e. the treatment with the herein defined combination therapy given to a patient/patient group in need thereof prior to surgery. It is also useful in adjuvant therapy (i.e. after surgery). VEGFR2 inhibition (and in particular the herein provided combination therapy) is thought to be primarily effective in the treatment of early cancer. Accordingly, the use of selective inhibitors of VEGFR-2 tyrosine kinase as defined herein (or a pharmaceutically acceptable salt, solvate and/or hydrate thereof) and one or more of the herein defined inhibitors of MEK (or a pharmaceutically acceptable salt, solvate and/or hydrate thereof) is preferred in the treatment of such an early cancer, especially in preventing relapse induced by residual tumor cells following complete resection of early stage tumors as part of adjuvant therapy.

The above-mentioned one or more additional therapies/cotherapies may be a chemotherapy and may, accordingly, include the administration of one ore more additional chemotherapeutic agents. Such an additional therapy may therefore comprise the administration of one or more of the following exemplary, non-limiting, drugs or agents: Cisplatin, Vinorelbin, Carboplatin, Paclitaxel, Gemcitabin, Docetaxel, Bevacizumab, Pemetrexed, Etoposid, Irinotecan, Ifosfamid, Topotecan in (the above drugs are particularly suitable as additional therapy in subjects/patients with small cell lung cancer (SCLC) or NSCLC (non-small cell lung cancer)),
(an) anti-angiogenic agent(s) like a VEGF blocker (such as bevacizumab/Avastin or sutent (sunitinib malate-SU-11248)), linomide, inhibitors of integrin $\alpha v\beta 3$ function, angiostatin, razoxin, thalidomide, and including vascular targeting agents (for example combretastatin phosphate or N-acetylcolchinol-O-phosphate));
(an) cytostatic agent(s) such as antioestrogens (for example tamoxifen, toremifene, raloxifene, droloxifene, iodoxyfene), progestogens (for example megestrol acetate), aromatase inhibitors (for example anastrozole, letrazole, vorazole, exemestane), antiprogestogens, antiandrogens (for example flutamide, nilutamide, bicalutamide, cyproterone acetate), LHRH agonists and antagonists (for example goserelin acetate, luprolide), inhibitors of testosterone $5\alpha$-dihydroreductase (for example finasteride), anti-invasion agents (for example metalloproteinase inhibitors like marimastat and inliibitors of urokinase plasminogen activator receptor function) and inhibitors of growth factor function, (such growth factors include for example platelet derived growth factor and hepatocyte growth factor such inhibitors include growth factor antibodies, growth factor receptor antibodies, tyrosine kinase inhibitors and serine/threonine kinase inhibitors);
biological response modifiers (for example interferon); (an) anti-metabolite agent(s) (for example gemcitabine); (an) anti-hormonal compound(s) such as (an) anti-estrogen(s); antibodies (for example edrecolomab); adjuvant (anti-) hormonal therapy/therapies (i.e. therapy with (an) adjuvant (anti-) hormone drug(s), such as tamoxifen; gene therapy approaches

(like antisense therapies); and/or immunotherapy approaches.

The cotherapy/additional therapy may also include the use of one or more of antiproliferative/antineoplastic drugs and combinations thereof, as used in medical oncology, such as (an) tyrosine kinase inhibitor(s), (a) raf inhibitor (s), (a) ras inhibitor(s), (a) dual tyrosine kinase inhibitor(s), taxol, (an) taxane(s) (like paclitaxel or docetaxel), (an) anthracycline(s), like doxorubicin or epirubicin, , aromatase inhibitors (such as anastrozole or letrozole) and/or vinorelbine; cyclophosphamide, methotrexate or fluorouracil (which is also known as 5-FU) can be used in such cotherapy individually or in form of a cotherapy comprising these three drugs ("CMF therapy"), optionally in combination with any of the other herein provided additional therapies. Particular examples of chemotherapeutic agents for use with a combination treatment of the present invention are pemetrexed, raltitrexed, etoposide, vinorelbine, paclitaxel, docetaxel, cisplatin, oxaliplatin, carboplatin, gemcitabine, irinotecan (CPT-1 1), 5-fluorouracil (5-FU, (including capecitabine)), doxorubicin, cyclophosphamide, temozolomide, hydroxyurea, (iii) antiproliferative/antineoplastic drugs and combinations thereof, as used in medical oncology, such as antimetabolites (for example antifolates like methotrexate, fluoropyrimidines like 5-fluorouracil, purine and adenosine analogues, cytosine arabinoside); antitumour antibiotics (for example anthracyclines like doxorubicin, daunomycin, epirubicin and idarubicin, mitomycin-C, dactinomycin, mithramycin); platinum derivatives (for example cisplatin, carboplatin); alkylating agents (for example nitrogen mustard, melphalan, chlorambucil, busulphan, cyclophosphamide, ifosfamide, nitrosoureas, thiotepa); antimitotic agents (for example vinca alkaloids like vincristine and taxoids like taxol, taxotere); topoisomerase inhibitors (for example epipodophyllotoxins like etoposide and teniposide, amsacrine, topotecan, and also irinotecan); also enzymes (for example asparaginase); and thymidylate synthase inhibitors (for example raltitrexed); and additional types of chemotherapeutic agents.

Inhibitors for use in accordance with the present invention are described herein and refer generally to known and/or commercially available inhibitors. However, also the use of inhibitors yet to be generated or known compounds to be tested for their inhibiting activity is envisaged in context of the present invention.

The cotherapy/additional therapy may also include the use of one or more of antiproliferative/antineoplastic drugs and combinations thereof, as used in the treatment of patients with non-small cell lung cancer (NSCLC) like Cisplatin, Vinorelbin, Carboplatin, Paclitaxel, Gemcitabin, Docetaxel, Bevacizumab, and/or Pemetrexed. The cotherapy/additional therapy may also include the use of one or more of antiproliferative/antineoplastic drugs and combinations thereof, as used in the treatment of patients with SCLC (small cell lung cancer), like Cisplatin, Vinorelbin, Carboplatin, Paclitaxel, Gemcitabin, Docetaxel, Bevacizumab, Pemetrexed, Etoposid, Irinotecan, Ifosfamid, Topotecan.

Therefore, the present invention relates to a method for assessing the activity of a candidate molecule suspected of being an inhibitor of VEGFR-2 tyrosine kinase comprising the steps of:

(a) contacting a cell, tissue or a non-human animal comprising a VEGFR-2 tyrosine kinase with said candidate molecule;
(b) detecting a decrease in VEGFR-2 tyrosine kinase activity; and
(c) selecting a candidate molecule that decreases VEGFR-2 tyrosine kinase activity;
wherein a decrease of the VEGFR-2 tyrosine kinase activity is in particular indicative for the capacity of the selected molecule to be useful in the treatment of cancer.

Further, the present invention relates to a method for identifying a candidate molecule suspected of being an inhibitor of VEGFR-2 tyrosine kinase comprising the steps of:

(a) contacting a cell, tissue or a non-human animal comprising a VEGFR-2 tyrosine kinase with said candidate molecule;
(b) detecting a decrease in VEGFR-2 tyrosine kinase activity; and
(c) selecting a candidate molecule that decreases VEGFR-2 tyrosine kinase activity;
wherein a decrease of the VEGFR-2 tyrosine kinase activity is in particular indicative for the capacity of the selected molecule to be useful in the treatment of cancer.

Likewise, the present invention relates to a method for assessing the activity of a candidate molecule suspected of being an inhibitor of MEK comprising the steps of:

(a) contacting a cell, tissue or a non-human animal comprising MEK with said candidate molecule;
(b) detecting a decrease in MEK activity; and
(c) selecting a candidate molecule that decreases MEK activity;
wherein a decrease of the MEK activity is in particular indicative for the capacity of the selected molecule to be useful in the treatment of cancer.

Further, the present invention relates to a method for identifying a candidate molecule suspected of being an

inhibitor of MEK comprising the steps of:

(a) contacting a cell, tissue or a non-human animal comprising a MEK with said candidate molecule;
(b) detecting a decrease in MEK activity; and
(c) selecting a candidate molecule that decreases MEK activity;

wherein a decrease of the MEK activity is in particular indicative for the capacity of the selected molecule to be useful in the treatment of cancer.

It is to be understood that the detected activity of a VEGFR-2 tyrosine kinase and MEK, respectively, is compared to a standard or reference value of the activity of a VEGFR-2 tyrosine kinase and MEK, respectively. The standard/reference value may be detected in a cell, tissue, or non-human animal as defined herein, which has not been contacted with a potential inhibitor or prior to the above contacting step. The decrease in the activity of the VEGFR-2 tyrosine kinase and MEK, respectively, upon contacting with (a) candidate molecule(s) may also be compared to the decrease in activity of VEGFR-2 tyrosine kinase and MEK, respectively, induced by (a) routinely used reference compound(s). A skilled person is easily in the position to determine/assess whether the activity and/or expression of a VEGFR-2 tyrosine kinase and MEK, respectively, is (preferably statistically significant) decreased.

In accordance with this invention, in particular the screening or identifying methods described herein, a cell, tissue or non-human animal to be contacted with a candidate molecule comprises the VEGFR-2 tyrosine kinase and MEK, respectively, as defined herein. For example said cell, tissue or non-human animal may express a VEGFR-2 tyrosine kinase gene or MEK gene, in particular also (an) additional (copy) copies of such a gene, (a) mutated gene(s), a recombinant gene construct and the like. As explained herein below, the capability of a candidate molecule to inhibit/antagonize VEGFR-2 tyrosine kinase and MEK, respectively, may, accordingly, be detected by measuring the expression level of such gene products of VEGFR-2 tyrosine kinase gene(s) or MEK gene(s), respectively, or of corresponding gene constructs (e.g. mRNA or protein), wherein a low expression level (compared to a standard or reference value) is indicative for the capability of the candidate molecule to act as inhibitor/antagonist.

The term "comprising VEGFR-2 tyrosine kinase" or "comprising MEK" may, for example, relate to to the VEGFR-2 tyrosine kinase gene(s) or proteins (or MEK gene(s) or proteins) known in the art and described herein, but also to a reporter construct which comprises the VEGFR-2 tyrosine kinase or MEK (or a functional fragment thereof) and a "reporter". Exemplary reporters (reporter gene products), which can be used in the screening methods of the invention are luciferase, (green/red) fluorescent protein and variants thereof, EGFP (enhanced green fluorescent protein), RFP (red fluorescent protein, like DsRed or DsRed2), CFP (cyan fluorescent protein), BFP (blue green fluorescent protein), YFP (yellow fluorescent protein), β-galactosidase or chloramphenicol acetyltransferase. The skilled person is readily in the position to generate and use also other reporters/reporter constructs, which can be employed in accordance with the present invention. The use of fusion proteins containing a VEGFR-2 tyrosine kinase protein or MEK protein (or a functional fragment thereof) and a reporter gene product is also envisaged in the methods of the present invention.

Inhibitors of VEGFR-2 or MEK may interfere with the transcription of the VEGFR-2 tyrosine kinase or MEK reporter construct, in particular fusion proteins. For example, the antagonists may bind to the promoter region of the VEGFR-2 tyrosine kinase or MEK gene or of the sequence encoding the fusion protein, thus preventing initiation of transcription or stopping the already initiated transcription process. The antagonists may also bind to/interfere with components of the transcription machinery, thereby effectively inhibiting initiation of transcription or continuation of transcription. Such an interference with the transcription of the VEGFR-2 tyrosine kinase or MEK gene, VEGFR-2 tyrosine kinase or MEK constructs or VEGFR-2 tyrosine kinase or MEK fusion proteins by a candidate molecule will be reflected in a decreased transcription activity and hence, a reduced transcript level (e.g. unspliced/partially spliced/spliced mRNA). It is also envisaged herein that a reporter construct to be used herein comprises the promoter of a VEGFR-2 tyrosine kinase gene or MEK gene linked to a reporter as described herein. Thus, activity of the VEGFR-2 tyrosine kinase or MEK may be reflected in an activation of its promoter and, hence, in turn reflected in the change/decrease of the reporter signal associated with the reporter.

Due to the reduced transcript level also the level of the translated gene product (i.e. the protein level) will be decreased. The level of the above described fusion proteins preferably correlates with the signal strength of a detectable signal associated with the reporter gene product. Exemplary VEGFR-2 tyrosine kinase or MEK fusion proteins are proteins comprising VEGFR-2 tyrosine kinase or MEK (or a functional fragment thereof) and a reporter as described above (e.g. luciferase, (green/red) fluorescent protein and variants thereof, EGFP (enhanced green fluorescent protein), and the like).

Accordingly, a decrease in VEGFR-2 tyrosine kinase or MEK (promoter) activity (which may, for example, be reflected in a decrease in the (promoter) reporter signal) upon contacting the cell/tissue/non-human animal with a candidate molecule will indicate that the candidate molecule is indeed a VEGFR-2 tyrosine kinase or MEK inhibitor/antagonist and, thus, suitable in the treatment of cancer. The candidate molecules which decrease VEGFR-2 tyrosine kinase or MEK (promoter) activity as defined herein above are selected out of the candidate molecules tested, wherein those

molecules are preferably selected which strongly decrease VEGFR-2 tyrosine kinase or MEK (promoter) activity (reflected, for example, in a pronounced decrease in the reporter signal). It is assumed that the VEGFR-2 tyrosine kinase or MEK antagonizing/inhibiting activity of a candidate molecule is the stronger the more the reporter signal is decreased.

It is envisaged in the context of the present invention (in particular the screening/identifying methods disclosed herein) that also cellular extracts can be contacted (e.g. cellular extracts comprising VEGFR-2 tyrosine kinase or MEK as described and defined herein). For example, these cellular extracts may be obtained from the (transgenic/genetically engineered) cell(s), tissue(s) and/or non-human animal(s) to be used herein, in particular to be contacted with the candidate molecule. The use of such cellular extracts is particular advantageous since it allows the assessment of the activity of a candidate molecule in vitro. The assessing/screening methods taking advantage of such (cellular) extracts can, for example, be used in prescreening candidate molecules, wherein the molecules selected in such a prescreen are then subject to subsequent screens, for example in the cell-based methods disclosed herein, in particular in methods wherein a (transgenic) cell(s), tissue(s) and/or non-human animal(s) are contacted with a candidate molecule. In this context, it is accordingly preferred that the candidate molecule has been selected in the in vitro pre-screening method, described herein above and below.

It is to be understood that in a high throughput screening routinely, many (often thousands of candidate molecules) are screened simultaneously. Accordingly, in a (first) screen candidate molecules are selected, which decrease VEGFR-2 tyrosine kinase or MEK activity.

Step (a) of the screening methods of the present invention, i.e. the "contacting step" may also be accomplished by adding a (biological) sample or composition containing said candidate molecule or a plurality of candidate molecules (i.e. various different candidate molecules) to the sample to be analyzed (e.g. (a) cell(s)/tissue(s)/non-human animal comprising VEGFR-2 tyrosine kinase or MEK or a functional fragment thereof).

Generally, the candidate molecule(s) or a composition comprising/containing the candidate molecule(s) may for example be added to a (transfected) cell, tissue or non-human animal comprising VEGFR-2 tyrosine kinase or MEK. As defined and disclosed herein, the term "comprising VEGFR-2 tyrosine kinase" or "comprising MEK " refers not only to the VEGFR-2 tyrosine kinase or MEK gene(s) or proteins known in the art and described herein, but also to reporter constructs comprising a reporter and VEGFR-2 tyrosine kinase or MEK. Exemplary reporters (preferably associated with the reporter signals disclosed herein) are luciferase and fluorescent proteins, like GFP, RFP and the like. Also reporter constructs comprising a promoter and/or enhancer region of VEGFR-2 tyrosine kinase or MEK and a reporter as defined herein can be used in the screening/identifying methods. Accordingly, the cell(s), tissue(s) and/or non-human animals used in the context of the present invention, in particular in context of the screening/identifying methods can be stably or transiently transfected with the reporter constructs disclosed herein.

In particular the identification/assessment of candidate molecules which are capable of inhibiting/antagonizing VEGFR-2 tyrosine kinase or MEK may be, inter alia, performed by transfecting an appropriate host with a nucleic acid molecule encoding VEGFR-2 tyrosine kinase or MEK (or a functional fragment thereof) and contacting said host with (a) candidate molecule(s). The host (cell, tissue, non-human animal) can also be transfected with the above described reporter constructs, e.g. luciferase reporter constructs, such as, but not limited to, reporter constructs comprising a luciferase reporter. The host may comprise CHO-cell, HEK 293, HeLa, Cos 7, PC12 or NIH3T3 cell, frog oocytes or primary cells like primary cardiomyocytes, fibroblasts, muscle, endothelial or embryonic stem cells. Alternatively, it is also possible to use cell lines stably transfected with a nucleic acid molecule encoding VEGFR-2 tyrosine kinase or MEK or a functional fragment thereof. The explanations given herein above in respect of "cells" also apply to tissues/non-human animals comprising or derived from these cells. A sample to be analyzed may also be a biological, medical or pathological sample, for example fluids that comprise cells, tissues or cell cultures. Such a fluid may be a body fluid or also excrements and may also be a culture sample. The body fluids may comprise but are not limited to blood, serum, plasma, urine, saliva, synovial fluid, spinal fluid, cerebrospinal fluid, tears, stool and the like.

The (biological) sample or composition, comprising a plurality of candidate molecules are usually subject to a first screen. The samples/compositions tested positive in the first screen are often subject to subsequent screens in order to verify the previous findings and to select the most potent inhibitors/antagonists of the VEGFR-2 tyrosine kinase or MEK. Upon multiple screening and selection rounds those candidate molecules will be selected which show a pronounced capacity to inhibit/antagonize VEGFR-2 tyrosine kinase or MEK as defined and disclosed herein. For example, batches (i.e. compositions/samples) containing many candidate molecules will be rescreened and batches with no or insufficient inhibitory activity of candidate molecules be discarded without re-testing.

For example, if a (biological) sample or composition with many different candidate molecules is tested and one (biological) sample or composition is tested positive, then it is either possible in a second screening to screen, preferably after purification, the individual molecule(s) of the (biological) sample or composition. It may also be possible to screen subgroups of the (biological) sample or composition of the first screen in (a) subsequent screen(s). The screening of compositions with subgroups of those candidate molecules tested in previous screening rounds will thus narrow in on (an) potential potent VEGFR-2 tyrosine kinase or MEK inhibitor(s). This may facilitate and accelerate the screening process in particular when a large number of molecules is screened. Accordingly, the cycle number of screening rounds

is reduced compared to testing each and every individual candidate molecule in (a) first (and subsequent) screen(s) (which is, of course, also possible). Thus, depending on the complexity or the number of the candidate molecules, the steps of the screening method described herein can be performed several times until the (biological) sample or composition to be screened comprises a limited number, preferably only one substance.

The term "decrease in VEGFR-2 tyrosine kinase (or MEK) activity" in step (b) of the screening method means that the "activity of the VEGFR-2 tyrosine kinase (or MEK)" is reduced upon contacting the cell, tissue, or non-human animal comprising a VEGFR-2 tyrosine kinase or MEK with the candidate molecule, preferably in comparison to a (control) standard or reference value, as defined herein.

Particularly preferred are optical measurement techniques that allow a resolution of fluorescence on the level of single cells or single cells of a tissue, preferably at the subcellular level. They may involve fluorescence, for example confocal microscopy, digital image recording, preferably a CCD camera and suitable picture analysis software. Preferably, step (b) is carried out after the measurement of a standard response by performing a control experiment. For example, the activity of a VEGFR-2 tyrosine kinase or MEK is measured in a cell, tissue or a non-human animal comprising a VEGFR-2 tyrosine kinase or MEK without contacting a candidate molecule in a first screen. In a second screen, after contacting the candidate molecule, the activity of the VEGFR-2 tyrosine kinase or MEK is measured. A difference in the activities will indicate whether the tested candidate molecule is indeed an antagonist of a VEGFR-2 tyrosine kinase or MEK.

The activity of a VEGFR-2 tyrosine kinase or MEK can be quantified by measuring, for example, the level of gene products (e.g. mRNA and/or protein of the VEGFR-2 tyrosine kinase or MEK and said component, respectively) by any of the herein described methods, activities, or other cellular functions, like inter alia, the involvement in signalling pathways or changes in intracellular localization.

As mentioned, a "VEGFR-2 tyrosine kinase or MEK activity" and, accordingly, a decreased concentration/amount of VEGFR-2 tyrosine kinase or MEK proteins in a sample may be reflected in a decreased expression of the corresponding gene(s) encoding the VEGFR-2 tyrosine kinase or MEK protein(s). Therefore, a quantitative assessment of the gene product (e.g. protein or spliced, unspliced or partially spliced mRNA) can be performed in order to evaluate decreased expression of the corresponding gene(s) encoding the VEGFR-2 tyrosine kinase or MEK protein(s). Also here, a person skilled in the art is aware of standard methods to be used in this context or may deduce these methods from standard textbooks (e.g. Sambrook, 2001, loc. cit.). For example, quantitative data on the respective concentration/amounts of mRNA from the VEGFR-2 tyrosine kinase or MEK can be obtained by Northern Blot, Real Time PCR and the like. Preferably, the concentration/amount of the gene product (e.g. the herein above described VEGFR-2 tyrosine kinase or MEK mRNA or VEGFR-2 tyrosine kinase or MEK protein) may be decreased by at least about 10 %, 20 %, 30 %, 40 %, preferably by at least 50 %, 60 %, 70 %, 80 %, 90 %, 91%, 92 %, 93 %, 94 %, 95 %, 96 %, 97 %, 98 %, 99 % or even 100 % compared to a control sample. A decrease by 100 % means that no gene product is detectable by herein described routine methods. It is preferred herein that VEGFR-2 tyrosine kinase or MEK proteins are (biologically) active or functional. Methods for determining the activity of a VEGFR-2 tyrosine kinase or MEK are described herein above and shown in the appended example. Since the VEGFR-2 tyrosine kinase or MEK proteins are preferably (biologically) active/functional (wherein it is preferred that at least 70 %, 75 %, preferably at least 80%, 85 %, 90 %, 95 %, 96, %, 97%, 98 % and most preferably, at least 99 % of VEGFR-2 tyrosine kinase or MEK proteins of a sample a (biologically) active/functional), an decreased concentration/amount of VEGFR-2 tyrosine kinase or MEK proteins in a sample reflects a decreased (biological) activity of the VEGFR-2 tyrosine kinase or MEK protein.

As mentioned, a person skilled in the art is aware of standard methods to be used for determining or quantitating expression of a nucleic acid molecule encoding, for example, the VEGFR-2 tyrosine kinase or MEK (or fragments thereof) as defined herein. For example, the expression can be determined on the protein level by taking advantage of immunoagglutination, immunoprecipitation (e.g. immunodiffusion, immunelectrophoresis, immune fixation), western blotting techniques (e.g. (in situ) immuno histochemistry, (in situ) immuno cytochemistry, affinity chromatography, enzyme immunoassays), and the like. Amounts of purified polypeptide in solution can be determined by physical methods, e.g. photometry. Methods of quantifying a particular polypeptide in a mixture rely on specific binding, e.g. of antibodies. Specific detection and quantitation methods exploiting the specificity of antibodies comprise for example immunohistochemistry (in situ). For example, concentration/amount of VEGFR-2 tyrosine kinase or MEK proteins in a cell, tissue or a non-human animal can be determined by enzyme linked-immunosorbent assay (ELISA). Alternatively, Western Blot analysis or immunohistochemical staining can be performed. Western blotting combines separation of a mixture of proteins by electrophoresis and specific detection with antibodies. Electrophoresis may be multi-dimensional such as 2D electrophoresis. Usually, polypeptides are separated in 2D electrophoresis by their apparent molecular weight along one dimension and by their isoelectric point along the other direction.

Expression can also be determined on the nucleic acid level (e.g. if the gene product/product of the coding nucleic acid sequence is an unspliced/partially spliced/spliced mRNA) by taking advantage of Northern blotting techniques or PCR techniques, like in-situ PCR or Real time PCR. Quantitative determination of mRNA can be performed by taking advantage of northern blotting techniques, hybridization on microarrays or DNA chips equipped with one or more probes

or probe sets specific for mRNA transcripts or PCR techniques referred to above, like, for example, quantitative PCR techniques, such as Real time PCR. These and other suitable methods for detection and/or determination of the concentration/amount of (specific) mRNA or protein(s)/polypeptide(s) are well known in the art and are, for example, described in Sambrook (2001), loc. cit.).

A skilled person is capable of determining the amount of mRNA or polypeptides/proteins, in particular the gene products described herein above, by taking advantage of a correlation, preferably a linear correlation, between the intensity of a detection signal and the amount of, for example, the mRNA or polypeptides/proteins to be determined. Accordingly, the activity of a VEGFR-2 tyrosine kinase or MEK (or a functional fragment thereof) may be quantified based on the mRNA or protein level of the VEGFR-2 tyrosine kinase or MEK or a functional fragment thereof and vice versa.

Genetic readout systems are also envisaged. Analogously, the activity of a VEGFR-2 tyrosine kinase or MEK or of a functional fragment thereof may be quantified by any molecular biological method as described herein. A skilled person is also aware of standard methods to be used in determining the amount/concentration of VEGFR-2 tyrosine kinase or MEK expression products (in particular the protein and the nucleic acid level of a VEGFR-2 tyrosine kinase or MEK) in a sample or may deduce corresponding methods from standard textbooks (e.g. Sambrook, 2001).

In samples obtained from (a) cell(s), tissue or a cell culture(s) transfected with appropriate constructs or obtained from transgenic animals or cell cultures derived from a non-human animal(s), the concentration/amount of VEGFR-2 tyrosine kinase or MEK protein can be determined by bioassays, if, for example, a VEGFR-2 tyrosine kinase- or MEK -inducible promoter is fused to a reporter gene. Apparently, decreased expression of the reporter gene/activity of the reporter gene product will reflect a decreased VEGFR-2 tyrosine kinase or MEK activity, in particular a decreased concentration/amount of VEGFR-2 tyrosine kinase or MEK protein. Alternatively, the effect of the VEGFR-2 tyrosine kinase or MEK protein on the expression of (a) reporter gene(s) may be evaluated by determining the amount/concentration of the gene product of the reporter gene(s) (e.g. protein or spliced, unspliced or partially spliced mRNA). Further methods to be used in the assessment of mRNA expression of a reporter gene are within the scope of a skilled person and also described herein below.

Also in this context, reporter constructs comprising a promoter and/or enhancer region of VEGFR-2 tyrosine kinase or MEK and a reporter as defined herein can be used in the screening/identifying methods. Exemplary reporters (preferably associated with the reporter signals disclosed herein) are luciferase and fluorescent proteins, like GFP, RFP and the like. Exemplary reporters (preferably associated with the reporter signals disclosed herein) are luciferase and fluorescent proteins, like GFP, RFP and the like. These and other reporters/reporter constructs/reporter signals are also described herein above and below.

The difference, as disclosed herein is statistically significant and a candidate molecule(s) is (are) selected, if the VEGFR-2 tyrosine kinase or MEK activity (or of a corresponding reporter signal) is strongly decreased, preferably is very low or non-dectable. For example, the VEGFR-2 tyrosine kinase or MEK activity (or of a corresponding reporter signal) may be decreased by at least 50%, 60%, 70%, 80%, more preferably by at least 90% compared to the (control) standard value. In a cell based method the cells can be transfected with one or more constructs encoding a VEGFR-2 tyrosine kinase or MEK or a functional fragment thereof as described above and optionally a reporter under the transcriptional control of the VEGFR-2 tyrosine kinase or MEK promoter or a functional fragment thereof as described above.

Preferably, the selected compound has a high VEGFR-2 tyrosine kinase or MEK inhibiting/antagonizing activity. This can be reflected in the capacity of the VEGFR-2 tyrosine kinase or MEK antagonist/inhibitor to potently decrease the activity of VEGFR-2 tyrosine kinase or MEK.

The above detected difference between the activity of a VEGFR-2 tyrosine kinase or MEK or the activity of a functional fragment of the VEGFR-2 tyrosine kinase or MEK in a cell, tissue or a non-human animal contacted with said candidate molecule and the activity in the (control) standard value (measured e.g. in the absence of said candidate molecule) may be reflected by the presence, the absence, the increase or the decrease of a specific signal in the readout system, as in the herein described fluorescence based system.

Preferably, candidate agents to be tested encompass numerous chemical classes, though typically they are organic compounds, preferably small (organic) molecules as defined herein above. Candidate agents may also comprise functional groups necessary for structural interaction with proteins, particularly hydrogen bonding, and typically include at least an amine, carbonyl, hydroxyl or carboxyl group, preferably at least two of the functional chemical groups. The candidate agents often comprise carbocyclic or heterocyclic structures and/or aromatic or polyaromatic structures substituted with one or more of the above functional groups.

Exemplary classes of candidate agents may include heterocycles, peptides, saccharides, steroids, and the like. The compounds may be modified to enhance efficacy, stability, pharmaceutical compatibility, and the like. Structural identification of an agent may be used to identify, generate, or screen additional agents. For example, where peptide agents are identified, they may be modified in a variety of ways to enhance their stability, such as using an unnatural amino acid, such as a D-amino acid, particularly D-alanine, by functionalizing the amino or carboxylic terminus, e.g. for the amino group, acylation or alkylation, and for the carboxyl group, esterification or amidification, or the like. Other methods

of stabilization may include encapsulation, for example, in liposomes, etc.
As mentioned above, candidate agents are also found among other biomolecules including amino acids, fatty acids, purines, pyrimidines, nucleic acids and derivatives, structural analogs or combinations thereof. Candidate agents are obtained from a wide variety of sources including libraries of synthetic or natural compounds. For example, numerous means are available for random and directed synthesis of a wide variety of organic compounds and biomolecules, including expression of randomized oligonucleotides and oligopeptides. Alternatively, libraries of natural compounds in the form of bacterial, fungal, plant and animal extracts are available or readily produced. Additionally, natural or synthetically produced libraries and compounds are readily modified through conventional chemical, physical and biochemical means, and may be used to produce combinatorial libraries. Known pharmacological agents may be subjected to directed or random chemical modifications, such as acylation, alkylation, esterification, amidification, etc. to produce structural analogs.

The reporter constructs for detecting VEGFR-2 tyrosine kinase or MEK inhibition as described herein above may be comprised in a cell, tissue or a non-human animal. Methods for transfecting cells or tissues are known in the art. Accordingly, calcium phosphate treatment or electroporation may be used for transfecting cells or tissues to express said reporter constructs (see Sambrook (1989), loc. cit.). Furthermore, nucleic acid molecules expressing said reporter constructs can be reconstituted into liposomes for delivery to target cells. As a further alternative, cells may be transduced to express specific reporter construct using genetically engineered viral vectors.

The non-human animal comprising said reporter construct for detecting VEGFR-2 tyrosine kinase or MEK inhibition may be a transgenic non-human animal. The non-human organism to be used in the described screening assays is preferably selected from the group consisting of C. elegans, yeast, drosophila, zebrafish, guinea pig, rat and mouse. The generation of such a transgenic animal is within the skill of a skilled artisan. Corresponding techniques are, inter alia, described in "Current Protocols in Neuroscience" (2001), John Wiley&Sons, Chapter 3.16. Accordingly, the invention also relates to a method for the generation of a non-human transgenic animal comprising the step of introducing a reporter construct for detecting VEGFR-2 tyrosine kinase or MEK inhibition as disclosed herein into an ES-cell or a germ cell. The non-human transgenic animal provided and described herein is particular useful in screening methods and pharmacological tests described herein above. In particular the non-human transgenic animal described herein may be employed in drug screening assays as well as in scientific and medical studies wherein antagonists/inhibitors of VEGFR-2 tyrosine kinase or MEK for the treatment of a cancer are tracked, selected and/or isolated.

The transgenic/genetically engineered cell(s), tissue(s), and/or non-human animals to be used in context of the present invention, in particular, the screening/identifying methods, preferably comprise the herein described and defined reporter constructs. Also in this context, reporter constructs may comprise a promoter and/or enhancer region of a VEGFR-2 tyrosine kinase or MEK and a reporter as defined herein. Exemplary reporters (preferably associated with the reporter signals disclosed herein) are luciferase and fluorescent proteins, like GFP, RFP and the like. Exemplary reporters (preferably associated with the reporter signals disclosed herein) are luciferase and fluorescent proteins, like GFP, RFP and the like. These and other reporters/reporter constructs/reporter signals are also described herein above and below.

The present invention relates to the use of a cell, tissue or a non-human animal for screening and/or validation of a compound suspected of being an antagonist of a VEGFR-2 tyrosine kinase or MEK. Correspondingly, the present invention relates to the use of a cell, tissue or a non-human animal for screening and/or validation of a compound suspected of being an agonist of a VEGFR-2 tyrosine kinase or MEK. Accordingly, herein envisaged is the use of a cell, tissue or a non-human animal for screening and/or validation of a compound suspected of being an antagonist of a VEGFR-2 tyrosine kinase or MEK.

The term "cell" as used in this context may also comprise a plurality of cell as well as cells comprised in a tissue.. For example, a high and stable expression of VEGFR-2 tyrosine kinase or MEK may facilitate the detection of a decrease in VEGFR-2 tyrosine kinase or MEK activity. Since wild-type cells have sometimes a low or unstable VEGFR-2 tyrosine kinase or MEK expression, the use of (a) transgenic cell(s), tissue(s), non-human animal is particularly envisaged, if these cells have a high VEGFR-2 tyrosine kinase or MEK expression (reflected in a high protein or mRNA level). (Transgenic) cells(s), tissue(s) and non-human animals to be used in accordance with the present invention are also described herein above.

The used non-human animal or cell may be transgenic or non transgenic. In this context the term "transgenic" particularly means that at least one of the VEGFR-2 tyrosine kinase or MEK genes as described herein is overexpressed; thus the VEGFR-2 tyrosine kinase or MEK activity in the non-human transgenic animal or a transgenic animal cell is enhanced. Generally, it is preferred herein that VEGFR-2 tyrosine kinase or MEK is highly expressed in (a) cell(s), tissue (s), non-human animal to be used in the screening methods as described above.

The term "transgenic non-human-animal", "transgenic cell" or "transgenic tissue" as used herein refers to an non-human animal, tissue or cell, not being a human that comprises different genetic material of a corresponding wild-type animal, tissue or cell. The term "genetic material" in this context may be any kind of a nucleic acid molecule, or analogues thereof, for example a nucleic acid molecule, or analogues thereof as defined herein. The term "different"

means that additional or fewer genetic material in comparison to the genome of the wild type animal or animal cell. An overview of different expression systems to be used for generating transgenic cell/animal refers for example to Methods in Enzymology 153 (1987), 385-516, in Bitter et al (Methods in Enzymology 153 (1987), 516-544) and in Sawers et al. (Applied Microbiology and Biotechnology 46 (1996), 1-9), Billman-Jacobe (Current Opinion in Biotechnology 7 (1996), 500-4), Hockney (Trends in Biotechnology 12 (1994), 456-463), Griffiths et al., (Methods in Molecular Biology 75 (1997), 427-440).

Tthe (transgenic) non-human animal or (transgenic) cell may be a mammal or may be derived from a mammal. Non-limiting examples of the (transgenic) non-human animal or derived (transgenic) cell are selected from the group consisting of a mouse, a rat, a rabbit, a guinea pig and Drosophila. Generally, the (transgenic) cell may be a prokaryotic or eukaryotic cell. For example, the (transgenic) cell in accordance with the present invention may be but is not limited to bacterial, yeast, fungus, plant or animal cell. In general, the transformation or genetically engineering of a cell with a nucleic acid construct or a vector can be carried out by standard methods, as for instance described in Sambrook and Russell (2001), Molecular Cloning: A Laboratory Manual, CSH Press, Cold Spring Harbor, NY, USA; Methods in Yeast Genetics, A Laboratory Course Manual, Cold Spring Harbor Laboratory Press, 1990.

VEGFR2 tyrosine kinase may be selected from the group consisting of

(a) a polypeptide comprising an amino acid encoded by a nucleic acid molecule having the nucleic acid sequence as depicted in SEQ ID NO: 1;
(b) a polypeptide having an amino acid sequence as depicted in SEQ ID NO: 2;
(c) a polypeptide encoded by a nucleic acid molecule encoding a peptide having an amino acid sequence as depicted in SEQ ID NO: 2;
(d) a polypeptide comprising an amino acid encoded by a nucleic acid molecule hybridizing under stringent conditions to the complementary strand of nucleic acid molecules as defined in (a) or (c) and encoding a functional VEGFR-2 tyrosine kinase, or a fragment thereof;
(e) a polypeptide having at least 60 % homology to the polypeptide of any one of (a) to (d), whereby said polypeptide is a functional VEGFR-2 tyrosine kinase, or a fragment thereof; and
(f) a polypeptide comprising an amino acid encoded by a nucleic acid molecule being degenerate as a result of the genetic code to the nucleotide sequence of a nucleic acid molecule as defined in (a), (c) and (d).

The term "functional VEGFR-2 tyrosine kinase " used in context of the present invention refers to a polypeptide having at least 60 % homology to a polypeptide as defined in section (a) to (d) of the above-described specific aspect of the present invention which has essentially the same biological activity as a polypeptide having 100 % homology to a polypeptide as indicated in section (a) to (d), i.e. a polypeptide being essentially identical to a polypeptide having an amino acid sequence as depicted in SEQ ID NO: 2. Methods for determining the activity of (a) polypeptide(s) are well known in the art and may, for example, be deduced from standard text books, such as Bioanalytik (Lottspeich/Zorbas (eds.), 1998, Spektrum Akademischer Verlag). Methods for determining the activity of a VEGFR-2 tyrosine kinase or MEK are also described herein below.

It is of note that a (functional) VEGFR-2 tyrosine kinase or a functional fragment thereof as described and defined herein may further comprise a heterologous polypeptide, for example, (an) amino acid sequence(s) for identification and/or purification of the recombinant protein (e.g. amino acid sequence from C-MYC, GST protein, FLAG peptide, HIS peptide and the like), an amino acid sequence used as reporter (e.g. green fluorescent protein, yellow fluorescent protein, red fluorescent protein, luciferase, and the like), or antibodies/antibody fragments (like scFV). A person skilled in the art knows that for determination of homology as described herein only a part of a polypeptide is to be used, whereby said part is VEGFR-2 tyrosine kinase (or a functional fragment thereof). Also further compounds (e.g. toxins or antibodies or fragments thereof) may be attached to VEGFR-2 tyrosine kinase (or a functional fragment thereof) by standard techniques. These compounds may, in particular, be useful in a medical setting as described herein, wherein antagonists of VEGFR-2 tyrosine kinase (or a functional fragment thereof) are used. A skilled person is aware of compounds to be used/attached in this context.

Methods and assays for determining the activity of "VEGFR-2 tyrosine kinase" are described herein. These methods also allow determining whether a polypeptide can be considered as a "functional VEGFR-2 tyrosine kinase". The activity exhibited by the following exemplary polypeptide can be considered as "reference activity" of a functional VEGFR-2 tyrosine kinase: a polypeptide having an amino acid sequence as depicted in SEQ ID NO: 2 ("human VEGFR-2 tyrosine kinase).

The meanings of the term "polypeptide" and "nucleic acid sequence(s)/molecule(s)" are well known in the art and are used accordingly in context of the present invention. For example, "nucleic acid sequence(s)/molecule(s)" as used herein refer(s) to all forms of naturally occurring or recombinantly generated types of nucleic acids and/or nucleic acid sequences/molecules as well as to chemically synthesized nucleic acid sequences/molecules. This term also encompasses nucleic acid analogues and nucleic acid derivatives such as e. g. locked DNA, PNA, oligonucleotide

thiophosphates and substituted ribo-oligonucleotides. Furthermore, the term "nucleic acid sequence(s)/molecules(s)" also refers to any molecule that comprises nucleotides or nucleotide analogues. Preferably, the term "nucleic acid sequence(s)/molecule(s)" refers to deoxyribonucleic acid (DNA) and ribonucleic acid (RNA). The "nucleic acid sequence (s)/molecule(s)" may be made by synthetic chemical methodology known to one of ordinary skill in the art, or by the use of recombinant technology, or may be isolated from natural sources, or by a combination thereof. The DNA and RNA may optionally comprise unnatural nucleotides and may be single or double stranded. "Nucleic acid sequence(s)/molecule (s)" also refers to sense and anti-sense DNA and RNA, that is, a nucleotide sequence which is complementary to a specific sequence of nucleotides in DNA and/or RNA. Furthermore, the term "nucleic acid sequence(s)/molecule(s)" may refer to DNA or RNA or hybrids thereof or any modification thereof that is known in the state of the art (see, e.g., US 5525711, US 4711955, US 5792608 or EP 302175 for examples of modifications). The nucleic acid molecule(s) may be single- or double-stranded, linear or circular, natural or synthetic, and without any size limitation. For instance, the nucleic acid molecule(s) may be genomic DNA, cDNA, mRNA, antisense RNA, ribozymal or a DNA encoding such RNAs or chimeroplasts (Colestrauss, Science (1996), 1386-1389. Said nucleic acid molecule(s) may be in the form of a plasmid or of viral DNA or RNA. "Nucleic acid sequence(s)/molecule(s)" may also refer to (an) oligonucleotide(s), wherein any of the state of the art modifications such as phosphothioates or peptide nucleic acids (PNA) are included.

The nucleic acid sequence of VEGFR-2 tyrosine kinase of other species than the herein provided human sequence can be identified by the skilled person using methods known in the art, e.g. by using hybridization assays or by using alignments, either manually or by using computer programs such as those mentioned herein below in connection with the definition of the term "hybridization" and degrees of homology.

The nucleic acid sequence encoding for orthologs of human VEGFR-2 tyrosine kinase may be at least 40% homologous to the nucleic acid sequence as shown in SEQ ID NO. 1. More preferably, the nucleic acid sequence encoding for orthologs of human VEGFR-2 tyrosine kinase is at least 45%, 50%, 55%, 60%, 65%, 70%, 75%, 80%, 85%, 90%, 91%, 92%, 93%, 94%, 95%, 96%, 97% or 98% homologous to the nucleic acid sequence as shown in SEQ ID NOs. 1, wherein the higher values are preferred. Most preferably, the nucleic acid sequence encoding for orthologues of VEGFR-2 tyrosine kinase is at least 99% homologous to the nucleic acid sequence as shown in SEQ ID NO. 1. The term "orthologous protein" or "orthologous gene" as used herein refers to proteins and genes, respectively, in different species that are similar to each other because they originated from a common ancestor.

The same definitions given herein in respect of human VEGFR-2 tyrosine kinase (and orthologues/homologs thereof) apply, mutatis mutandis, to MEK (and orthologues/homologs thereof) with the exception that corresponding nucleic acid and amino acid sequence of MEK are shown in SEQ ID NO. 3, 4, 5, 6, 11 and 12 respectively. The definitions and explanations also apply, mutatis mutandis, to other VEGFR-2 tyrosine kinases or MEK kinases, such as kinases isolated/ derived from further sources, like the herein described animal sources such as pigs or guinea pigs and the like.

Hybridization assays for the characterization of orthologs of known nucleic acid sequences are well known in the art; see e.g. Sambrook, Russell "Molecular Cloning, A Laboratory Manual", Cold Spring Harbor Laboratory, N.Y. (2001); Ausubel, "Current Protocols in Molecular Biology", Green Publishing Associates and Wiley Interscience, N.Y. (1989). The term "hybridization" or "hybridizes" as used herein may relate to hybridizations under stringent or non-stringent conditions. If not further specified, the conditions are preferably non-stringent. Said hybridization conditions may be established according to conventional protocols described, e.g., in Sambrook (2001) loc. cit.; Ausubel (1989) loc. cit., or Higgins and Hames (Eds.) "Nucleic acid hybridization, a practical approach" IRL Press Oxford, Washington DC, (1985). The setting of conditions is well within the skill of the artisan and can be determined according to protocols described in the art. Thus, the detection of only specifically hybridizing sequences will usually require stringent hybridization and washing conditions such as, for example, the highly stringent hybridization conditions of 0.1 x SSC, 0.1% SDS at 65°C or 2 x SSC, 60°C, 0.1 % SDS. Low stringent hybridization conditions for the detection of homologous or not exactly complementary sequences may, for example, be set at 6 x SSC, 1% SDS at 65°C. As is well known, the length of the probe and the composition of the nucleic acid to be determined constitute further parameters of the hybridization conditions.

In accordance with the present invention, the terms "homology" or "percent homology" or "identical" or "percent identity" or "percentage identity" or "sequence identity" in the context of two or more nucleic acid sequences refers to two or more sequences or subsequences that are the same, or that have a specified percentage of nucleotides that are the same (preferably at least 40% identity, more preferably at least 45%, 50%, 55%, 60%, 65%, 70%, 75%, 80%, 85%, 90%, 91%, 92%, 93%, 94%, 95%, 96%, 97% or 98% identity, most preferably at least 99% identity), when compared and aligned for maximum correspondence over a window of comparison (preferably over the full length), or over a designated region as measured using a sequence comparison algorithm as known in the art, or by manual alignment and visual inspection. Sequences having, for example, 75% to 90% or greater sequence identity may be considered to be substantially identical. Such a definition also applies to the complement of a test sequence. Preferably the described identity exists over a region that is at least about 15 to 25 nucleotides in length, more preferably, over a region that is at least about 50 to 100 nucleotides in length and most preferably, over a region that is at least about 800 to 1200 nucleotides in length. Those having skill in the art will know how to determine percent identity between/among sequences

using, for example, algorithms such as those based on CLUSTALW computer program (Thompson Nucl. Acids Res. 2 (1994), 4673-4680) or FASTDB (Brutlag Comp. App. Biosci. 6 (1990), 237-245), as known in the art.

Although the FASTDB algorithm typically does not consider internal non-matching deletions or additions in sequences, i.e., gaps, in its calculation, this can be corrected manually to avoid an overestimation of the % identity. CLUSTALW, however, does take sequence gaps into account in its identity calculations. Also available to those having skill in this art are the BLAST and BLAST 2.0 algorithms (Altschul, (1997) Nucl. Acids Res. 25:3389-3402; Altschul (1993) J. Mol. Evol. 36:290-300; Altschul (1990) J. Mol. Biol. 215:403-410). The BLASTN program for nucleic acid sequences uses as defaults a word length (W) of 11, an expectation (E) of 10, M=5, N=4, and a comparison of both strands. The BLOSUM62 scoring matrix (Henikoff (1989) PNAS 89:10915) uses alignments (B) of 50, expectation (E) of 10, M=5, N=4, and a comparison of both strands.

In order to determine whether an nucleotide residue in a nucleic acid sequence corresponds to a certain position in the nucleotide sequence of e.g. SEQ ID NOs: 1, 3, 5, 7, 9 or 11, respectively, the skilled person can use means and methods well-known in the art, e.g., alignments, either manually or by using computer programs such as those mentioned herein. For example, BLAST 2.0, which stands for Basic Local Alignment Search Tool BLAST (Altschul (1997), loc. cit.; Altschul (1993), loc. cit.; Altschul (1990), loc. cit.), can be used to search for local sequence alignments. BLAST, as discussed above, produces alignments of nucleotide sequences to determine sequence similarity. Because of the local nature of the alignments, BLAST is especially useful in determining exact matches or in identifying similar sequences. The fundamental unit of BLAST algorithm output is the High-scoring Segment Pair (HSP). An HSP consists of two sequence fragments of arbitrary but equal lengths whose alignment is locally maximal and for which the alignment score meets or exceeds a threshold or cut-off score set by the user. The BLAST approach is to look for HSPs between a query sequence and a database sequence, to evaluate the statistical significance of any matches found, and to report only those matches which satisfy the user-selected threshold of significance. The parameter E establishes the statistically significant threshold for reporting database sequence matches. E is interpreted as the upper bound of the expected frequency of chance occurrence of an HSP (or set of HSPs) within the context of the entire database search. Any database sequence whose match satisfies E is reported in the program output.

Analogous computer techniques using BLAST (Altschul (1997), loc. cit.; Altschul (1993), loc. cit.; Altschul (1990), loc. cit.) are used to search for identical or related molecules in nucleotide databases such as GenBank or EMBL. This analysis is much faster than multiple membrane-based hybridizations. In addition, the sensitivity of the computer search can be modified to determine whether any particular match is categorized as exact or similar. The basis of the search is the product score, which is defined as:

$$\frac{\text{\% sequence identity x \% maximum BLAST score}}{100}$$

and it takes into account both the degree of similarity between two sequences and the length of the sequence match. For example, with a product score of 40, the match will be exact within a 1-2% error; and at 70, the match will be exact. Similar molecules are usually identified by selecting those, which show product scores between 15 and 40, although lower scores may identify related molecules. Another example for a program capable of generating sequence alignments is the CLUSTALW computer program (Thompson (1994) Nucl. Acids Res. 2:4673-4680) or FASTDB (Brutlag (1990) Comp. App. Biosci. 6:237-245), as known in the art.

The explanations and definitions given herein above in respect of "homology of nucleic acid sequences" apply, mutatis mutandis, to "amino acid sequences" of VEGFR-2 tyrosine kinase as depicted in SEQ ID NO: 2 (human VEGFR-2 tyrosine kinase) or MEK as depicted in SEQ ID NO: 4, 6 or 12 (human MEK), as explained below. All the definitions given herein above, apply mutatis mutandis, also to epidermal growth factor receptor (EGFR) tyrosine kinase. An exemplary nucleic acid sequence thereof is depicted in SEQ ID NO: 9 (human EGFR) and an exemplary amino acid sequence thereof is depicted in SEQ ID NO: 10 (human EGFR).

The polypeptide to be used in accordance with the present invention may have at least 40 % homology to the VEGFR-2 tyrosine kinase or MEK having the amino acid sequence as, for example, depicted in SEQ ID NO: 2 (human VEGFR-2 tyrosine kinase) or as depicited in SEQ ID NO: 4, 6 or 12 (human MEK), respectively. More preferably, the polypeptide has at least 45%, 50%, 55%, 60%, 65%, 70%, 75%, 80%, 85%, 90%, 91%, 92%, 93%, 94%, 95%, 96%, 97% or 98% homology to VEGFR-2 tyrosine kinase or MEK as depicted in SEQ ID NO: 2 (human VEGFR-2 tyrosine kinase) or as depicited in SEQ ID NO: 4, 6 or 12 (human MEK), respectively, wherein the higher values are preferred. Most preferably, the polypeptide has at least 99% homology to the VEGFR-2 tyrosine kinase or MEK, as depicted in SEQ ID NO: 2 (human VEGFR-2 tyrosine kinase) or as depicited in SEQ ID NO: 4, 6 or 12 (human MEK), respectively.

In one aspect, the present invention provides for the identification of subjects or patients which are susceptible to cancer therapy, in particular to the above described combination therapy. As documented in the appended examples the herein provided combination therapy using a selective inhibitor of VEGFR2 and a MEK inhibitor is highly effective

in cancer treatment, as it leads to an unexpected and strong reduction of tumor volume; even more surprisingly it was found herein that a high expression level of VEGF and VEGFR2 indicates a particularly pronounced susceptibility to cancer therapy, in particular to the herein provided combination therapy.

As shown in Figure 10A, test animals ("H441" and "H1975") treated with the herein provided combination therapy using a selective inhibitor of VEGFR2 (Vandetanib) and a MEK inhibitor (PD0325901) showed a drastic tumor shrinkage: the tumor almost completely disappeared after 3 to 4 weeks of treatment (tumor volume after treatment compared to initial tumor volume at start of the treatment). In contrast, monotherapy with the selective VEGFR-2 inhibitor Vandetanib and the MEK inhibitor (PD0325901), respectively, did not reduce the tumor volume - rather, the tumor volume continued to increase, although at a slightly reduced speed compared to the non-treated control.

The animal models ("H441" and "H1975") used in the therapies of Example 2 (the results thereof are shown in Figure 10A ) have a high VEGF and VEGFR2 expression level in the tumor, i.e. the tumor/cancer is characterized by a high VEGF and VEGFR2 expression level. In Figure 10B the results of combination therapy using a selective inhibitor of VEGFR2 (Vandetanib) and a MEK inhibitor (PD0325901) in a further animal model ("H1650") are shown : this animal model is characterized by a very low VEGF and VEGFR2 expression level. Here, therapy with the selective inhibitor of VEGFR2 (Vandetanib) and a MEK inhibitor (PD0325901) did not at all reduce the tumor volume: the tumor volume increased alike in the monotherapy using only the MEK inhibitor (PD0325901) and in the combination therapy of Vandetanib and PD0325901. Yet, the tumor volume in the monotherapy using only Vandetanib increased even more compared with the MEK inhibitor monotherapy or the combination therapy. Indeed, tumor volume in the Vandetanib monotherapy was almost comparable to the non-treated control. The animal model ("H1650"), especially the tumor of said animal model, used in Example 2 (Fig. 10B) can, therefore, be considered as an example of a "non-responder" as defined herein to the herein provided combination therapy.

Thus, it was found herein that the efficacy of the combination therapy depends on the expression level of VEGFR2/VEGF. While the herein disclosed combination therapy is highly effective in a VEGFR2/VEGF expressing setting, it is even more effective in subject/patients having a high VEGFR2/VEGF expressing (or, more precisely, patients/subjects having or suffering from tumors characterized by a high VEGFR2/VEGF expression). For example, it is thought that the tumor shrinkage will be observed in subjects/patients having a higher VEGFR2 and/or VEGF expression (e.g. at least 1.5 fold higher) compared to a control as defined herein (like the "H1650" control). Thus, cancer patients susceptible to cancer treatment (in particular chemotherapy as desribed and defined herein such as treatment with a selective VEGFR-2 inhibitor (e.g. Vandetanib) and a MEK inhibitor (e.g. PD0325901), i.e. the herein provided combination therapy) can be identified based on the VEGFR2/VEGF expression level.

Accordingly, the present invention relates to a method for determining the susceptibility of a mammalian tumor or cancer cell to cancer treatment, in particular to the herein provided combination therapy. In accordance therewith, the present invention relates to a method for determining the susceptibility of a mammalian tumor or cancer cell to a a selective inhibitor of VEGFR-2 tyrosine kinase as defined herein and an inhibitor of MAP kinase kinase (MEK) as defined herein, said method comprising evaluating the expression level of VEGF and/or VEGFR2, wherein said expression level is indicative of the susceptibility of said cell to said inhibitors.

In a further aspect, the present invention relates to a method for predicting the susceptibility of an individual to cancer treatment, in particular to the herein provided combination therapy. In accordance therewith, the present invention relates to a method for predicting the susceptibility of an individual to a selective inhibitor of VEGFR-2 tyrosine kinase as defined herein and an inhibitor of MAP kinase kinase (MEK) as defined herein, said method comprising evaluating the expression level of VEGF and/or VEGFR2 in a sample of an individual suffering from cancer, suspected to suffer from cancer, or being prone to suffer from cancer, wherein said expression level is indicative of a susceptible individual to said inhibitors.

The determination of the activity of the autocrine loop as reflected in the expression level of VEGFR2 and/or VEGF and/or the phosphorylation level of VEGFR2 may be conducted by measuring VEGF bound to VEGFR2 by the use of antibodies. Said activity (e.g. expression level of VEGFR2 and/or VEGF and/or the phosphorylation level of VEGFR2) may be determined by measuring the amount of VEGF and/or VEGFR2 expression (and/or VEGFR2 phosphorylation status) on/of tumor cells/tumor tissue and the like using the following exemplary antibodies by antibody staining (e.g. in immunohistochemistry, immunofluorescence, flow cytometry, immunoblotting, etc.): Anti-VEGF (Vascular endothelial growth factor, VEGFA, VEGF-A), PhosphoDetect™ Anti-VEGFR2 (pTyr1054/1059) Rabbit pAb, PhosphoDetect™ Anti-VEGFR2 (pTyr1214), Anti-phospho-VEGFR2/Flk-I/KDR (Tyr1223), Anti-phospho-KDR/Flk-I/VEGFR2 (Tyr1054), Anti-phospho VEGFR2/Flk-I/KDR (Ser1188), Anti-phospho-KDR/Flk-I/VEGFR2 (Tyr1214), Anti-phospho-KDR/Flk-I/VEGFR2 (Tyr1059) und Anti-KDR/Flk-I/VEGFR2. These antibodies are commercially available at e.g. Millipore, Billerica, MA 01821, US.

The expression level of VEGF and/or VEGFR2 and/or the phosphorylation status of VEGFR2 is indicative of the susceptibility of said cell to the inhibitor or of a susceptible individual to the cancer treatment, in particular to the one or more selective inhibitors of VEGFR-2 tyrosine kinase as defined herein and the one ore more inhibitors of MAP kinase kinase (MEK) (i.e. to the herein provided combination therapy). All explanations and definitions given herein above in

context of the combination therapy i.e. the (pharmaceutical) compositions comprising one or more selective inhibitors of VEGFR-2 tyrosine kinase as defined herein and one or more inhibitors of MAP kinase kinase (MEK) as defined herein, apply mutatis mutandis, in the this context. Said composition is preferably for use in the treatment of cancer, like lung cancer, such as non-small cell lung cancer, lung adenocarcinoma or small cell lung cancer. Preferably, said composition is a pharmaceutical composition optionally comprising pharmaceutically acceptable excipients and/or carriers. Also in this context, the determination or prediction of the susceptibility to N-[(2R)-2,3-dihydroxypropoxy]-3,4-difluoro-2-[(2-tluoro-4-iodophenyl)amino]-benzamide (PD0325901) as shown in formula (VII) or a pharmaceutically acceptable salt, solvate or hydrate thereof in combination therapy with N-(4-bromo-2-fluorophenyl)-6-methoxy-7-[(1-methylpiperidin-4-yl)methoxy]quinazolin-4-amine (Vandetanib/Zactima/ZD6474) as shown in formula (I) or a pharmaceutically acceptable salt, solvate or hydrate thereof is very preferred.

Furthermore, the definitions and explanations given herein in context of the expression level of VEGF and/or VEGFR2 (also in respect of, for example, the samples to be assessed) apply, mutatis mutandis, to the phosphorylation status of VEGFR2. For example, the cancer to be treated in accordance with the present invention may be characterized by or associated with phosphorylation of VEGFR2, in particular increased phosphorylation of VEGFR2 (i.e. the phosphorylation/phosphorylation status of VEGFR2 in a tumor/cancer sample is (preferably statistically significantly) higher compared to a control sample. The phosphorylation status may, for example, be evaluated at known or yet to be identified phosphorylation sites of VEGFR2. The phosphorylation status of VEGFR2 at one or more phosphorylation sites may, for example, be at least 1.5-fold, 2.0-fold, 2.5-fold, 3.0-fold, 3.5-fold, 4.0-fold, or at least 5-fold or even more increased at one or more phosphorylation sites in comparison to the control. As explained herein above in context of the increased VEGF and/or VEGFR expression level, an increased phosphorylation status indicates susceptibility of a tumor cell or subject/patient/individual having or suffering from cancer, being suspected to suffer from cancer, or being prone to suffering from cancer, to the herein provided combination therapy, such as treatment with vandetanib and PD0325901.

The expression level of VEGF and/or VEGFR2 or the phosphorylation status ofVEGFR2 may be determined by routine measures using a sample of an individual suspected to suffer from or being prone to suffer from cancer. Said sample may, for example, be obtained by (a) biopsy (biopsies). Preferably, said sample is obtained from a patient suspected to suffer from or being prone to suffer from solid cancer / solid tumor. Preferably, the cancer is characterized by VEGF and/or VEGFR2 expression, in particular VEGF and/or VEGFR2 overexpression (i.e. the expression level of VEGF and/or VEGFR2 in a tumor/cancer sample is (preferably statistically significantly) higher compared to a control sample as explained in more detail further below. The solid cancer may be lung cancer, such as, non-small cell lung cancer, lung adenocarcinoma or small cell lung cancer. Also other cancers/tumors that are characterized by or associated with VEGF and/or VEGFR2 expression as described herein can be treated in accordance with the present invention, such as tyroid cancer (such as medullary thyroid cancer), pancreatic cancer, colorectal cancer, colon cancer, rectum cancer, breast cancer, head and neck cancer, ovarian cancer, endometrial cancer, gastrointestinal cancer (including gastric and esophageal cancer), renal cell cancer, urinary tract carcinomas, prostate cancer, lymphomas, melanomas, brain tumors/brain cancer, pediatric tumors and sarcomas, esophagus cancer, stomach cancer, liver cancer, biliary tract cancer, kidney cancer, cervix cancer, overy cancer, uterus cancer, skin cancer (including melanoma, uveal melanoma, and squamous cell carcinoma), gallbladder cancer, pancreas cancer, genital cancer (including vaginal carcinoma and penile carcinoma), embryonal carcinomas, hepatocellular carcinoma, neuroendocrine carcinomas, and/or cancer of unknown primary (CUP syndrome). The cancer to be treated may also include haematological malignancies such as leukemias, multiple myeloma and/or lymphoma. Preferably, the cancer is lung cancer, particularly lung adenocarcinoma, non-small cell lung cancer or small cell lung cancer and especially non-small cell lung cancer.

It is preferred herein that said sample is obtained from (a) tumor(s) and, accordingly, is (a) tumor cell(s) or (a) tumor tissue(s) suspected of being for example a lung tumour, and the like. A person skilled in the art is in the position to identify such tumors and/or individuals/patients suffering from corresponding cancer using standard techniques known in the art and methods disclosed herein.

Preferably, the cell(s), tissue(s) or cell culture(s) to be contacted with/exposed to an inhibitor comprise/are derived from or are (a) tumor cell(s). The tumor cells may, for example, be obtained from a biopsy, in particular a biopsy/biopsies from a patient/subject suffering from cancer or, though less preferred a patient/subject being prone to suffer from cancer. It is preferred herein that said subject is a human. The term "mammalian tumor cell(s)" used herein refers to (a) tumor cell(s) which is derived from or is a tumor cell from a mammal, the term mammal being derived herein below. As described herein above in respect of "cell(s)", "tissue(s)" and "cell culture(s)" the "mammalian tumor cells" may be obtained from a biopsy, in particular a biopsy/biopsies from a patient/subject suffering from cancer or, though less preferred a patient/subject being prone to suffer from cancer. The term "tumor cell" also relates to "cancer cells". Generally, said tumor cell or cancer cell may be obtained from any biological source/organism, particularly any biological source/organism, suffering from the above-mentioned cancer.

Preferably, the (tumor) cell(s) or (cancer) cell to be contacted is (are) obtained/derived from an animal. More preferably, said (tumor)/(cancer) cell(s) is (are) derived from a mammal. The meaning of the terms "animal" or "mammal" is well known in the art and can, for example, be deduced from Wehner und Gehring (1995; Thieme Verlag). Non-limiting

examples for mammals are even-toed ungulates such as sheep, cattle and pig, odd-toed angulates such as horses as well as carnivors such as cats and dogs. In the context of this invention, it is particularly envisaged that samples are derived from organisms that are economically, agronomically or scientifically important. Scientifically or experimentally important organisms include, but are not limited to, mice, rats, rabbits, guinea pigs and pigs.

The tumor cell(s) may also be obtained from primates which comprise lemurs, monkeys and apes. The meaning of the terms "primate", "lemur", "monkey" and "ape" is known and may, for example, be deduced by an artisan from Wehner und Gehring (1995, Thieme Verlag). As mentioned above, the tumor or cancer cell(s) is (are) most preferably derived from a human being suffering from the above-mentioned cancer. In context of this invention particular useful cells, in particular tumor or cancer cells, are, accordingly, human cells. These cells can be obtained from e.g. biopsies or from biological samples but the term "cell" also relates to in vitro cultured cells.

As mentioned, it has been found herein that the expression level of VEGF and/or VEGFR2 can be used to evaluate whether an individual or a cell and the like respond to cancer treatment and particularly to the herein provided combination therapy. Accordingly, an increase in the expression level as compared to the control is indicative of the susceptibility of said cell or a susceptible individual to cancer treatment, particularly to the herein described one or more selective inhibitors of VEGFR-2 tyrosine kinase as defined herein and the one ore more inhibitors of MAP kinase kinase (MEK) (combination therapy). Preferably, the cancer to be treated is characterized by or associated with VEGF and/or VEGFR2 expression, in particular VEGF and/or VEGFR2 overexpression (i.e. the expression level of VEGF and/or VEGFR2 in a tumor/cancer sample is (preferably statistically significantly higher) compared to a control sample, such as a "non-responder" or "non susceptible" control (e.g. cell line H1650 as used in Example 2). Preferably, the expression level of VEGF and/or VEGFR2 is at least 1.1-fold, 1.2-fold, 1.3-fold, 1.4-fold, more preferably at least 1.5-fold, 1.6 -fold, 1.7 - fold, 1.8-fold, 1.9-fold, 2.0-fold, 2.1-fold, 2.2-fold, 2.3-fold, 2.4-fold, even more preferably at least 2.5-fold, 2.6-fold, 2.7-fold 2.8-fold, 2.9-fold, at least 3.0-fold, 3.1-fold, 3.2-fold, 3.3-fold, 3.4-fold, 3.5-fold, 4.0-fold, and yet more preferably at least 5-fold, 5.5-fold, 6-fold, or even more, like 7-fold, 8-fold, 9-fold, 10-fold, 11-fold, 12-fold, 13-fold, 14-fold, 15-fold or more increased in comparison to the control.

It is envisaged herein that the expression level may be reflected in the activity of the gene product/protein. Accordingly, also the activity of VEGF and/or VEGFR2 can be measured and evaluated in addition or in the alternative to the expression level of integrin β4 in accordance with the present invention. A person skilled in the art is aware of corresponding means and methods for detecting and evaluating the VEGF and/or VEGFR2 expression level and/or activity. Exemplary methods to be used include but are not limited to molecular assessments such as Western Blots, Northern Blots, Real-Time PCR and the like. Such methods have been described herein above in detail.

The expression level of VEGF and/or VEGFR2 may be the mRNA expression level of VEGF and/or VEGFR2. If the gene product is an RNA, in particular an mRNA (e.g. unspliced, partially spliced or spliced mRNA), determination can be performed by taking advantage of northern blotting techniques, in situ hybridization, hybridization on microarrays or DNA chips equipped with one or more probes or probe sets specific for mRNA transcripts or PCR techniques, like, quantitative PCR techniques, such as Real time PCR. These and other suitable methods for binding (specific) mRNA are well known in the art and are, for example, described in Sambrook and Russell (2001, loc. cit.). A skilled person is capable of determining the amount of the component, in particular said gene products, by taking advantage of a correlation, preferably a linear correlation, between the intensity of a detection signal and the amount of the gene product to be determined.

The expression level may be the protein expression level of VEGF and/or VEGFR-2. Quantification of the protein expression level can be performed by taking advantage of the well known techniques such as western blotting techniques, immunoassays, gel- or blot-based methods, IHC, mass spectrometry, flow cytometry, FACS and the like. Generally, a person skilled in the art is aware of methods for the quantitation of (a) polypeptide(s)/protein(s). Amounts of purified polypeptide in solution can be determined by physical methods, e.g. photometry. Methods of quantifying a particular polypeptide in a mixture may rely on specific binding, e.g of antibodies. Specific detection and quantitation methods exploiting the specificity of antibodies comprise for example immunohistochemistry (*in situ*). Western blotting combines separation of a mixture of proteins by electrophoresis and specific detection with antibodies. Electrophoresis may be multi-dimensional such as 2D electrophoresis. Usually, polypeptides are separated in 2D electrophoresis by their apparent molecular weight along one dimension and by their isoelectric point along the other direction. Alternatively, protein quantitation methods may involve but are not limited to mass spectrometry or enzyme-linked immunosorbant assay methods.

Also the use of high throughput screening (HTS) is envisaged in the context of the present invention, in particular the screening methods of cell(s), tissue(s) and/or cell culture(s) for susceptibility to cancer treatment, particularly to the herein described one or more selective inhibitors of VEGFR-2 tyrosine kinase as defined herein and the one ore more inhibitors of MAP kinase kinase (MEK) (combination therapy).

Suitable (HTS) approaches are known in the art and have also been described above in detail. A person skilled in the art is readily in the position to adapt such protocols or known HTS approaches to the performance of the methods of the present invention. Such assays are usually performed in liquid phase, wherein for each cell/tissue/cell culture to

be tested at least one reaction batch is made. Typical containers to be used are micro titer plates having for example, 384, 1536, or 3456 wells (i.e. multiples of the "original" 96 reaction vessels). Robotics, data processing and control software, and sensitive detectors, are further commonly used components of a HTS device. Often robot system are used to transport micro titer plates from station to station for addition and mixing of sample(s) and reagent(s), incubating the reagents and final readout (detection). Usually, HTS can be used in the simultaneous preparation, incubation and analysis of many plates. The assay can be performed in a single reaction (which is usually preferred), may, however, also comprise washing and/or transfer steps. Detection can be performed taking advantage of radioactivity, luminescence or fluorescence, like fluorescence-resonance-energytransfer (FRET) and fluorescence polarisation (FP) and the like. The biological samples described herein can also be used in such a context. In particular cellular assays and in vivo assays can be employed in HTS. Cellular assays may also comprise cellular extracts, i.e. extracts from cells, tissues and the like. However, preferred herein is the use of cell(s) or tissue(s) as biological sample (in particular a sample obtained from a patient/subject suffering or being prone to suffer from cancer), whereas in vivo assays are particularly useful in the validation of potential inhibitors to be used herein. Depending on the results of a first assay, follow up assays can be performed by re-running the experiment to collect further data on a narrowed set (e.g. samples found "positive" in the first assay), confirming and refining observations.

As used in context of the methods of the present invention, a non-limiting example of a "control" is preferably a "non-responder" or "non susceptible" control, for example a sample/cell/tissue obtained from one or more healthy subjects or one or more patients that suffer from a cancer/tumor and are known to be not responsive to cancer treatment, particularly to the herein described one or more selective inhibitors of VEGFR-2 tyrosine kinase as defined herein and the one ore more inhibitors of MAP kinase kinase (MEK) (combination therapy). Another example for a "non-responder" control is a cell line/sample/cell/tissue that shows no response to cancer treatment, particularly to the herein described one or more selective inhibitors of VEGFR-2 tyrosine kinase as defined herein and the one ore more inhibitors of MAP kinase kinase (MEK) (combination therapy) in an ex-vivo test. Another non-limiting example of a "control" is an "internal standard", for example a mixture of purified or synthetically produced proteins and/or peptides, where the amounts of each protein/peptide is gauged by using the "non-responder"/ "non susceptible" control described above. In particular, this mixture can contain the the VEGFR2 and/or VEGF protein as desribed and defined herein.

A further non-limiting example of a "control" may be a "healthy" control, for example a sample/cell/tissue obtained from a healthy subject or patient that is not suffering from a cancer/tumor or a cell obtained from such a subject. In accordance with the above, the reference or control status e.g. of VEGFR2 and/or VEGF is that determined in (a sample of) the corresponding healthy control subject/patient, i.e. it is the "normal" status of e.g. VEGFR2 and/or VEGF. The control may also be a sample/cell/tissue obtained from the individual or patient suspected of suffering from the cancer provided that the sample/cell/tissue does not contain tumor or cancer cells. In a further alternative, the "control" may be a sample/cell/tissue obtained from an individual or patient suffering from the cancer, that has been obtained prior to the development or diagnosis of said cancer.

In a further aspect, the present invention relates to the use of a nucleic acid or antibody capable of detecting the expression level of VEGF and/or VEGFR-2 for determining the susceptibility of a cancer cell or a susceptible individual to a tyrosine kinase inhibitor as defined herein and an inhibitor of MAP kinase kinase (MEK) as defined herein. Preferably, the oligonucleotide(s) is (are) about 15 to 100 nucleotides in length. A person skilled in the art is, based on his general knowledge and the teaching provided herein, easily in the position to identify and/or prepare (a) an oligo- or polynucleotide capable of detecting the expression level of VEGF and/or VEGFR-2. In particular these oligo- or polynucleotides may be used as probe(s) in the detection methods described herein. A skilled person will know, for example, computer programs which may be useful for the identification of corresponding probes to be used herein. For example, VEGF nucleic acid sequence (SEQ ID NO: 7) and/or the VEGFR-2 nucleic acid sequence (SEQ ID NO: 1) may be used in this context for identifying specific probes for detecting the expression level of VEGF and/or VEGFR-2, respectively. Exemplary nucleic acid sequences are available on corresponding databases, such as the NCBI database (www.ncbi.nlm.nih.gov/sites/entrez).

Furthermore, a composition is provided herein which is a diagnostic composition further comprising, optionally, means for detection/determining/evaluating the expression level of VEGF and VEGFR-2. Such means for detection, are, for example, the above-described nucleotides and/or antibodies. Accordingly, the present invention relates to such means (e.g. such nucleotides and/or antibodies) for the preparation of a diagnostic composition for determining the susceptibility to a selective inhibitor of VEGFR-2 tyrosine kinase as defined herein and an inhibitor of MAP kinase kinase as defined herein.

In an alternative aspect, the present invention relates to such means for detection (e.g the above-described nucleotides and/or antibodies and/or the "binding molecules" described below in context of the kit to be used in accordance with the present invenion) for use in determining the susceptibility to a selective inhibitor of VEGFR-2 tyrosine kinase as defined herein and an inhibitor of MAP kinase kinase as defined herein. Preferably, the present invention relates to (an) antibody/antibodies for use in determining the susceptibility to a selective inhibitor of VEGFR-2 tyrosine kinase as defined herein and an inhibitor of MAP kinase kinase as defined herein. In a further aspect, the present invention provides

VEGF and VEGFR-2 s defined herein for use in determining the susceptibility to a selective inhibitor of VEGFR-2 tyrosine kinase as defined herein and an inhibitor of MAP kinase kinase as defined herein or for use in detecting an individual responsive to a selective inhibitor of VEGFR-2 tyrosine kinase as defined herein and an inhibitor of MAP kinase kinase as defined herein. In one aspect, the present invention provides for the use of such means for detection (e.g the above-described nucleotides and/or antibodies) for the preparation of a diagnostic kit for use in determining the susceptibility to a selective inhibitor of VEGFR-2 tyrosine kinase as defined herein and an inhibitor of MAP kinase kinase as defined herein.

The phrases "detecting susceptibility to a selective inhibitor of VEGFR-2 tyrosine kinase as defined herein and an inhibitor of MAP kinase kinase as defined herein " or "diagnosing susceptibility to a selective inhibitor of VEGFR-2 tyrosine kinase as defined herein and an inhibitor of MAP kinase kinase as defined herein " or determining susceptibility to a selective inhibitor of VEGFR-2 tyrosine kinase as defined herein and an inhibitor of MAP kinase kinase as defined herein" as used herein refer to determining susceptibility to a selective inhibitor of VEGFR-2 tyrosine kinase as defined herein and an inhibitor of MAP kinase kinase as defined herein in a subject, preferably in a human. Such a "detection"/"diagnosis"/"determination" may, in particular, comprise (i) the diagnosis for curative purposes stricto sensu representing the deductive medical or veterinary decision phase as a purely intellectual exercise, (ii) the preceding steps which are constitutive for making that diagnosis, and (iii) the specific interactions with the human or animal body which occur when carrying those out among these preceding steps which are of a technical nature.

Furthermore, the present invention also relates to a kit useful for carrying out the herein provided methods, the kit comprising a nucleic acid or an antibody capable of detecting the expression level of VEGF and/or VEGFR-2 (and/or (an) antibody capable of detecting the phosphorylation status of VEGFR-2). Also envisaged herein is the use of the herein described kit for carrying out the herein provided methods. Said kit useful for carrying out the methods and uses described herein may comprise oligonucleotides or polynucleotides capable of determining the expression level of VEGF and/or VEGFR-2. For example, said kit may comprise (a) compound(s) required for specifically determining the expression level of VEGF and/or VEGFR-2. Moreover, the present invention also relates to the use of (a) compound(s) required for specifically determining the expression level of VEGF and/or VEGFR-2 for the preparation of a kit for carrying out the methods or uses of this invention. On the basis of the teaching of this invention, the skilled person knows which compound(s) is (are) required for specifically determining the expression level of VEGF and/or VEGFR-2. For example, such compound(s) may be (a) "binding molecule(s)", like, for example, (a) "binding molecule(s)" Particularly, such compound(s) may be (a) (nucleotide) probe(s), (a) primer(s) (pair(s)), (an) antibody(ies) and/or (an) aptamer(s) specific for VEGF gene and/or VEGFR-2 gene or for a gene product thereof. The kit (to be prepared in context) of this invention may be a diagnostic kit.

The kit (to be prepared in context) of this invention or the methods and uses of the invention may further comprise or be provided with (an) instruction manual(s). For example, said instruction manual(s) may guide the skilled person (how) to determine the (reference/control) expression level of VEGF and/or VEGFR-2 or (how) to diagnose/ determine susceptibility to a selective inhibitor of VEGFR-2 tyrosine kinase and an inhibitor of MAP kinase kinase. Particularly, said instruction manual(s) may comprise guidance to use or apply the herein provided methods or uses. The kit (to be prepared in context) of this invention may further comprise substances/chemicals and/or equipment suitable/ required for carrying out the methods and uses of this invention. For example, such substances/chemicals and/or equipment are solvents, diluents and/or buffers for stabilizing and/or storing (a) compound(s) required for specifically determining the expression level of VEGF and/or VEGFR-2.

The present invention relates to a pharmaceutical composition comprising a selective inhibitor of VEGFR-2 tyrosine kinase as defined herein and an inhibitor of MAP kinase kinase (MEK) as defined herein for use in the treatment of cancer in an individual or patient identified by the herein provided methods. Also envisaged herein is a selective inhibitor of VEGFR-2 tyrosine kinase as defined herein and an inhibitor of MAP kinase kinase (MEK) as defined herein for use in the treatment of cancer in an individual or patient identified by the herein provided methods. The present invention also relates to the use of a VEGFR-2 tyrosine kinase as defined herein and an inhibitor of MAP kinase kinase (MEK) as defined herein for the preparation of a pharmaceutical composition for the treatment of a cancer patient identified by the herein provided methods. The present invention also provides a method for the treatment of cancer comprising administering an effective amount of a selective inhibitor of VEGFR-2 tyrosine kinase as defined herein and an inhibitor of MAP kinase kinase (MEK) as defined herein to an individual identified by the method provided herein in need of such a treatment. Preferably, the individual or patient is a human. All explanations given herein above in context of the treatment of cancer with a selective inhibitor of VEGFR-2 tyrosine kinase as defined herein and an inhibitor of MAP kinase kinase (MEK) as defined herein, apply, mutatis mutandis, in context of the treatment of this particularly susceptible patient group.

As explained above, it has been found that subjects/individuals/patients suffering from a tumor/cancer which is characterized by or associated with VEGF and/or VEGFR2 expression are highly responsive/susceptible to the herein provided combination therapy. In these subjects, the combination therapy shows a more than additive effect, especially a reduction in the tumor volume during the course of the treatment, compared with the respective monotherapies of only

a selective inhibitor of VEGFR-2 tyrosine kinase as defined herein or of only an inhibitor of MAP kinase kinase (MEK). Accordingly, the present invention provides a method for predicting or determining the susceptibility of an individual to a selective inhibitor of VEGFR-2 tyrosine kinase as defined herein and an inhibitor of MAP kinase kinase (MEK) as defined herein, said method comprising evaluating the expression level of VEGF and/or VEGFR2 in a sample of an individual suffering from cancer, suspected to suffer from cancer, or being prone to suffer from cancer, wherein said expression level is indicative of a susceptible individual to said inhibitors. The individuals identified by said method as being susceptible can be effectively treated with the combination therapy. In these individuals, the combination therapy will show a more than additive effect as explained above. Accordingly, the present invention relates to a pharmaceutical composition comprising a selective inhibitor of VEGFR-2 tyrosine kinase as defined herein and an inhibitor of MAP kinase kinase (MEK) as defined herein for use in the treatment of cancer in an individual or patient identified by the herein provided methods, wherein the combination therapy shows a more than additive effect, especially a reduction in the tumor volume during the course of the treatment, compared to the respective monotherapies of only a selective inhibitor of VEGFR-2 tyrosine kinase as defined herein or of only an inhibitor of MAP kinase kinase (MEK).

The present invention is further illustrated by reference to the following non-limiting figures and examples. Unless otherwise indicated, established methods of recombinant gene technology were used as described, for example, in Sambrook, Russell "Molecular Cloning, A Laboratory Manual", Cold Spring Harbor Laboratory, N.Y. (2001) ) which is incorporated herein by reference in its entirety.

The Figures show:

**Figure 1. A VEGF-VEGFR2 feed-forward loop in tumor cells boosts VEGF secretion. A** H1975 cells were engrafted subcutaneously in nude mice; mice with established tumors were treated with ZD6474 and PET imaging was performed at the indicated time points after treatment. Representative results are shown from one mouse. Left panels, results obtained by FLT-PET. Right, results obtained with MET-PET. **B** Tumor size of subcutaneously grown H1975 tumors was determined at the indicated time points under treatment with either vehicle or ZD6474. In **C** Secretion of VEGF by H1975 and H441 cells was determined in vitro by ELISA following stimulation with increasing amounts of recombinant VEGF (upper panels). H1975 and H441 cells were stimulated with VEGF (V) and pretreated with the indicated compounds (Z=Zactima 1$\mu$M, R=Rapamycin, 250nM, T=Torin 1, 250nM) (lower panels). Impact on activation of downstream signaling was determined by immunoblotting employing the indicated phospho-specific antibodies **D. F** H1975 cells were stably transduced with retroviruses expressing a drug-resistant version of VEGFR2 (T916M) that prevents binding of ZD6474. The resulting mutants (H1975$^{KDRmut}$) were treated with VEGF and the indicated dose of ZD6474 and phosphorylation of VEGFR2 was determined by immunoblotting (upper panels). Stably transduced H1975 cells expressing the ZD6474-resistant mutant ("mut") or the wildtype control ("wt") were injected into nuce mice and treated with ZD6474 or vehicle on day 1 after tumor cell injections.

**Figure 2. Requirement of autocrine VEGF:VEGFR2 signaling for induction of angiogenesis and tumor formation in vivo**

**A** H1975 cells (wt) were stably transduced with lentiviral shRNA vectors targeting VEGFR2 ((VEGFR2-KD) or with empty vector control (eV)). Knockdown efficiency was determined by western blotting (upper panel, Flt-1; lower panel, VEGFR2). **B** Stable cell lines were cultured over time and quantified under normoxic contions or exposed to hypoxia (1% $O_2$) and **(C)** VEGF secretion was determined over time by ELISA. **D** Stable cell lines were injected into nude mice and tumor growth was monitiored over time. **D** Tumors were harvested and stained for pan VEGF, CD31, human VEGF (employing FITC-labeled Avastin), human VEGF complexed with VEGFR2, Ki67 and pErk. **E** Stable cell lines were injected into nude mice and uptake of $^{11}$O-labeled H20 was determined by PET.

**Figure 3. The VEGF:VEGFR2 feed forward loop in primary human lung adenocarcinomas**

Human adenocarcinomas were immunfluorescently stained to show co-expression of VEGF and VEGFR2 by the same tumor cell population. In A a representative case is shown. B Tissue microarrays of 117 lung adenocarcinomas were stained with antibodies recognizing human VEGFR2, VEGF and CD31. Association of staining intensity of the different antibodies in the same cohort is given in chi-square tables. Levels of significance were determined using Fischer's exact test. In C a representative patient (1) with an high angiogenic phenotype represented by high VEGF: VEGFR2 staining and high CD31 positive cells. In contrast, patient (2) presents a low angiogenic phenotype with only moderate VEGF:VEGFR2 positive tumor cells corresponding to low density of CD31 positive cells.

**Figure 4. ZD6474 treatment does not affect tumor cell proliferation**

H1975 and H441 cells were stained for BRDU using flow cytometry after treatement with ZD6474 or vehicle. Incorporation of BRDU as marker for cellular replication is not changed after treatment with ZD6474.

**Figure 5. Reduced MET uptake during ZD6474 treatment in vivo**

In A ZD6474 treatment reduces MET uptake in PET whereas FLT uptake slightly increased during treatment. Stable silencing of VEGFR2 does not alter VEGFR1 levels in H441 cells.

**Figure 6. ZD6474 inhibits tumor VEGF secretion under hypoxic conditions**
In A H441 and H1975 cells were treated with ZD6474 (1μM) or DMSO and transferred to hypoxia (1% 02). VEGF levels were assessed by ELISA. In line with the feed-forward loop assumption ZD6474 inhibits VEGF secretion. In B the gatekeeper mutant VEGFR2$^{V916M}$ is illustrated. This gatekeeper mutant in VEGFR2 prevents ZD6474 binding to the tyrosine kinase pocket of VEGFR2.

**Figure 7. Silencing of VEGFR2 in H441 cells dramatically inhibits tumor growth**
A H1975 cells (wt) were stably transduced with lentiviral shRNA vectors targeting VEGFR2 ((VEGFR2-KD) or with empty vector control (eV)). Knockdown efficiency was determined by western blotting. B Stable cell lines were injected into nude mice and tumor growth was monitiored over time.

**Figure 8. ZD6474 treatment reduces microvessel density**
H1975 cells were injected into nude mice and treated with either ZD6474 (75mg/kg) or vehicle. Tumors were resected. The images show reduced tumor vessel density after ZD6474 treatment.

**Figure 9. Expression of VEGF/VEGFR2 on human tumor cells is related with microvessel density (CD31 positive endothelial cells)**
Human adenocarcinomas were immunfluorescently stained to show co-expression of CD31, VEGF and VEGFR2 by the same tumor cell population. Three representative cases are shown. Patient 1 with a non-angiogenic phenotype refelected by low CD31 expression and low expression of VEGFR2 and VEGF. In contrast, human lung tumors with an angiogenic phenotype as patient 3 present high expression levels of VEGF and VEGFR2 on tumor cells.

**Figure 10.**
A H1975 and H441 and H1650 cells were engrafted subcutaneously in nude mice; mice with established tumors were treated with ZD6474, PD0325901, vehicle and combined ZD6474 / PD0325901. Tumor size of subcutaneously grown H1975 / H441 / H1650 tumors was determined at the indicated time points. Resected xenografts of H1975 and H441 were immunfluorescently stained IRS-1, perk (B / C). A representative tumor is shown.

**<u>Example 1. A tumor cell-autonomous autocrine VEGF:VEGFR2 feed-forward loop triggers the angiogenic switch in lung cancer.</u>**

**Material and Methods**

*Cell lines and reagents*

NSCLC cell lines H441 and H1975 and H1650 **[?]** were obtained from own and other cell culture collections and were maintained as described previously (Sos et al, J Clin Invest. 2009 Jun;119(6):1727-40). ZD6474 was purchased from Astra-Zeneca, Rapamycin and Torinl were purchased from LC Laboraties, Woburn, MA 01801, US and Tocris bioscience. Compounds were dissolved in DMSO or vehicle solution and stored at -20°C.

*Western blotting*

Western blotting was performed as described previously (Sos, loc. cit.). The following antibodies were used: β-actin (clone C4) (MPBioscience, USA), p-AKT (S473), pS6K, S6K, AKT, p-ERK, ERK, VEGFR-2, VEGFR-1 (Cell Signaling Technology, USA), anti-rabbit-HRP, anti-mouse-HRP-antibody (Millipore, Germany).

*Lentiviral RNAi and retroviral expression*

The V916M mutation was introduced into H1975 cells using pBABEpuro - by site - directed mutagenesis. Replication - incompetent retroviruses were produced by contransfection with the pCL - ampho plasmid in HEK 293T cells. Hairpins targeting the different genes were ordered from Sigma (www.sigmaaldrich.com). Replication - incompetent lentiviruses were produced from pLKO.1 - Puro based vectors by contransfection with Δ8.9 and pMGD2 in 293T cells (www.broadinstitute.org/rnai/trc/lib). After transduction cells were selected with puromycin.

*ELISA assay*

Cells were either treated with the indicated compounds or DMSO alone. After 4 hours cells were stimulated with 40 ng/ml of VEGF165, or incubated in medium containing DMSO only. Cells were then washed twice with PBS and supplemented with serum-free medium and allowed to secrete VEGF for 48 hours. Secretion of VEGF into cell culture supernatants was measured by Human VEGF Quantikine Kit (R&D Systems) according to manufacturer's instructions.

*PET imaging*

Tumor bearing mice were investigated using a FOCUS microPET scanner (Concord Microsystems, Inc., Knoxville, TN). [$^{18}$F]FLT and [11C]MET synthesis were performed as described previously [17,18]. No-carrier-added [$^{18}$F] FLT, [11C]MET and [11O]H2O PET were administered i.v. (tail vein) into experimental animals with a dose of 200 μCi/ mouse of [18F]FLT, 400 μCi/mouse of [11C]MET and 900 μCi/mouse [11O]H2O. [$^{18}$F]FLT PET images were performed 60 min after injection, [11C]MET PET 20 min after i. v. injection and [11O]H2O PET dynamically during injections. Data evaluation was based on a volume of interest (VOI) analysis of the entire tumor. For data analysis we used the maximal voxel radioactivity within the tumors. To determine the uptake ratio we chose the mediastinum as reference since we observed constant uptake for [18F]FLT and [11C]MET in this region. We used the heart as reference for calculation of the [11O]H2O. All data were decay corrected.

*Flow cytometry*

For detection of apoptosis, the BRDU Detection Kit (BD Biosciences, Germany) was used. Cells were plated in 6-well plates and after 24 hours of incubation, cells were treated with the ZD6474 for further 4 hours. Cells were then harvested using 0.05% Trypsin EDTA solution, washed with cold PBS and then resuspended in BRDU binding buffer. Finally, cells were stained with BRDU and PI and incubated in the dark for 20min before analysis on a FACS Canto Flow Cytometer (BD Biosciences, Germany). Results were calculated using FACS Diva Software v5.0.

*RNAi and stable transduction*

Replication incompetent retroviruses were produced from pBabe-based vectors in HEK 293T cells (Orbigen, USA) using TRANS-IT (Mirus, USA). Replication incompetent lentiviruses were produced from pLKO.1-Puro based vectors (www.broad.mit.edu/genome_bio/trc/) in HEK 293T cells using TRANS-IT (Mirus, USA). Cells were transduced in the presence of polybrene. After 24h medium was changed and pooled stable cell lines were selected with puromycin.

*Xenograft mouse models*

All animal procedures were approved by the local animal protection committee and the local authorities. H1975, H441 and H1650 cells (5x$10^6$ cells) were injected subcutaneously into male nude mice. Tunors were treated either with injections of the tumor cells or treatment was started when tunors had reached a size of abput 50$mm^3$. Mice were treated daily by oral gavage of ZD6474 (50 mg/kg, solved in vehicle), PD0325901 (12mg/kg, dissolved in propylene glycol:water (1:1)), the combination of ZD6474 (50 mg/kg) and PD0325901 (12mg/kg) or vehicle detergent alone. Tumor size of tumors expressing high levels of VEGFR2 (H441, H1975) and tumor with low expression of VEGFR2 (H1650) were monitored measuring perpendicular diameters.

*Tumor Samples*

All tumor samples stem from the CIO Biobank at the Institute of Pathology, University of Bonn, Germany. All tumors were clinically and pathologically identified as being the primary and only neoplastic lesion and classified according to World Health Organization (WHO) guidelines (Brambilla et al., 2001). Sections were stained and evaluated as previously described (Heukamp et al., 2006; Zimmer et al., 2008). Staining intensities were individually evaluated by three independent observers using a four-tier scoring system as described before (Zimmer et al., 2008). Immunofluorescence double-staining of tumor sections was performed as described (Friedrichs et al., 2007).

**Results**

The molecular mechanisms remain poorly defined that control the shift in the balance between anti- and pro-angiogenic factors, considered to *switch* tumor growth toward established tumors with autonomous blood supply[1]. An autocrine feed-forward loop in tumor cells was surprisingly identified herein wherein tumor-derived VEGF stimulates

VEGF production via VEGFR-2-dependent activation of mTor, providing a several-fold amplification of the initial pro-angiogenic signal. Disruption of this feed-forward loop by chemical perturbation or knockdown of VEGFR2 in tumor cells dramatically inhibited production of VEGF in vitro and in vivo. Remarkably, this disruption was sufficient to prevent tumor growth in vivo, although cellular proliferation was not reduced. While control tumors expressed high amounts of VEGF and were highly vascularized, VEGFR2-deficient tumor cells induced small tumors that lacked expression of VEGF and had only few tumor microvessels. Finally, expression of VEGFR2 correlated with expression of VEGF on tumor cells of primary human lung adenocarcinomas. Furthermore, these tumor cells stained positive with an antibody specifically recognizing VEGFR2-bound VEGF suggesting that this feed-forward loop supports angiogenesis in primary human cancers. Thus, a tumor cell-autonomous VEGF:VEGFR2 feed-forward loop provides a signal amplification required for establishment of fully angiogenic tumors in cancer, such as lung cancer.

Herein, the impact of VEGFR2 inhibition on whole tumors was analyzed by combining genetically controlled pharmacological perturbations with multimodal positron emission tomography (PET) imaging. The human lung cancer cell line H441, a KRAS-mutant cell line that is insensitive to EGFR inhibition (Figure 4) and the EGFR-mutant lung cancer cell line H1975 that is resistant to EGFR inhibition due to the presence of the T790M gatekeeper mutation of EGFR[7] (Figure 4). We treated mice engrafted with these two cell lines with the dual VEGFR2/EGFR inhibitor ZD6474 (Vandetanib/ZACTIMA), which has 40-fold lower activity against Flt1 (VEGF-R1)[8]. Thus, any therapeutic impact on subcutaneous tumor growth of these lines is primarily due to VEGFR2 inhibition and cannot be attributable to inhibition of EGFR. ZD6474 treatment completely abolished methionine uptake after seven days of treatment as determined by methyl-L-[$^{11}$C]-methionine PET (11C-MET; Fig. 1a, right panels, Figure 5), which correlates with VEGF levels in vivo[9]. By contrast, uptake of 18F-labeled 3'-Deoxy-3'-[$^{18}$F]-fluoro-L-thymidine (18F-FLT), a marker of proliferation[10], was at least slightly increased (Fig. 1a, Figure 5), suggesting that the cells continued to progress through the cell cycle. Remarkably, the reduction in methionine uptake extended through the entire diameter of the tumor and therefore likely affected tumor cells as well as endothelial cells (Fig. 1a). ZD6474 treatment inhibited tumor growth but failed to induce tumor regression (Fig. 1b). Thus, VEGFR2 inhibition inhibited VEGFR2-dependent signaling in tumor cells and endothelial cells without affecting cellular proliferation. It was assumed that VEGF bound VEGFR2 on tumor cells in an autocrine manner thus supporting angiogenesis by signal amplification.

Consistent with a feed-forward loop stimulating VEGF secretion in a VEGFR2-dependent fashion, VEGF levels were strongly induced following addition of exogenous VEGF in H1975 and H441 cells (Fig. 1c, upper panels) and this induction was blunted by treatment of cells with the VEGFR2 inhibitor ZD6474 (Fig. 1c, lower panels). Under hypoxic conditions induction of VEGF secretion was similarly inhibited by VEGFR2 inhibition (Figure 6), suggesting that this autocrine loop is also active in the physiological response to hypoxia. Supporting the hypothesis that VEGFR2-dependent secretion of VEGF was under the control of mTOR that regulates methionine uptake via the LAT-1 transporter[11], rapamycin treatment similarly blunted VEGF-induced VEGF secretion of tumor cells and combined VEGFR2 and mTOR inhibition had an at least additive inhibitory effect on VEGF secretion (Fig. 1d). However, while VEGF stimulation induced S6 phosphorylation (Fig. 1d), we found a consistent reduction in phosphorylation of Erk and of Akt (Fig. 1d) in H1975 cells. By contrast, in one of the two cell lines, phosphorylation of S6 coincided with the activation of PDK1 (Fig. 1d), providing an alternative route for mTOR activation[12]. Thus, in the setting of autocrine VEGF:VEGFR2 signaling, the slight reduction in tumor growth observed in response to VEGFR2 inhibition is likely independent of Erk-mediated proliferation. These results are in line with our PET experiments showing a continuous uptake of 18F-FLT (Fig. 1a). Finally, we confirmed that the observed ZD6474-mediated effects were due to inhibition of VEGFR2 in tumor cells by ectopically expressing a ZD6474 resistance mutation of VEGFR2 in H1975 (Figure 6b)[13]. This mutant abolished ZD6474-mediated VEGFR2-dephosphorylation and inhibition of VEGF secretion in vitro and suppressed the tumor-growth inhibiting effects of the compound in vivo (Fig. 1e). Thus, the ZD6474-mediated effects on tumor growth are predominantly due to inhibition of VEGFR2 on the tumor cells.

In order to validate this finding in an alternative approach, VEGFR2 was stably silenced by lentiviral shRNA in H1975 and H441 cells (Fig. 2a, Figure 7). In line with the FLT PET data, selective silencing of VEGFR2 by shRNA also showed no effect on tumor cell proliferation in vitro (Fig. 2b). By contrast, silencing of VEGFR2 dramatically reduced secretion of VEGF by tumor cells in response to hypoxia (Fig. 2c), thereby confirming that binding of VEGF to VEGFR2 amplifies VEGF secretion in a VEGFR2-dependent manner. Surprisingly, knockdown of VEGFR2 in tumor cells alone was sufficient to almost entirely abolish initiation of tumor growth in vivo (Fig. 2d and Figure 7b) suggesting that autocrine VEGF:VEGFR2 signaling in tumor cells is required for establishment of tumors in vivo. While the large tumors transduced with empty control vector ($H1975^{wt}$) exhibited a highly angiogenic phenotype with many CD31-positive blood vessels, the small residual tumors in which VEGFR2 had been stably silenced ($H1975^{VEGFR2KD}$) almost completely lacked blood vessels (Fig. 2e). Consistent with the tissue culture results, these tumors lacked expression of VEGF while $H1975^{wt}$ tumors expressed high amounts of VEGF (Fig. 2e). Surprisingly, $H1975^{wt}$ tumors exhibited fewer Ki67- and p-Erk-posive cells compared to $H1975^{VEGFR2KD}$ (Fig. 2e), thus confirming that the VEGF:VEGFR2 feed-forward loop primarily controls tumor initiation by stimulating angiogenesis and not proliferation. Binding of tumor cell-derived VEGF to VEGFR2 on tumor cells in $H1975^{wt}$ was confirmed but to a much lesser degree in $H1975^{VEGFR2KD}$ xenograft tumors by staining with

an antibody specifically recognizing human VEGF bound to human VEGFR2[14], confirming the presence of the autocrine stimulation in vivo (Fig. 2e). Furthermore, H1975$^{VEGFR2KD}$ tumors had a markedly reduced uptake of [$^{15}$O]H2O, a measure of tumor blood flow as a functional read-out of angiogenesis[15], when compared to H1975$^{VEGFR2KD}$ tumors (Fig. 2f). As an alternative way to determine whether VEGFR2 inhibition on tumor cells can prevent tumor formation, mice were treated with the VEGFR2 inhibitor ZD6474 simultaneous to tumor inoculation. Consistent with the results obtained with the VEGFR2-knockdown cells, concomitant VEGFR2 inhibition completely abrogated the establishment of tumors in vivo (Fig. 1e), paralleled by a strong reduction in tumor vessel density (Figure 8). In summary, the VEGF: VEGFR2 feed-forward loop is active in vivo, is required for the establishment of fully angiogenic tumors, and its disruption is sufficient to completely prevent tumor formation in vivo.

Next immunhistochemical stainings of VEGF and VEGFR2 in 117 surgically resected primary human lung adenocarcinomas were performed (Fig. 3a) revealing that VEGF expression correlated significantly with expression of VEGFR2 on the same tumor cells (p=2.612x10^-5) as well as with microvessel density (p=2.2x10^-11; Fig. 3b and Figure 9). Thus, activation of the autocrine VEGFR:VEGFR2 signaling loop is a feature of highly angiogenic lung adenocarcinomas. Finally, staining of representative tumors with the antibody specifically recognizing human VEGF when bound to VEGFR2, confirmed that in the tumors with co-expression of VEGF and VEGFR2, VEGF was indeed bound to VEGFR2 on tumor cells. Moreover, adenocarcinomas that exhibited co-expression and autocrine binding of VEGF and VEGFR2, presented a highly angiogenic phenotype (Fig. 3c). These results support a critical role of the VEGF:VEGFR2 feed-forward loop also in primary lung tumors of patients.

In sum, herein a VEGF:VEGFR2 feed-forward loop in tumor cells was identified that leads to a signal amplification and a boost in VEGF secretion, which is required for establishment of fully angiogenic tumors in vivo. This observation supports a model wherein tumor cell-autonomous autocrine VEGF signaling loops are an integral part of the early phase of tumor development promoting both proliferation[6] and nutrient supply through angiogenesis. In line with this notion, it is believed that tumor cells might reduce tumor cell proliferation as long as the nutrient supply remains decreased. This is supported by the finding of Erk inhibition upon VEGF stimulation and by the observation that knockdown of VEGFR2 in tumor cells leads to Erk activation and enhancement of cellular proliferation in vivo. Clinically, the observation that inhibition of VEGFR2 on tumor cells stimulates proliferation suggests that addition of an inhibitor of Erk to therapeutic VEGFR2 inhibition may be synergistic.

Furthermore, inhibition of the VEGF:VEGFR2 autocrine loop on tumor cells was sufficient to abrogate the establishment of tumors in vivo by preventing the outgrowth of blood vessels. Consequently, VEGFR2 inhibition is thought to be effective in preventing relapse induced by residual tumor cells following complete resection of early stage tumors as part of adjuvant therapy. Finally, the fact that dual expression of VEGF and VEGFR2 correlates with microvessel density in primary lung cancer biopsy specimens suggests that this phenotype may serve as a predictive marker for therapeutic efficacy of VEGFR2 inhibition. In summary, a novel autocrine VEGF:VEGFR2 signaling loop is provided that amplifies VEGF secretion of tumor cells and that is required to provide a switch for development of fully angiogenic tumors in vivo.

**Example 2: Cotherapy of the VEGFR-2 inhibitor Vandetanib and MEK inhibitor PD0325901 drastically reduces tumor volume in an animal model of lung cancer.**

**Material and Methods**

All animal procedures were approved by the local animal protection committee and the local authorities. High VEGFR2 expressing tumor cell H1975 and H441 ($5x10^6$ cells), and very low VEGFR2 expressing cells H1650 ($5x10^6$ cells) were injected subcutaneously into male nude mice. Treatment of the tumors was started when tunors had reached a size of about $50mm^3$. Mice were treated daily by oral gavage of ZD6474 (50 mg/kg, solved in vehicle), PD0325901 (12mg/kg, dissolved in propylene glycol:water (1:1)), the combination of ZD6474 (50 mg/kg) and PD0325901 (12mg/kg) or vehicle detergent alone. Tumor size of tumors expressing high levels of VEGFR2 (H441, H1975) and tumor with low expression of VEGFR2 (H1650) were monitored during treatment measuring perpendicular diameters.

**Results**

H1975$^{VEGFR2KD}$ tumors exhibited increased levels of Ki67- and p-Erk-positive cells compared to H1975$^{wt}$ (Fig. 2e). In the same manner VEGF stimulation in vitro reduced pERK signaling that was again increased upon ZD6474 treatment (Fig. 1c). Thus, inhibiting the VEGF:VEGFR2 feed-forward loop results in an activation of the ERK signaling pathway and, thus, seems to induce an ERK-dependant proliferative phenotype. In line with this assumption, combined Vandetanib and PD0325901 treatment of tumors expressing high levels of VEGFR2 (H441, H1975) results in complete tumor shrinkage in vivo (Fig. 10 a (H441), (H1975)). Of note, tumor shrinkage was most strongly observed in tumors with high VEGFR2 expression; in line with our assumption tumor cells with very low VEGFR2 expression did not show tumor shrinkage during combined Vandetanib and PD0325901 treatment in vivo (Fig. 10b (H1650)). In line with these

findings we found that inhibiting the VEGF-VEGFR2-mTor autocrine feed-forward loop in high VEGFR2 expressing tumors enhanced ERK signaling occurs as a result of activation of the insulin growth factor (IGFR) signaling pathway via IRS-1 in vivo as well as in vitro (Fig. 10c, d).

The present invention refers to the following nucleotide and amino acid sequences:

The sequences provided herein are available in the NCBI database and can be retrieved from www.ncbi.nlm.nih.gov/sites/entrez?db=gene; Theses sequences also relate to annotated and modified sequences. The present invention also provides techniques and methods wherein homologous sequences, and variants of the concise sequences provided herein are used. Preferably, such "variants" are genetic variants.

SEQ ID No. 1:

Nucleotide sequence encoding homo sapiens vascular endothelial growth factor receptor-2 (VEGFR2) tyrosine kinase (NCBI accession number ID:3791).

ATGCAGAGCAAGGTGCTGCTGGCCGTCGCCCTGTGGCTCTGCGTGGAGACCCGGG
CCGCCTCTGTGGGTTTGCCTAGTGTTTCTCTTGATCTGCCCAGGCTCAGCATACAA
AAAGACATACTTACAATTAAGGCTAATACAACTCTTCAAATTACTTGCAGGGGAC
AGAGGGACTTGGACTGGCTTTGGCCCAATAATCAGAGTGGCAGTGAGCAAAGGG
TGGAGGTGACTGAGTGCAGCGATGGCCTCTTCTGTAAGACACTCACAATTCCAAA
AGTGATCGGAAATGACACTGGAGCCTACAAGTGCTTCTACCGGGAAACTGACTTG
GCCTCGGTCATTTATGTCTATGTTCAAGATTACAGATCTCCATTTATTGCTTCTGTT
AGTGACCAACATGGAGTCGTGTACATTACTGAGAACAAAAACAAAACTGTGGTG
ATTCCATGTCTCGGGTCCATTTCAAATCTCAACGTGTCACTTTGTGCAAGATACCC
AGAAAAGAGATTTGTTCCTGATGGTAACAGAATTTCCTGGGACAGCAAGAAGGG
CTTTACTATTCCCAGCTACATGATCAGCTATGCTGGCATGGTCTTCTGTGAAGCAA
AAATTAATGATGAAAGTTACCAGTCTATTATGTACATAGTTGTCGTTGTAGGGTA
TAGGATTTATGATGTGGTTCTGAGTCCGTCTCATGGAATTGAACTATCTGTTGGAG
AAAAGCTTGTCTTAAATTGTACAGCAAGAACTGAACTAAATGTGGGGATTGACTT
CAACTGGGAATACCCTTCTTCGAAGCATCAGCATAAGAAACTTGTAAACCGAGAC
CTAAAAACCCAGTCTGGGAGTGAGATGAAGAAATTTTTGAGCACCTTAACTATAG
ATGGTGTAACCCGGAGTGACCAAGGATTGTACACCTGTGCAGCATCCAGTGGGCT
GATGACCAAGAAGAACAGCACATTTGTCAGGGTCCATGAAAAACCTTTTGTTGCT
TTTGGAAGTGGCATGGAATCTCTGGTGGAAGCCACGGTGGGGGAGCGTGTCAGA
ATCCCTGCGAAGTACCTTGGTTACCCACCCCCAGAAATAAAATGGTATAAAAATG
GAATACCCCTTGAGTCCAATCACACAATTAAAGCGGGGCATGTACTGACGATTAT
GGAAGTGAGTGAAAGAGACACAGGAAATTACACTGTCATCCTTACCAATCCCATT
TCAAAGGAGAAGCAGAGCCATGTGGTCTCTCTGGTTGTGTATGTCCCACCCCAGA
TTGGTGAGAAATCTCTAATCTCTCCTGTGGATTCCTACCAGTACGGCACCACTCAA
ACGCTGACATGTACGGTCTATGCCATTCCTCCCCCGCATCACATCCACTGGTATTG
GCAGTTGGAGGAAGAGTGCGCCAACGAGCCCAGCCAAGCTGTCTCAGTGACAAA
CCCATACCCTTGTGAAGAATGGAGAAGTGTGGAGGACTTCCAGGGAGGAAATAA
AATTGAAGTTAATAAAAATCAATTTGCTCTAATTGAAGGAAAAAACAAAACTGTA
AGTACCCTTGTTATCCAAGCGGCAAATGTGTCAGCTTTGTACAAATGTGAAGCGG
TCAACAAAGTCGGGAGAGGAGAGAGGGTGATCTCCTTCCACGTGACCAGGGGTC
CTGAAATTACTTTGCAACCTGACATGCAGCCCACTGAGCAGGAGAGCGTGTCTTT
GTGGTGCACTGCAGACAGATCTACGTTTGAGAACCTCACATGGTACAAGCTTGGC

CCACAGCCTCTGCCAATCCATGTGGGAGAGTTGCCCACACCTGTTTGCAAGAACT
TGGATACTCTTTGGAAATTGAATGCCACCATGTTCTCTAATAGCACAAATGACAT
TTTGATCATGGAGCTTAAGAATGCATCCTTGCAGGACCAAGGAGACTATGTCTGC
CTTGCTCAAGACAGGAAGACCAAGAAAAGACATTGCGTGGTCAGGCAGCTCACA
GTCCTAGAGCGTGTGGCACCCACGATCACAGGAAACCTGGAGAATCAGACGACA
AGTATTGGGGAAAGCATCGAAGTCTCATGCACGGCATCTGGGAATCCCCCTCCAC
AGATCATGTGGTTTAAAGATAATGAGACCCTTGTAGAAGACTCAGGCATTGTATT
GAAGGATGGGAACCGGAACCTCACTATCCGCAGAGTGAGGAAGGAGGACGAAG
GCCTCTACACCTGCCAGGCATGCAGTGTTCTTGGCTGTGCAAAAGTGGAGGCATT
TTTCATAATAGAAGGTGCCCAGGAAAAGACGAACTTGGAAATCATTATTCTAGTA
GGCACGGCGGTGATTGCCATGTTCTTCTGGCTACTTCTTGTCATCATCCTACGGAC
CGTTAAGCGGGCCAATGGAGGGGAACTGAAGACAGGCTACTTGTCCATCGTCAT
GGATCCAGATGAACTCCCATTGGATGAACATTGTGAACGACTGCCTTATGATGCC
AGCAAATGGGAATTCCCCAGAGACCGGCTGAAGCTAGGTAAGCCTCTTGGCCGT
GGTGCCTTTGGCCAAGTGATTGAAGCAGATGCCTTTGGAATTGACAAGACAGCAA
CTTGCAGGACAGTAGCAGTCAAAATGTTGAAAGAAGGAGCAACACACAGTGAGC
ATCGAGCTCTCATGTCTGAACTCAAGATCCTCATTCATATTGGTCACCATCTCAAT
GTGGTCAACCTTCTAGGTGCCTGTACCAAGCCAGGAGGGCCACTCATGGTGATTG
TGGAATTCTGCAAATTTGGAAACCTGTCCACTTACCTGAGGAGCAAGAGAAATGA
ATTTGTCCCCTACAAGACCAAAGGGGCACGATTCCGTCAAGGGAAAGACTACGTT
GGAGCAATCCCTGTGGATCTGAAACGGCGCTTGGACAGCATCACCAGTAGCCAG
AGCTCAGCCAGCTCTGGATTTGTGGAGGAGAAGTCCCTCAGTGATGTAGAAGAA
GAGGAAGCTCCTGAAGATCTGTATAAGGACTTCCTGACCTTGGAGCATCTCATCT
GTTACAGCTTCCAAGTGGCTAAGGGCATGGAGTTCTTGGCATCGCGAAAGTGTAT
CCACAGGGACCTGGCGGCACGAAATATCCTCTTATCGGAGAAGAACGTGGTTAA
AATCTGTGACTTTGGCTTGGCCCGGGATATTTATAAAGATCCAGATTATGTCAGA
AAAGGAGATGCTCGCCTCCCTTTGAAATGGATGGCCCCAGAAACAATTTTTGACA
GAGTGTACACAATCCAGAGTGACGTCTGGTCTTTTGGTGTTTTGCTGTGGGAAAT
ATTTTCCTTAGGTGCTTCTCCATATCCTGGGGTAAAGATTGATGAAGAATTTTGTA
GGCGATTGAAAGAAGGAACTAGAATGAGGGCCCCTGATTATACTACACCAGAAA
TGTACCAGACCATGCTGGACTGCTGGCACGGGGAGCCCAGTCAGAGACCCACGTT
TTCAGAGTTGGTGGAACATTTGGGAAATCTCTTGCAAGCTAATGCTCAGCAGGAT
GGCAAAGACTACATTGTTCTTCCGATATCAGAGACTTTGAGCATGGAAGAGGATT
CTGGACTCTCTCTGCCTACCTCACCTGTTTCCTGTATGGAGGAGGAGGAAGTATGT
GACCCCAAATTCCATTATGACAACACAGCAGGAATCAGTCAGTATCTGCAGAACA
GTAAGCGAAAGAGCCGGCCTGTGAGTGTAAAAACATTTGAAGATATCCCGTTAG
AAGAACCAGAAGTAAAAGTAATCCCAGATGACAACCAGACGGACAGTGGTATGG
TTCTTGCCTCAGAAGAGCTGAAAACTTTGGAAGACAGAACCAAATTATCTCCATC

TTTTGGTGGAATGGTGCCCAGCAAAAGCAGGGAGTCTGTGGCATCTGAAGGCTCA
AACCAGACAAGCGGCTACCAGTCCGGATATCACTCCGATGACACAGACACCACC
GTGTACTCCAGTGAGGAAGCAGAACTTTTAAAGCTGATAGAGATTGGAGTGCAA
ACCGGTAGCACAGCCCAGATTCTCCAGCCTGACTCGGGGACCACACTGAGCTCTC
CTCCTGTTTAA

SEQ ID No. 2:

Amino acid sequence of homo sapiens vascular endothelial growth factor receptor-2 (VEGFR2) tyrsonse kinase (NCBI accession number ID:3791).

MQSKVLLAVALWLCVETRAASVGLPSVSLDLPRLSIQKDILTIKANTTLQITCRGQRD
LDWLWPNNQSGSEQRVEVTECSDGLFCKTLTIPKVIGNDTGAYKCFYRETDLASVIY
VYVQDYRSPFIASVSDQHGVVYITENKNKTVVIPCLGSISNLNVSLCARYPEKRFVPD
GNRISWDSKKGFTIPSYMISYAGMVFCEAKINDESYQSIMYIVVVVGYRIYDVVLSPS
HGIELSVGEKLVLNCTARTELNVGIDFNWEYPSSKHQHKKLVNRDLKTQSGSEMKKF
LSTLTIDGVTRSDQGLYTCAASSGLMTKKNSTFVRVHEKPFVAFGSGMESLVEATVG
ERVRIPAKYLGYPPPEIKWYKNGIPLESNHTIKAGHVLTIMEVSERDTGNYTVILTNPIS
KEKQSHVVSLVVYVPPQIGEKSLISPVDSYQYGTTQTLTCTVYAIPPPHHIHWYWQLE
EECANEPSQAVSVTNPYPCEEWRSVEDFQGGNKIEVNKNQFALIEGKNKTVSTLVIQ
AANVSALYKCEAVNKVGRGERVISFHVTRGPEITLQPDMQPTEQESVSLWCTADRST
FENLTWYKLGPQPLPIHVGELPTPVCKNLDTLWKLNATMFSNSTNDILIMELKNASLQ
DQGDYVCLAQDRKTKKRHCVVRQLTVLERVAPTITGNLENQTTSIGESIEVSCTASG
NPPPQIMWFKDNETLVEDSGIVLKDGNRNLTIRRVRKEDEGLYTCQACSVLGCAKVE
AFFIIEGAQEKTNLEIIILVGTAVIAMFFWLLLVIILRTVKRANGGELKTGYLSIVMDPD
ELPLDEHCERLPYDASKWEFPRDRLKLGKPLGRGAFGQVIEADAFGIDKTATCRTVA
VKMLKEGATHSEHRALMSELKILIHIGHHLNVVNLLGACTKPGGPLMVIVEFCKFGN
LSTYLRSKRNEFVPYKTKGARFRQGKDYVGAIPVDLKRRLDSITSSQSSASSGFVEEK
SLSDVEEEEAPEDLYKDFLTLEHLICYSFQVAKGMEFLASRKCIHRDLAARNILLSEK
NVVKICDFGLARDIYKDPDYVRKGDARLPLKWMAPETIFDRVYTIQSDVWSFGVLL
WEIFSLGASPYPGVKIDEEFCRRLKEGTRMRAPDYTTPEMYQTMLDCWHGEPSQRPT
FSELVEHLGNLLQANAQQDGKDYIVLPISETLSMEEDSGLSLPTSPVSCMEEEEVCDP
KFHYDNTAGISQYLQNSKRKSRPVSVKTFEDIPLEEPEVKVIPDDNQTDSGMVLASEE
LKTLEDRTKLSPSFGGMVPSKSRESVASEGSNQTSGYQSGYHSDDTDTTVYSSEEAEL
LKLIEIGVQTGSTAQILQPDSGTTLSSPPV

SEQ ID No. 3:

Nucleotide sequence encoding homo sapiens MAP kinase kinase 1 (MEK1) (NCBI accession number ID: 5594).

ATGGCGGCGGCGGCGGCGGCGGGCGCGGGCCCGGAGATGGTCCGCGGGCAGGTG
TTCGACGTGGGGCCGCGCTACACCAACCTCTCGTACATCGGCGAGGGCGCCTACG
GCATGGTGTGCTCTGCTTATGATAATGTCAACAAAGTTCGAGTAGCTATCAAGAA
AATCAGCCCCTTTGAGCACCAGACCTACTGCCAGAGAACCCTGAGGGAGATAAA
AATCTTACTGCGCTTCAGACATGAGAACATCATTGGAATCAATGACATTATTCGA
GCACCAACCATCGAGCAAATGAAAGATGTATATATAGTACAGGACCTCATGGAA
ACAGATCTTTACAAGCTCTTGAAGACACAACACCTCAGCAATGACCATATCTGCT
ATTTTCTCTACCAGATCCTCAGAGGGTTAAAATATATCCATTCAGCTAACGTTCTG
CACCGTGACCTCAAGCCTTCCAACCTGCTGCTCAACACCACCTGTGATCTCAAGA
TCTGTGACTTTGGCCTGGCCCGTGTTGCAGATCCAGACCATGATCACACAGGGTT
CCTGACAGAATATGTGGCCACACGTTGGTACAGGGCTCCAGAAATTATGTTGAAT
TCCAAGGGCTACACCAAGTCCATTGATATTTGGTCTGTAGGCTGCATTCTGGCAG
AAATGCTTTCTAACAGGCCCATCTTTCCAGGGAAGCATTATCTTGACCAGCTGAA
CCACATTTTGGGTATTCTTGGATCCCCATCACAAGAAGACCTGAATTGTATAATA
AATTTAAAAGCTAGGAACTATTTGCTTTCTCTTCCACACAAAAATAAGGTGCCAT
GGAACAGGCTGTTCCCAAATGCTGACTCCAAAGCTCTGGACTTATTGGACAAAAT
GTTGACATTCAACCCACACAAGAGGATTGAAGTAGAACAGGCTCTGGCCCACCC
ATATCTGGAGCAGTATTACGACCCGAGTGACGAGCCCATCGCCGAAGCACCATTC
AAGTTCGACATGGAATTGGATGACTTGCCTAAGGAAAAGCTCAAAGAACTAATTT
TTGAAGAGACTGCTAGATTCCAGCCAGGATACAGATCTTAA

SEQ ID No. 4:

Amino acid sequence of homo sapiens MAP kinase kinase 1 (MEK1) (NCBI accession number ID: 5594).

MAAAAAAGAGPEMVRGQVFDVGPRYTNLSYIGEGAYGMVCSAYDNVNKVRVAIK
KISPFEHQTYCQRTLREIKILLRFRHENIIGINDIIRAPTIEQMKDVYIVQDLMETDLYKL
LKTQHLSNDHICYFLYQILRGLKYIHSANVLHRDLKPSNLLLNTTCDLKICDFGLARV
ADPDHDHTGFLTEYVATRWYRAPEIMLNSKGYTKSIDIWSVGCILAEMLSNRPIFPGK
HYLDQLNHILGILGSPSQEDLNCIINLKARNYLLSLPHKNKVPWNRLFPNADSKALDL
LDKMLTFNPHKRIEVEQALAHPYLEQYYDPSDEPIAEAPFKFDMELDDLPKEKLKELI
FEETARFQPGYRS

SEQ ID No. 5:

Nucleotide sequence encoding homo sapiens MAP kinase kinase 2 (MEK 2) (NCBI accession number ID: 5605).

ATGCTGGCCCGGAGGAAGCCGGTGCTGCCGGCGCTCACCATCAACCCTACCATCG
CCGAGGGCCCATCCCCTACCAGCGAGGGCGCCTCCGAGGCAAACCTGGTGGACC
TGCAGAAGAAGCTGGAGGAGCTGGAACTTGACGAGCAGCAGAAGAAGCGGCTG
GAAGCCTTTCTCACCCAGAAAGCCAAGGTCGGCGAACTCAAAGACGATGACTTC
GAAAGGATCTCAGAGCTGGGCGCGGGCAACGGCGGGGTGGTCACCAAAGTCCAG
CACAGACCCTCGGGCCTCATCATGGCCAGGAAGCTGATCCACCTTGAGATCAAGC
CGGCCATCCGGAACCAGATCATCCGCGAGCTGCAGGTCCTGCACGAATGCAACTC
GCCGTACATCGTGGGCTTCTACGGGGCCTTCTACAGTGACGGGGAGATCAGCATT
TGCATGGAACACATGGACGGCGGCTCCCTGGACCAGGTGCTGAAAGAGGCCAAG
AGGATTCCCGAGGAGATCCTGGGGAAAGTCAGCATCGCGGTTCTCCGGGGCTTGG
CGTACCTCCGAGAGAAGCACCAGATCATGCACCGAGATGTGAAGCCCTCCAACA
TCCTCGTGAACTCTAGAGGGGAGATCAAGCTGTGTGACTTCGGGGTGAGCGGCCA
GCTCATCGACTCCATGGCCAACTCCTTCGTGGGCACGCGCTCCTACATGGCTCCG
GAGCGGTTGCAGGGCACACATTACTCGGTGCAGTCGGACATCTGGAGCATGGGC
CTGTCCCTGGTGGAGCTGGCCGTCGGAAGGTACCCCATCCCCCCGCCCGACGCCA
AAGAGCTGGAGGCCATCTTTGGCCGGCCCGTGGTCGACGGGGAAGAAGGAGAGC
CTCACAGCATCTCGCCTCGGCCGAGGCCCCCCGGGCGCCCCGTCAGCGGTCACGG
GATGGATAGCCGGCCTGCCATGGCCATCTTTGAACTCCTGGACTATATTGTGAAC
GAGCCACCTCCTAAGCTGCCCAACGGTGTGTTCACCCCCGACTTCCAGGAGTTTG
TCAATAAATGCCTCATCAAGAACCCAGCGGAGCGGGCGGACCTGAAGATGCTCA
CAAACCACACCTTCATCAAGCGGTCCGAGGTGGAAGAAGTGGATTTTGCCGGCTG
GTTGTGTAAAACCCTGCGGCTGAACCAGCCCGGCACACCCACGCGCACCGCCGTG
TGA

SEQ ID No. 6:

Amino acid sequence of homo sapiens MAP kinase kinase 2 (MEK2) (NCBI accession number ID: 5605).

MLARRKPVLPALTINPTIAEGPSPTSEGASEANLVDLQKKLEELELDEQQKKRLEAFL
TQKAKVGELKDDDFERISELGAGNGGVVTKVQHRPSGLIMARKLIHLEIKPAIRNQIIR
ELQVLHECNSPYIVGFYGAFYSDGEISICMEHMDGGSLDQVLKEAKRIPEEILGKVSIA
VLRGLAYLREKHQIMHRDVKPSNILVNSRGEIKLCDFGVSGQLIDSMANSFVGTRSY
MAPERLQGTHYSVQSDIWSMGLSLVELAVGRYPIPPPDAKELEAIFGRPVVDGEEGEP
HSISPRPRPPGRPVSGHGMDSRPAMAIFELLDYIVNEPPPKLPNGVFTPDFQEFVNKCLI
KNPAERADLKMLTNHTFIKRSEVEEVDFAGWLCKTLRLNQPGTPTRTAV

SEQ ID No. 7:

Nucleotide sequence encoding homo sapiens vascular endothelial growth factor (VEGF-A) (NCBI accession number ID:7422).

CTGACGGACAGACAGACAGACACCGCCCCCAGCCCCAGCTACCACCTCCTCCCCG
GCCGGCGGCGGACAGTGGACGCGGCGGCGAGCCGCGGGCAGGGGCCGGAGCCC
GCGCCCGGAGGCGGGGTGGAGGGGGTCGGGGCTCGCGGCGTCGCACTGAAACTT
TTCGTCCAACTTCTGGGCTGTTCTCGCTTCGGAGGAGCCGTGGTCCGCGCGGGGG
AAGCCGAGCCGAGCGGAGCCGCGAGAAGTGCTAGCTCGGGCCGGGAGGAGCCGC
AGCCGGAGGAGGGGGAGGAGGAAGAAGAGAAGGAAGAGGAGAGGGGGCCGCA
GTGGCGACTCGGCGCTCGGAAGCCGGGCTCATGGACGGGTGAGGCGGCGGTGTG
CGCAGACAGTGCTCCAGCCGCGCGCGCTCCCCAGGCCCTGGCCCGGGCCTCGGGC
CGGGGAGGAAGAGTAGCTCGCCGAGGCGCCGAGGAGAGCGGGCCGCCCCACAG
CCCGAGCCGGAGAGGGAGCGCGAGCCGCGCCGGCCCCGGTCGGGCCTCCGAAAC
CATGAACTTTCTGCTGTCTTGGGTGCATTGGAGCCTTGCCTTGCTGCTCTACCTCC
ACCATGCCAAGTGGTCCCAGGCTGCACCCATGGCAGAAGGAGGAGGGCAGAATC
ATCACGAAGTGGTGAAGTTCATGGATGTCTATCAGCGCAGCTACTGCCATCCAAT
CGAGACCCTGGTGGACATCTTCCAGGAGTACCCTGATGAGATCGAGTACATCTTC
AAGCCATCCTGTGTGCCCCTGATGCGATGCGGGGGCTGCTGCAATGACGAGGGCC
TGGAGTGTGTGCCCACTGAGGAGTCCAACATCACCATGCAGATTATGCGGATCAA
ACCTCACCAAGGCCAGCACATAGGAGAGATGAGCTTCCTACAGCACAACAAATG
TGAATGCAGACCAAAGAAAGATAGAGCAAGACAAGAAAAAAAATCAGTTCGAG
GAAAGGGAAAGGGGCAAAAACGAAAGCGCAAGAAATCCCGGTATAAGTCCTGG
AGCGTTCCCTGTGGGCCTTGCTCAGAGCGGAGAAAGCATTTGTTTGTACAAGATC
CGCAGACGTGTAAATGTTCCTGCAAAAACACAGACTCGCGTTGCAAGGCGAGGC
AGCTTGAGTTAAACGAACGTACTTGCAGATGTGACAAGCCGAGGCGGTGA

SEQ ID No. 8:

Amino acid sequence of homo sapiens vascular endothelial growth factor (VEGF-A) (NCBI accession number ID:7422).

MTDRQTDTAPSPSYHLLPGRRRTVDAAASRGQGPEPAPGGGVEGVGARGVALKLFV
QLLGCSRFGGAVVRAGEAEPSGAARSASSGREEPQPEEGEEEEEKEEERGPQWRLGA
RKPGSWTGEAAVCADSAPAARAPQALARASGRGGRVARRGAEESGPPHSPSRRGSA
SRAGPGRASETMNFLLSWVHWSLALLLYLHHAKWSQAAPMAEGGGQNHHEVVKF
MDVYQRSYCHPIETLVDIFQEYPDEIEYIFKPSCVPLMRCGGCCNDEGLECVPTEESNI
TMQIMRIKPHQGQHIGEMSFLQHNKCECRPKKDRARQEKKSVRGKGKGQKRKRKKS
RYKSWSVPCGPCSERRKHLFVQDPQTCKCSCKNTDSRCKARQLELNERTCRCDKPRR

SEQ ID No. 9:

Nucleotide sequence encoding homo sapiens epidermal growth factor receptor (EGFR) tyrosine kinase (EGFR tyrosine kinase) (NCBI accession number ID:1956).

ATGCGACCCTCCGGGACGGCCGGGGCAGCGCTCCTGGCGCTGCTGGCTGCGCTCT GCCCGGCGAGTCGGGCTCTGGAGGAAAAGAAAGTTTGCCAAGGCACGAGTAACA AGCTCACGCAGTTGGGCACTTTTGAAGATCATTTTCTCAGCCTCCAGAGGATGTTC AATAACTGTGAGGTGGTCCTTGGGAATTTGGAAATTACCTATGTGCAGAGGAATT ATGATCTTTCCTTCTTAAAGACCATCCAGGAGGTGGCTGGTTATGTCCTCATTGCC CTCAACACAGTGGAGCGAATTCCTTTGGAAAACCTGCAGATCATCAGAGGAAAT ATGTACTACGAAAATTCCTATGCCTTAGCAGTCTTATCTAACTATGATGCAAATA AAACCGGACTGAAGGAGCTGCCCATGAGAAATTTACAGGAAATCCTGCATGGCG CCGTGCGGTTCAGCAACAACCCTGCCCTGTGCAACGTGGAGAGCATCCAGTGGCG GGACATAGTCAGCAGTGACTTTCTCAGCAACATGTCGATGGACTTCCAGAACCAC CTGGGCAGCTGCCAAAAGTGTGATCCAAGCTGTCCCAATGGGAGCTGCTGGGGTG CAGGAGAGGAGAACTGCCAGAAACTGACCAAAATCATCTGTGCCCAGCAGTGCT CCGGGCGCTGCCGTGGCAAGTCCCCCAGTGACTGCTGCCACAACCAGTGTGCTGC AGGCTGCACAGGCCCCCGGGAGAGCGACTGCCTGGTCTGCCGCAAATTCCGAGA CGAAGCCACGTGCAAGGACACCTGCCCCCCACTCATGCTCTACAACCCCACCACG TACCAGATGGATGTGAACCCCGAGGGCAAATACAGCTTTGGTGCCACCTGCGTGA AGAAGTGTCCCCGTAATTATGTGGTGACAGATCACGGCTCGTGCGTCCGAGCCTG TGGGGCCGACAGCTATGAGATGGAGGAAGACGGCGTCCGCAAGTGTAAGAAGTG CGAAGGGCCTTGCCGCAAAGTGTGTAACGGAATAGGTATTGGTGAATTTAAAGA CTCACTCTCCATAAATGCTACGAATATTAAACACTTCAAAAACTGCACCTCCATC AGTGGCGATCTCCACATCCTGCCGGTGGCATTTAGGGGTGACTCCTTCACACATA CTCCTCCTCTGGATCCACAGGAACTGGATATTCTGAAAACCGTAAAGGAAATCAC AGGGTTTTTGCTGATTCAGGCTTGGCCTGAAAACAGGACGGACCTCCATGCCTTT GAGAACCTAGAAATCATACGCGGCAGGACCAAGCAACATGGTCAGTTTTCTCTTG CAGTCGTCAGCCTGAACATAACATCCTTGGGATTACGCTCCCTCAAGGAGATAAG TGATGGAGATGTGATAATTTCAGGAAACAAAAATTTGTGCTATGCAAATACAATA AACTGGAAAAAACTGTTTGGGACCTCCGGTCAGAAAACCAAAATTATAAGCAAC AGAGGTGAAAACAGCTGCAAGGCCACAGGCCAGGTCTGCCATGCCTTGTGCTCCC CCGAGGGCTGCTGGGGCCCGGAGCCCAGGGACTGCGTCTCTTGCCGGAATGTCAG CCGAGGCAGGGAATGCGTGGACAAGTGCAACCTTCTGGAGGGTGAGCCAAGGGA GTTTGTGGAGAACTCTGAGTGCATACAGTGCCACCCAGAGTGCCTGCCTCAGGCC ATGAACATCACCTGCACAGGACGGGGACCAGACAACTGTATCCAGTGTGCCCACT ACATTGACGGCCCCCACTGCGTCAAGACCTGCCCGGCAGGAGTCATGGGAGAAA ACAACACCCTGGTCTGGAAGTACGCAGACGCCGGCCATGTGTGCCACCTGTGCCA

TCCAAACTGCACCTACGGATGCACTGGGCCAGGTCTTGAAGGCTGTCCAACGAAT
GGGCCTAAGATCCCGTCCATCGCCACTGGGATGGTGGGGGCCCTCCTCTTGCTGC
TGGTGGTGGCCCTGGGGATCGGCCTCTTCATGCGAAGGCGCCACATCGTTCGGAA
GCGCACGCTGCGGAGGCTGCTGCAGGAGAGGGAGCTTGTGGAGCCTCTTACACC
CAGTGGAGAAGCTCCCAACCAAGCTCTCTTGAGGATCTTGAAGGAAACTGAATTC
AAAAAGATCAAAGTGCTGGGCTCCGGTGCGTTCGGCACGGTGTATAAGGGACTCT
GGATCCCAGAAGGTGAGAAAGTTAAAATTCCCGTCGCTATCAAGGAATTAAGAG
AAGCAACATCTCCGAAAGCCAACAAGGAAATCCTCGATGAAGCCTACGTGATGG
CCAGCGTGGACAACCCCCACGTGTGCCGCCTGCTGGGCATCTGCCTCACCTCCAC
CGTGCAGCTCATCACGCAGCTCATGCCCTTCGGCTGCCTCCTGGACTATGTCCGG
GAACACAAAGACAATATTGGCTCCCAGTACCTGCTCAACTGGTGTGTGCAGATCG
CAAAGGGCATGAACTACTTGGAGGACCGTCGCTTGGTGCACCGCGACCTGGCAG
CCAGGAACGTACTGGTGAAAACACCGCAGCATGTCAAGATCACAGATTTTGGGCT
GGCCAAACTGCTGGGTGCGGAAGAGAAAGAATACCATGCAGAAGGAGGCAAAG
TGCCTATCAAGTGGATGGCATTGGAATCAATTTTACACAGAATCTATACCCACCA
GAGTGATGTCTGGAGCTACGGGGTGACTGTTTGGGAGTTGATGACCTTTGGATCC
AAGCCATATGACGGAATCCCTGCCAGCGAGATCTCCTCCATCCTGGAGAAAGGA
GAACGCCTCCCTCAGCCACCCATATGTACCATCGATGTCTACATGATCATGGTCA
AGTGCTGGATGATAGACGCAGATAGTCGCCCAAAGTTCCGTGAGTTGATCATCGA
ATTCTCCAAAATGGCCCGAGACCCCCAGCGCTACCTTGTCATTCAGGGGGATGAA
AGAATGCATTTGCCAAGTCCTACAGACTCCAACTTCTACCGTGCCCTGATGGATG
AAGAAGACATGGACGACGTGGTGGATGCCGACGAGTACCTCATCCCACAGCAGG
GCTTCTTCAGCAGCCCCTCCACGTCACGGACTCCCCTCCTGAGCTCTCTGAGTGCA
ACCAGCAACAATTCCACCGTGGCTTGCATTGATAGAAATGGGCTGCAAAGCTGTC
CCATCAAGGAAGACAGCTTCTTGCAGCGATACAGCTCAGACCCCACAGGCGCCTT
GACTGAGGACAGCATAGACGACACCTTCCTCCCAGTGCCTGAATACATAAACCAG
TCCGTTCCCAAAAGGCCCGCTGGCTCTGTGCAGAATCCTGTCTATCACAATCAGC
CTCTGAACCCCGCGCCCAGCAGAGACCCACACTACCAGGACCCCCACAGCACTGC
AGTGGGCAACCCCGAGTATCTCAACACTGTCCAGCCCACCTGTGTCAACAGCACA
TTCGACAGCCCTGCCCACTGGGCCCAGAAAGGCAGCCACCAAATTAGCCTGGAC
AACCCTGACTACCAGCAGGACTTCTTTCCCAAGGAAGCCAAGCCAAATGGCATCT
TTAAGGGCTCCACAGCTGAAAATGCAGAATACCTAAGGGTCGCGCCACAAAGCA
GTGAATTTATTGGAGCATGA

SEQ ID No. 10:

Amino acid sequence of homo sapiens epidermal growth factor receptor (EGFR) tyrosine kinase (EGFR tyrosine kinase) (NCBI accession number ID:1956).

MRPSGTAGAALLALLAALCPASRALEEKKVCQGTSNKLTQLGTFEDHFLSLQRMFN
NCEVVLGNLEITYVQRNYDLSFLKTIQEVAGYVLIALNTVERIPLENLQIIRGNMYYE
NSYALAVLSNYDANKTGLKELPMRNLQEILHGAVRFSNNPALCNVESIQWRDIVSSD
FLSNMSMDFQNHLGSCQKCDPSCPNGSCWGAGEENCQKLTKIICAQQCSGRCRGKSP
SDCCHNQCAAGCTGPRESDCLVCRKFRDEATCKDTCPPLMLYNPTTYQMDVNPEGK
YSFGATCVKKCPRNYVVTDHGSCVRACGADSYEMEEDGVRKCKKCEGPCRKVCNG
IGIGEFKDSLSINATNIKHFKNCTSISGDLHILPVAFRGDSFTHTPPLDPQELDILKTVKE
ITGFLLIQAWPENRTDLHAFENLEIIRGRTKQHGQFSLAVVSLNITSLGLRSLKEISDGD
VIISGNKNLCYANTINWKKLFGTSGQKTKIISNRGENSCKATGQVCHALCSPEGCWGP
EPRDCVSCRNVSRGRECVDKCNLLEGEPREFVENSECIQCHPECLPQAMNITCTGRGP
DNCIQCAHYIDGPHCVKTCPAGVMGENNTLVWKYADAGHVCHLCHPNCTYGCTG
PGLEGCPTNGPKIPSIATGMVGALLLLLVVALGIGLFMRRRHIVRKRTLRRLLQEREL
VEPLTPSGEAPNQALLRILKETEFKKIKVLGSGAFGTVYKGLWIPEGEKVKIPVAIKEL
REATSPKANKEILDEAYVMASVDNPHVCRLLGICLTSTVQLITQLMPFGCLLDYVREH
KDNIGSQYLLNWCVQIAKGMNYLEDRRLVHRDLAARNVLVKTPQHVKITDFGLAKL
LGAEEKEYHAEGGKVPIKWMALESILHRIYTHQSDVWSYGVTVWELMTFGSKPYDG
IPASEISSILEKGERLPQPPICTIDVYMIMVKCWMIDADSRPKFRELIIEFSKMARDPQR
YLVIQGDERMHLPSPTDSNFYRALMDEEDMDDVVDADEYLIPQQGFFSSPSTSRTPLL
SSLSATSNNSTVACIDRNGLQSCPIKEDSFLQRYSSDPTGALTEDSIDDTFLPVPEYINQ
SVPKRPAGSVQNPVYHNQPLNPAPSRDPHYQDPHSTAVGNPEYLNTVQPTCVNSTFD
SPAHWAQKGSHQISLDNPDYQQDFFPKEAKPNGIFKGSTAENAEYLRVAPQSSEFIGA

SEQ ID No. 11:

Nucleotide sequence encoding homo sapiens MAP kinase kinase 5 (MEK 5) (NCBI accession number ID: 5607).

ATGATGGAGGGTCACTTTCCCCAGAGCGATGTGATAGGCCAGGTTCTGCCTGAAG
CAACAACTACAGCATTTGAATATGAAGATGAAGATGGTGATCGAATTACAGTGA
GAAGTGATGAGGAAATGAAGGCAATGCTGTCATATTATTATTCCACAGTAATGGA
ACAGCAAGTAAATGGACAGTTAATAGAGCCTCTGCAGATATTTCCAAGAGCCTGC
AAGCCTCCTGGGGAACGGAACATACATGGCCTGAAGGTGAATACTCGGGCCGGA
CCCTCTCAACACAGCAGCCCAGCAGTCTCAGATTCACTTCCAAGCAATAGCTTAA
AGAAGTCTTCTGCTGAACTGAAAAAAATACTAGCCAATGGCCAGATGAATGAAC
AAGACATACGATATCGGGACACTCTTGGTCATGGCAACGGAGGCACAGTCTACA
AAGCATATCATGTCCCGAGTGGGAAAATATTAGCTGTAAAGGTCATACTACTAGA

TATTACACTGGAACTTCAGAAGCAAATTATGTCTGAATTGGAAATTCTTTATAAG
TGCGATTCATCATATATCATTGGATTTTATGGAGCATTTTTGTAGAAAACAGGAT
TTCAATATGTACAGAATTCATGGATGGGGGATCTTTGGATGTATATAGGAAAATG
CCAGAACATGTCCTTGGAAGAATTGCAGTAGCAGTTGTTAAAGGCCTTACTTATT
TGTGGAGTTTAAAGATTTTACATAGAGACGTGAAGCCCTCCAATATGCTAGTAAA
CACAAGAGGACAGGTTAAGCTGTGTGATTTTGGAGTTAGCACTCAGCTGGTGAAT
TCTATAGCCAAGACGTATGTTGGAACAAATGCTTATATGGCGCCTGAAAGGATTT
CAGGGGAGCAGTATGGAATTCATTCTGATGTCTGGAGCTTAGGAATCTCTTTTAT
GGAGCTTGCTCTTGGGAGGTTTCCATATCCTCAGATTCAGAAAAACCAGGGATCT
TTAATGCCTCTCCAGCTTCTGCAGTGCATTGTTGATGAGGATTCGCCCGTCCTTCC
AGTTGGAGAGTTCTCGGAGCCATTTGTACATTTCATCACTCAGTGTATGCGAAAA
CAGCCAAAAGAAAGGCCAGCACCTGAAGAATTGATGGGCCACCCGTTCATCGTG
CAGTTCAATGATGGAAATGCCGCCGTGGTGTCCATGTGGGTGTGCCGGGCGCTGG
AGGAGAGGCGGAGCCAGCAGGGGCCCCCGTGA

SEQ ID No. 12:

Amino acid sequence of homo sapiens MAP kinase kinase 2 (MEK2) (NCBI accession number ID: 5607).

MMEGHFPQSDVIGQVLPEATTTAFEYEDEDGDRITVRSDEEMKAMLSYYYSTVMEQ
QVNGQLIEPLQIFPRACKPPGERNIHGLKVNTRAGPSQHSSPAVSDSLPSNSLKKSSAE
LKKILANGQMNEQDIRYRDTLGHGNGGTVYKAYHVPSGKILAVKVILLDITLELQKQ
IMSELEILYKCDSSYIIGFYGAFFVENRISICTEFMDGGSLDVYRKMPEHVLGRIAVAV
VKGLTYLWSLKILHRDVKPSNMLVNTRGQVKLCDFGVSTQLVNSIAKTYVGTNAYM
APERISGEQYGIHSDVWSLGISFMELALGRFPYPQIQKNQGSLMPLQLLQCIVDEDSPV
LPVGEFSEPFVHFITQCMRKQPKERPAPEELMGHPFIVQFNDGNAAVVSMWVCRALE
ERRSQQGPP

**List of References**

1. Baeriswyl, V. & Christofori, G. The angiogenic switch in carcinogenesis. Semin Cancer Biol 19, 329-337 (2009).
2. Carmeliet, P. Mechanisms of angiogenesis and arteriogenesis. Nature medicine 6, 389-395 (2000).
3. Lee, T.H., et al. Vascular endothelial growth factor mediates intracrine survival in human breast carcinoma cells through internally expressed VEGFR1/FLT1. PLoS medicine 4, e186 (2007).
4. Chung, G.G., et al. Vascular endothelial growth factor, FLT-1, and FLK-1 analysis in a pancreatic cancer tissue microarray. Cancer 106, 1677-1684 (2006).
5. Silva, S.R., et al. VEGFR-2 expression in carcinoid cancer cells and its role in tumor growth and metastasis. Int J Cancer (2010).
6. Lichtenberger, B.M., et al. Autocrine VEGF signaling synergizes with EGFR in tumor cells to promote epithelial cancer development. Cell 140, 268-279 (2010).

7. Pao, W., et al. Acquired resistance of lung adenocarcinomas to gefitinib or erlotinib is associated with a second mutation in the EGFR kinase domain. PLoS Med 2, e73 (2005).
8. Wedge, S.R., et al. ZD6474 inhibits vascular endothelial growth factor signaling, angiogenesis, and tumor growth following oral administration. Cancer Res 62, 4645-4655 (2002).
9. Ullrich, R.T., et al. Methyl-L-11C-methionine PET as a diagnostic marker for malignant progression in patients with glioma. J Nucl Med 50, 1962-1968 (2009).
10. Shields, A.F., et al. Imaging proliferation in vivo with [F-18]FLT and positron emission tomography. Nature medicine 4, 1334-1336 (1998).
11. Kanai, Y., et al. Expression cloning and characterization of a transporter for large neutral amino acids activated by the heavy chain of 4F2 antigen (CD98). The Journal of biological chemistry 273, 23629-23632 (1998).
12. Vasudevan, K.M., et al. AKT-independent signaling downstream of oncogenic PIK3CA mutations in human cancer. Cancer Cell 16, 21-32 (2009).
13. Blencke, S., et al. Characterization of a conserved structural determinant controlling protein kinase sensitivity to selective inhibitors. Chem Biol 11, 691-701 (2004).
14. Brekken, R.A., Huang, X., King, S.W. & Thorpe, P.E. Vascular endothelial growth factor as a marker of tumor endothelium. Cancer Res 58, 1952-1959 (1998).
15. Laking, G.R. & Price, P.M. Positron emission tomographic imaging of angiogenesis and vascular function. Br J Radiol 76 Spec No 1, S50-59 (2003).
16. Roberts, D.M., et al. The vascular endothelial growth factor (VEGF) receptor Flt-1 (VEGFR-1) modulates Flk-1 (VEGFR-2) signaling during blood vessel formation. Am J Pathol 164, 1531-1535 (2004).
17. Hamacher, K., Coenen, H.H. & Stocklin, G. Efficient stereospecific synthesis of no-carrier-added 2-[18F]-fluoro-2-deoxy-D-glucose using aminopolyether supported nucleophilic substitution. J Nucl Med 27, 235-238 (1986).
18. Machulla, H., et al. Simplified labeling approach for synthesizing 3-deoxy-3-[F-18]fluorothymidine ([F-18]FLT). J Radiochem Nucl Chem 243, 843- 846 (2000).

All references cited herein are fully incorporated by reference. Having now fully described the invention, it will be understood by a person skilled in the art that the invention may be practiced within a wide and equivalent range of conditions, parameters and the like, without affecting the spirit or scope of the invention or any embodiment thereof.

The present invention relates to the following items:

1. A pharmaceutical composition comprising a selective inhibitor of vascular endothelial growth factor receptor-2 (VEGFR-2) tyrosine kinase or a pharmaceutically acceptable salt, solvate and/or hydrate thereof, and an inhibitor of MAP kinase kinase (MEK) or a pharmaceutically acceptable salt, solvate and/or hydrate thereof, for use in the treatment of cancer.

2. A method for treating cancer comprising administering an effective amount of a selective inhibitor of vascular endothelial growth factor receptor-2 (VEGFR-2) tyrosine kinase or a pharmaceutically acceptable salt, solvate and/or hydrate thereof, and an inhibitor of MAP kinase kinase (MEK) or a pharmaceutically acceptable salt, solvate and/or hydrate thereof to a subject in need thereof.

3. The pharmaceutical composition according to item 1, or the method of item 2, wherein said selective inhibitor of VEGFR-2 tyrosine kinase inhibits in addition epidermal growth factor receptor (EGFR) tyrosine kinase.

4. The pharmaceutical composition according to item 1 or 3, or the method of item 2 or 3, wherein said MEK inhibitor inhibits MEK1 and/or MEK2; and/or MEK1 and MEK2 and, in addition or in the alternative MEK5.

5. The pharmaceutical composition according to any one of items 1, 3 and 4, or the method of any one of items 2 to 4, wherein said selective inhibitor of VEGFR-2 tyrosine kinase is selected from the group consisting of N-(4-bromo-2-fluorophenyl)-6-methoxy-7-[(1-methylpiperidin-4-yl)methoxy]quinazolin-4-amine (Vandetanib/Zactima/ZD6474) as shown in formula (I) below or a pharmaceutically acceptable salt, solvate or hydrate thereof:

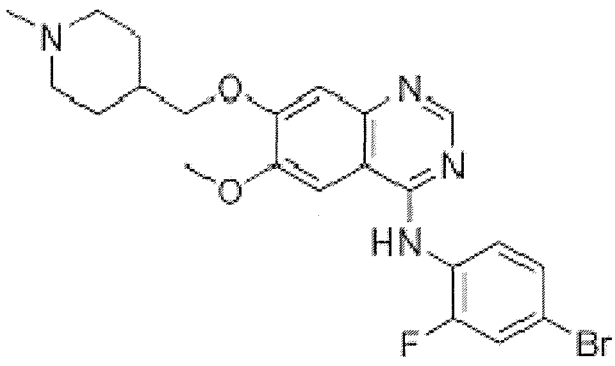

(I);

Cabozantinib (BMS-907351) as shown in formula (II) below or a pharmaceutically acceptable salt, solvate or hydrate thereof:

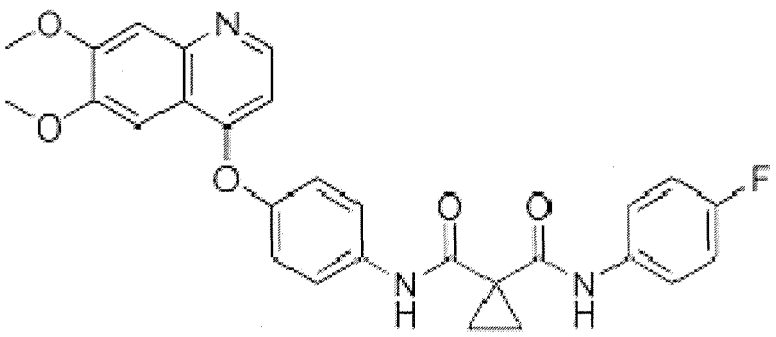

(II);

Apatinib (YN968D1) as shown in formula (III) below or a pharmaceutically acceptable salt, solvate or hydrate thereof:

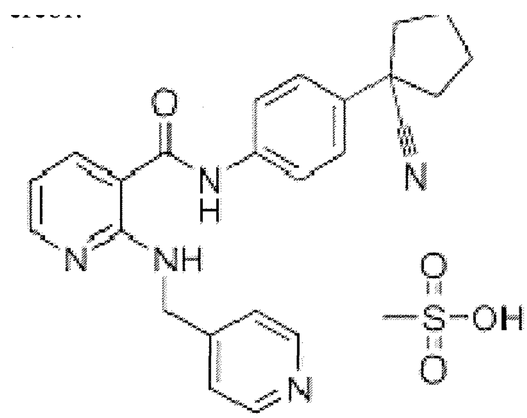

(III);

BMS 794833 as shown in formula (IV) below or a pharmaceutically acceptable salt, solvate or hydrate thereof:

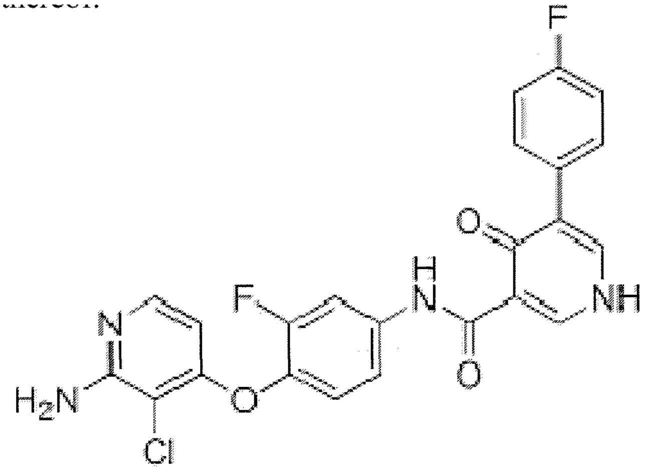

(IV);

Ki8751 as shown in formula (V) below or a pharmaceutically acceptable salt, solvate or hydrate thereof:

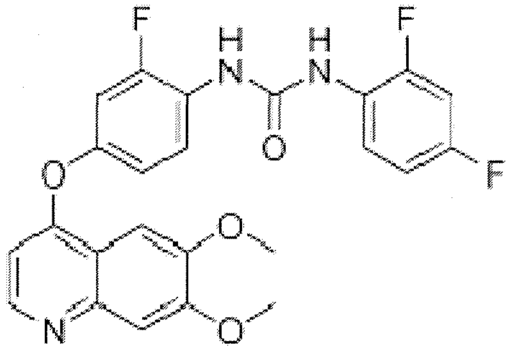

(V); and

OSI-930 as shown in formula (VI) below or a pharmaceutically acceptable salt, solvate or hydrate thereof:

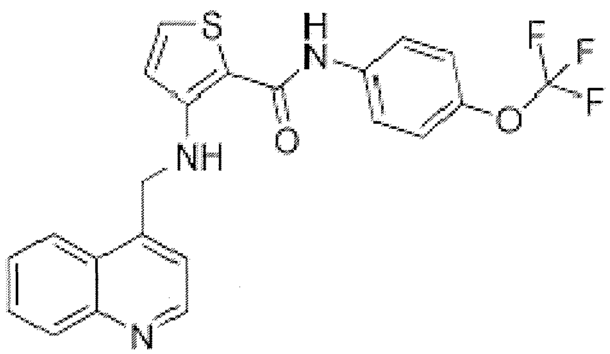

(VI).

6. The pharmaceutical composition according to any one of items 1, 3 and 5, or the method of any one of items 2 to 5, wherein said MEK inhibitor is selected from the group consisting of N-[(2R)-2,3-dihydroxypropoxy]-3,4-difluoro-2-[(2-fluoro-4-iodophenyl)amino]-benzamide (PD0325901) as shown in

formula (VII) below or a pharmaceutically acceptable salt, solvate or hydrate thereof:

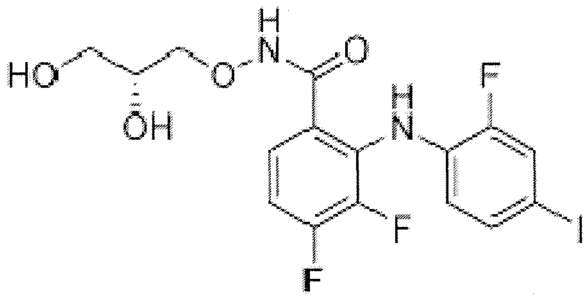

(VII);

AS703026 as shown in formula (VIII) below or a pharmaceutically acceptable salt, solvate or hydrate thereof:

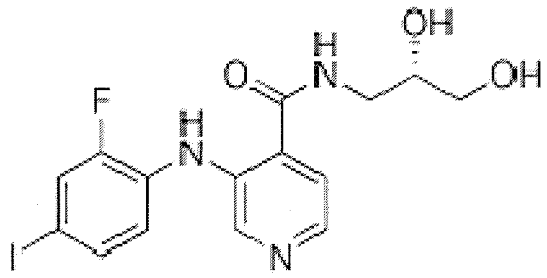

(VIII);

AZD6244 (Selumetinib) as shown in formula (IX) below or a pharmaceutically acceptable salt, solvate or hydrate thereof:

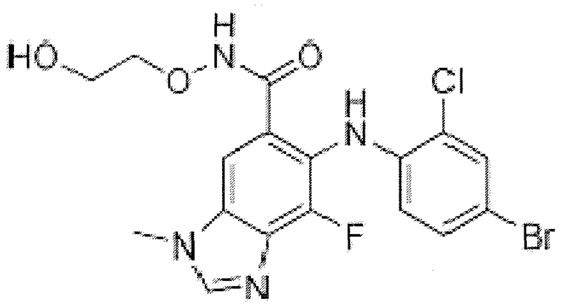

(IX);

AZD8330 as shown in formula (X) below or a pharmaceutically acceptable salt, solvate or hydrate thereof:

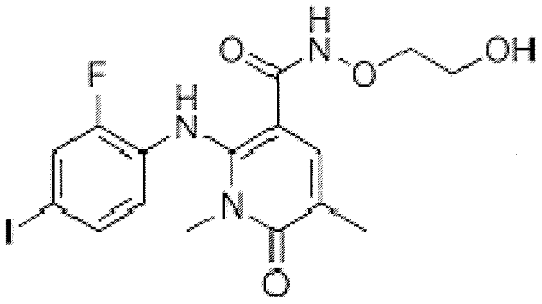

(X);

PD318088 as shown in formula (XI) below or a pharmaceutically acceptable salt, solvate or hydrate thereof:

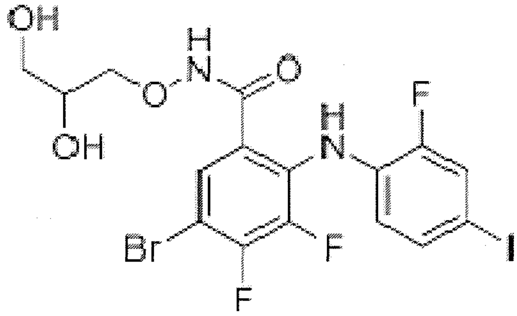

(XI);

PD98059 as shown in formula (XII) below or a pharmaceutically acceptable salt, solvate or hydrate thereof;

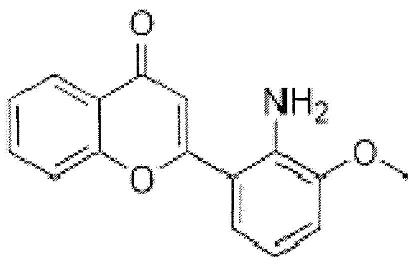

(XII);

TAK-733 as shown in formula (XIII) below or a pharmaceutically acceptable salt, solvate or hydrate thereof:

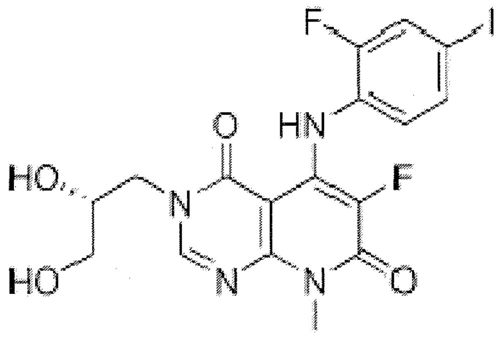

(XIII); and

U0126 as shown in formula (XIV) below or a pharmaceutically acceptable salt, solvate or hydrate thereof

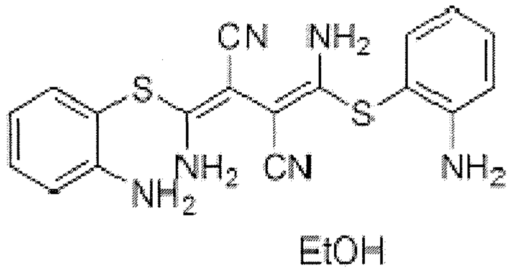

(XIV).

7. A method for determining the susceptibility of a mammalian tumor or cancer cell to a a selective inhibitor of VEGFR-2 tyrosine kinase as defined in any one of items 1 to 5 and an inhibitor of MAP kinase kinase (MEK) as defined in any one of items 1 to 6, said method comprising evaluating the expression level of VEGF and/or VEGFR2, wherein said expression level is indicative of the susceptibility of said cell to said inhibitors.

8. A method for predicting the susceptibility of an individual to a selective inhibitor of VEGFR-2 tyrosine kinase as defined in any one of items 1 to 5 and an inhibitor of MAP kinase kinase (MEK) as defined in any one of items 1 to 6, said method comprising evaluating the expression level of VEGF and/or VEGFR2 in a sample of an individual suffering from cancer, suspected to suffer from cancer, or being prone to suffer from cancer, wherein said expression level is indicative of a susceptible individual to said inhibitors.

9. The method of item 7 or 8, wherein an increase in said expression level as compared to the control is indicative of the susceptibility of said cell or a susceptible individual to said inhibitors.

10. The method of item 9, wherein the expression level is at least 1.5 fold increased as compared to the control.

11. The method of any one of items 7 to 10, wherein the expression level is the mRNA expression level of VEGF and/or VEGFR-2.

12. The method of item 11, wherein the mRNA expression level is assessed by in situ hybridization, micro-arrays, or RealTime PCR.

13. The method of any one of items 7 to 10, wherein the expression level is the protein expression level of VEGF and/or VEGFR-2.

14. The method of item 13, wherein said protein expression level is assessed by immunoassay, gel- or blot-based

methods, IHC, mass spectrometry, flow cytometry, or FACS.

15. Use of a nucleic acid or antibody capable of detecting the expression level of VEGF and/or VEGFR-2 for determining the susceptibility of a cancer cell or a susceptible individual to a tyrosine kinase inhibitor as defined in any one of items 1 to 5 and an inhibitor of MAP kinase kinase (MEK) as defined in any one of items 1 to 6.

16. A kit useful for carrying out the method of any one of items 7 to 14 comprising a nucleic acid or an antibody capable of detecting the expression level of VEGF and/or VEGFR-2.

17. A pharmaceutical composition comprising a selective inhibitor of VEGFR-2 tyrosine kinase as defined in any one of items 1 to 5 and an inhibitor of MAP kinase kinase (MEK) as defined in any one of items 1 to 6 for use in the treatment of cancer in an individual identified by the method of any one of items 7 to 14.

18. A method for the treatment of cancer comprising administering an effective amount of a selective inhibitor of VEGFR-2 tyrosine kinase as defined in any one of items 1 to 5 and an inhibitor of MAP kinase kinase (MEK) as defined in any one of items 1 to 6 to an individual identified by the method of any one of items 7 to 14 in need of such a treatment.

19. The pharmaceutical composition according to any one of items 1, 3 to 6 and 17, or the method according to any one of items 2 to 14 and 18, wherein the cancer is solid cancer.

20. The pharmaceutical composition according to item 19, or the method according to item 19, wherein said solid cancer is selected from the group consisting of lung cancer, such as non-small cell lung cancer, lung adenocarcinoma, and small cell lung cancer.

21. The pharmaceutical composition according to any one of items 1, 3 to 6, 17, 19 and 20, or the method according to any one of items 2 to 14 and 18 to 20, wherein said inhibitors are administered by any one of oral route, parenteral route, intravenous route, subcutaneous route, intranasal route or transdermal route.

22. The pharmaceutical composition according to any one of items 1, 3 to 6, 17, and 19 to 21, or the method according to any one of items 2 to 14 and 18 to 21, wherein said inhibitors are to be administered in a neoadjuvant or adjuvant setting.

23. The method according to any one of items 2 to 14 and 18 to 22, wherein said individual or subject is a human.

24. A composition comprising a selective inhibitor of VEGFR-2 tyrosine kinase or a pharmaceutically acceptable salt, solvate and/or hydrate thereof, as defined in any one of items 1 to 5 and an inhibitor of MAP kinase kinase (MEK) as defined in any one of items 1 to 6.

25. The composition of item 24 for use in medicine.

## Claims

1. A pharmaceutical composition comprising a selective inhibitor of vascular endothelial growth factor receptor-2 (VEGFR-2) tyrosine kinase or a pharmaceutically acceptable salt, solvate and/or hydrate thereof, and an inhibitor of MAP kinase kinase (MEK) or a pharmaceutically acceptable salt, solvate and/or hydrate thereof, for use in the treatment of cancer.

2. The pharmaceutical composition of claim 1,
wherein said selective inhibitor of vascular endothelial growth factor receptor-2 (VEGFR-2) tyrosine kinase is N-(4-bromo-2-fluorophenyl)-6-methoxy-7-[(1-methylpiperidin-4-yl)methoxy]quinazolin-4-amine (Vandetanib/Zactima/ZD6474) as shown in formula (I) below or a pharmaceutically acceptable salt, solvate or hydrate thereof:

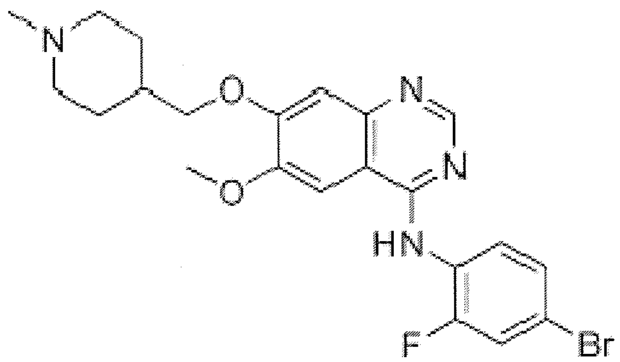

(I); and

wherein said inhibitor of MAP kinase kinase (MEK) is N-[(2R)-2,3-dihydroxypropoxy]-3,4-difluoro-2-[(2-fluoro-4-iodophenyl)amino]-benzamide (PD0325901) as shown in formula (VII) below or a pharmaceutically acceptable salt, solvate or hydrate thereof:

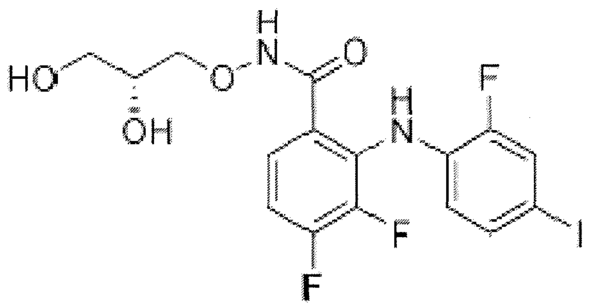

(VII).

3. The pharmaceutical composition according to claim 1, wherein said selective inhibitor of VEGFR-2 tyrosine kinase is selected from the group consisting of
Cabozantinib (BMS-907351) as shown in formula (II) below or a pharmaceutically acceptable salt, solvate or hydrate thereof:

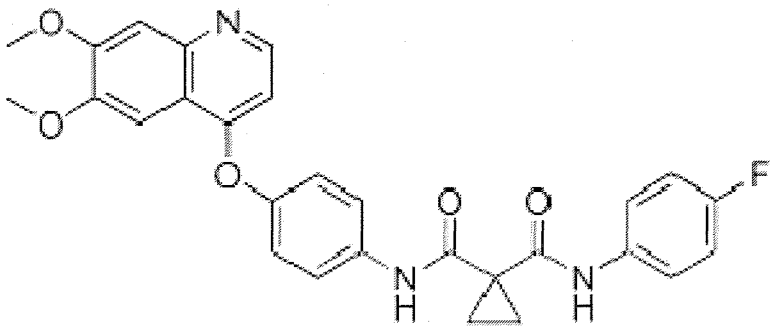

(II);

Apatinib (YN968D1) as shown in formula (III) below or a pharmaceutically acceptable salt, solvate or hydrate thereof:

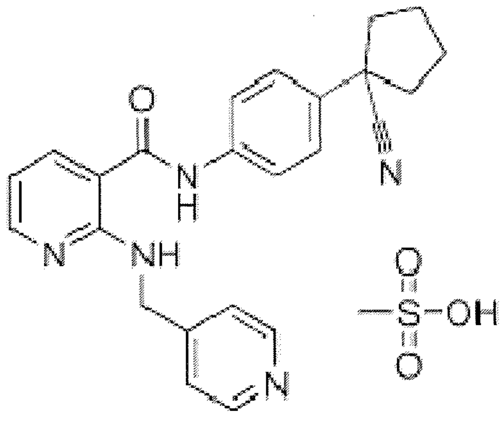

(III);

BMS 794833 as shown in formula (IV) below or a pharmaceutically acceptable salt, solvate or hydrate thereof:

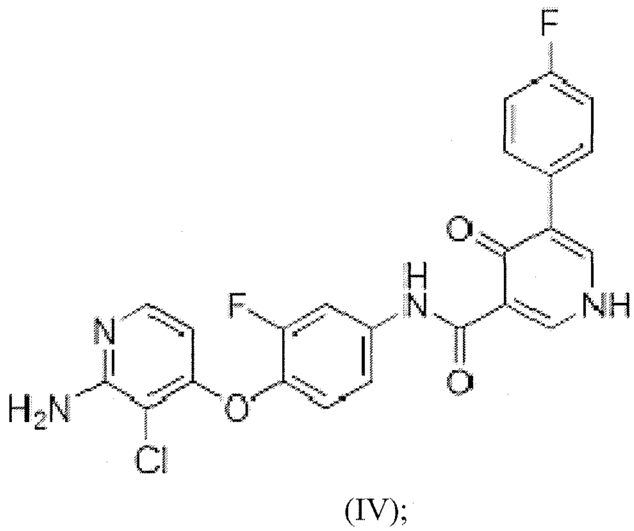

(IV);

Ki8751 as shown in formula (V) below or a pharmaceutically acceptable salt, solvate or hydrate thereof:

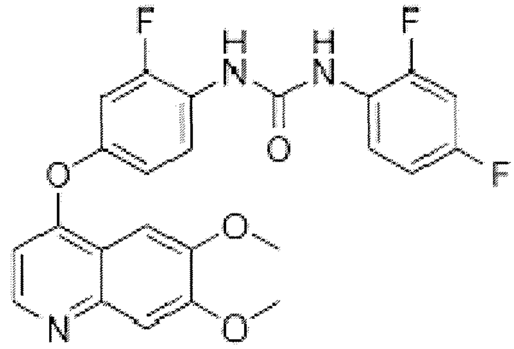

(V); and

OSI-930 as shown in formula (VI) below or a pharmaceutically acceptable salt, solvate or hydrate thereof:

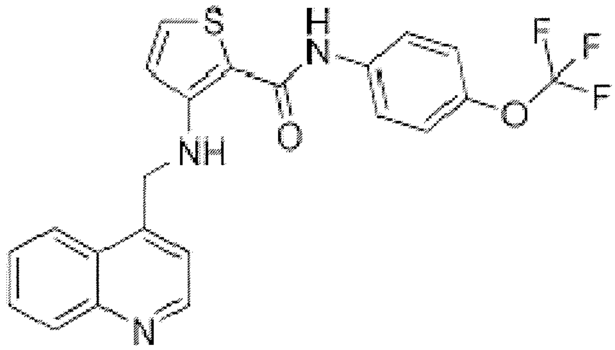

(VI).

**4.** The pharmaceutical composition according to claim 1 or 3, wherein said MEK inhibitor is selected from the group consisting of
AS703026 as shown in formula (VIII) below or a pharmaceutically acceptable salt, solvate or hydrate thereof

:

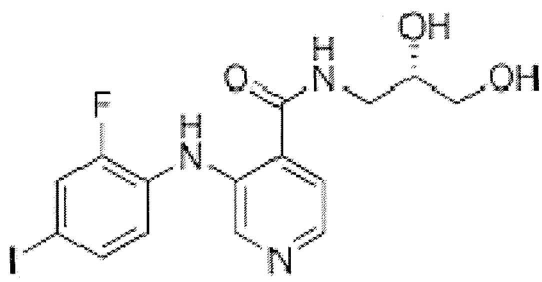

(VIII);

AZD6244 (Selumetinib) as shown in formula (IX) below or a pharmaceutically acceptable salt, solvate or hydrate thereof:

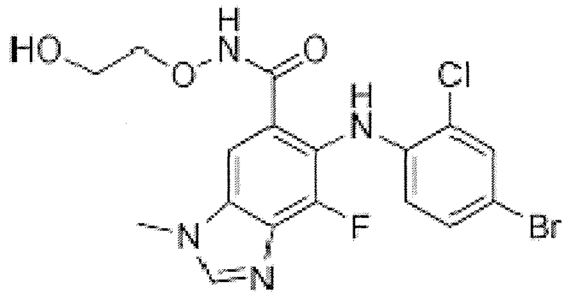

(IX);

AZD8330 as shown in formula (X) below or a pharmaceutically acceptable salt, solvate or hydrate thereof:

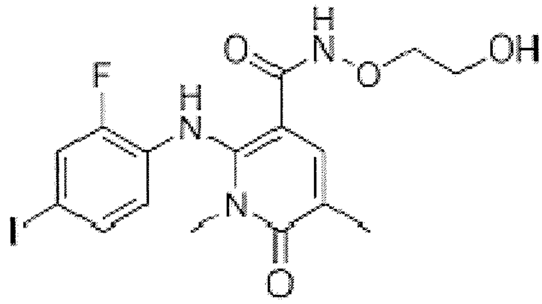

(X);

PD318088 as shown in formula (XI) below or a pharmaceutically acceptable salt, solvate or hydrate thereof:

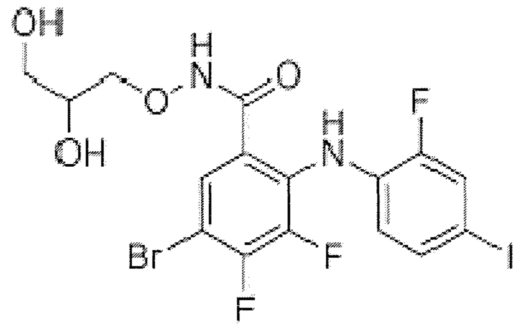

(XI);

PD98059 as shown in formula (XII) below or a pharmaceutically acceptable salt, solvate or hydrate thereof;

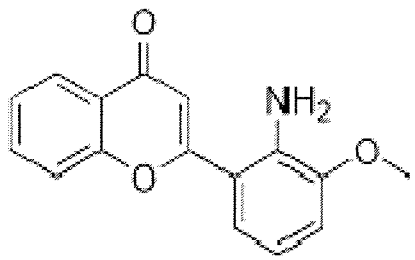

(XII);

TAK-733 as shown in formula (XIII) below or a pharmaceutically acceptable salt, solvate or hydrate thereof:

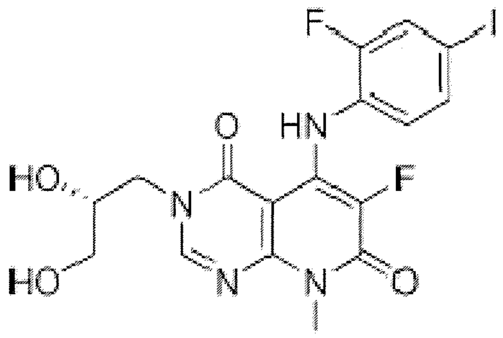

(XIII); and

U0126 as shown in formula (XIV) below or a pharmaceutically acceptable salt, solvate or hydrate thereof

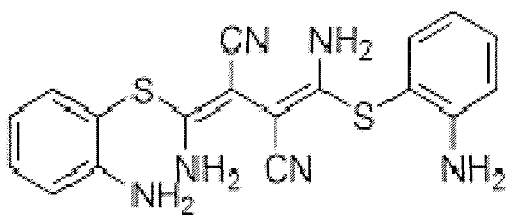

EtOH

(XIV).

**5.** A method for determining the susceptibility of a mammalian tumor or cancer cell to a a selective inhibitor of VEGFR-2 tyrosine kinase as defined in any one of claims 1 to 3 and an inhibitor of MAP kinase kinase (MEK) as defined in any one of claims 1 to 4, said method comprising evaluating the expression level of VEGF and/or VEGFR2, wherein said expression level is indicative of the susceptibility of said cell to said inhibitors.

**6.** A method for predicting the susceptibility of an individual to a selective inhibitor of VEGFR-2 tyrosine kinase as defined in any one of claims 1 to 3 and an inhibitor of MAP kinase kinase (MEK) as defined in any one of claims 1 to 4, said method comprising evaluating the expression level of VEGF and/or VEGFR2 in a sample of an individual suffering from cancer, suspected to suffer from cancer, or being prone to suffer from cancer, wherein said expression level is indicative of a susceptible individual to said inhibitors.

7. The method of claim 5 or 6, wherein an increase in said expression level as compared to the control is indicative of the susceptibility of said cell to said inhibitors or a susceptible individual to said inhibitors.

8. The method of claim 7, wherein the expression level is at least 1.5 fold increased as compared to the control.

9. The method of any one of claims 5 to 8, wherein the expression level is the mRNA expression level of VEGF and/or VEGFR-2, wherein the mRNA expression level is, for example, assessed by in situ hybridization, micro-arrays, or RealTime PCR.

10. The method of any one of claims 5 to 8, wherein the expression level is the protein expression level of VEGF and/or VEGFR-2, wherein said protein expression level is, for example, assessed by immunoassay, gel- or blot-based methods, IHC, mass spectrometry, flow cytometry, or FACS.

11. A pharmaceutical composition comprising a selective inhibitor of VEGFR-2 tyrosine kinase as defined in any one of claims 1 to 3 and an inhibitor of MAP kinase kinase (MEK) as defined in any one of claims 1 to 4 for use in the treatment of cancer in an individual identified by the method of any one of claims 5 to 10.

12. The pharmaceutical composition according to any one of claims 1 to 4 and 11, wherein the cancer is solid cancer.

13. The pharmaceutical composition according to claim 12, wherein said solid cancer is lung cancer, such as non-small cell lung cancerlung adenocarcinoma or small cell lung cancer.

14. A composition comprising a selective inhibitor of VEGFR-2 tyrosine kinase or a pharmaceutically acceptable salt, solvate and/or hydrate thereof, as defined in any one of claims 1 to 3 and an inhibitor of MAP kinase kinase (MEK) as defined in any one of claims 1 to 4.

15. The composition of claim 14 for use in medicine.

Figure 1.

A.

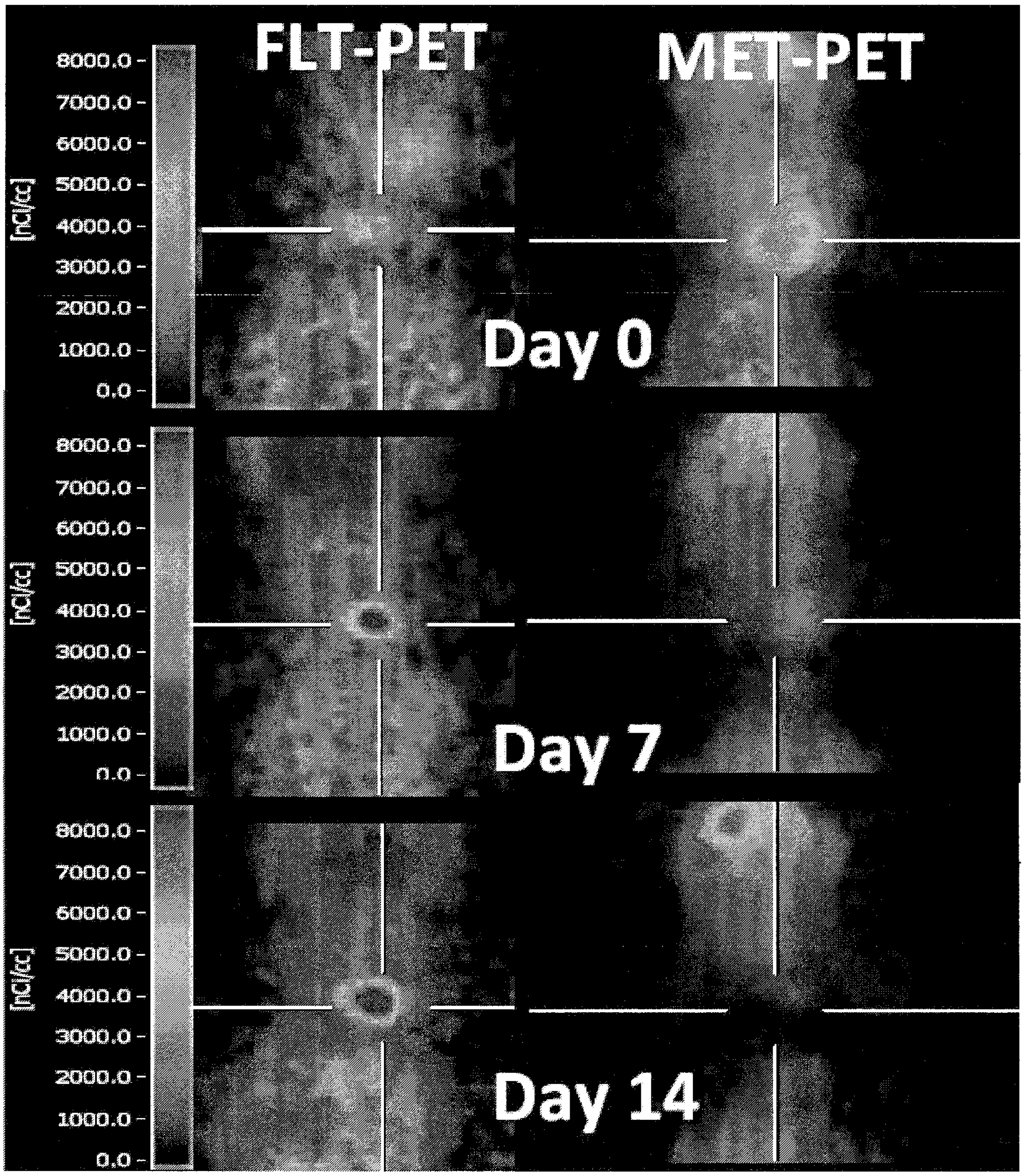
FLT-PET
MET-PET
8000.0
7000.0
6000.0
5000.0
4000.0
3000.0
2000.0
1000.0
0.0
[nCi/cc]
Day 0
Day 7
Day 14

Figure 1 (cont.).

B.

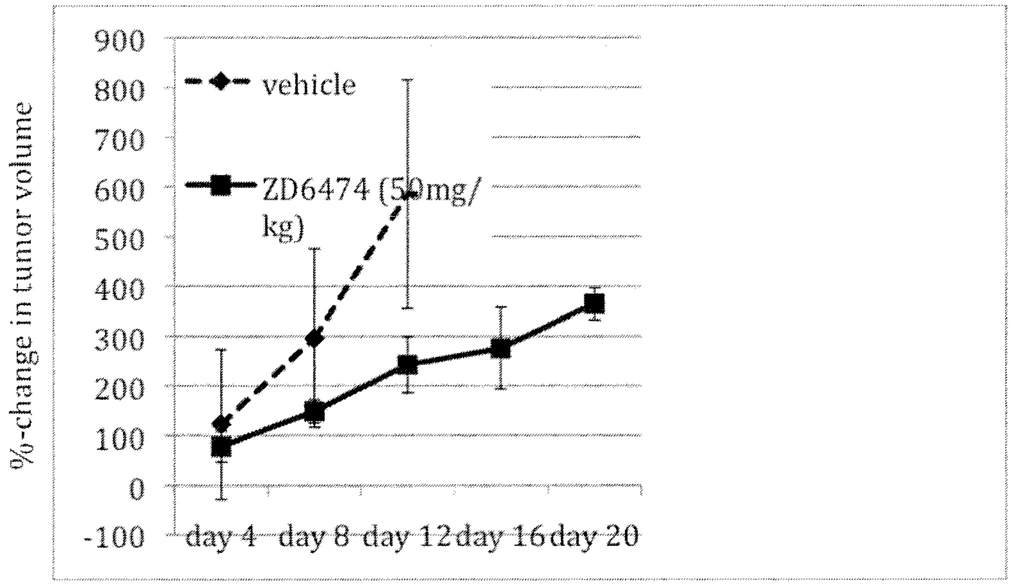


C.

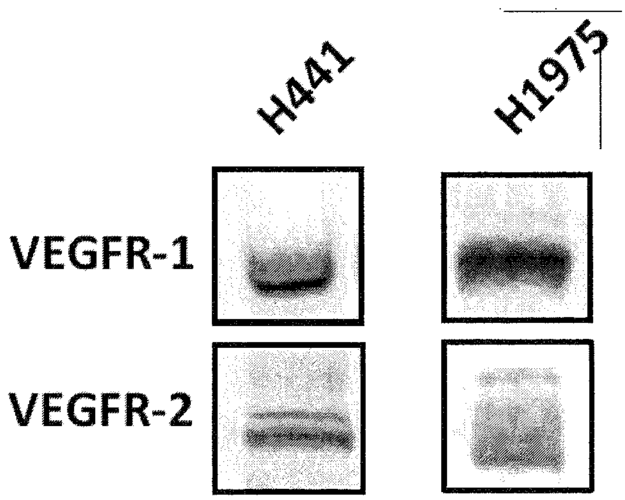

**Figure 1 (cont.).**

**D.**

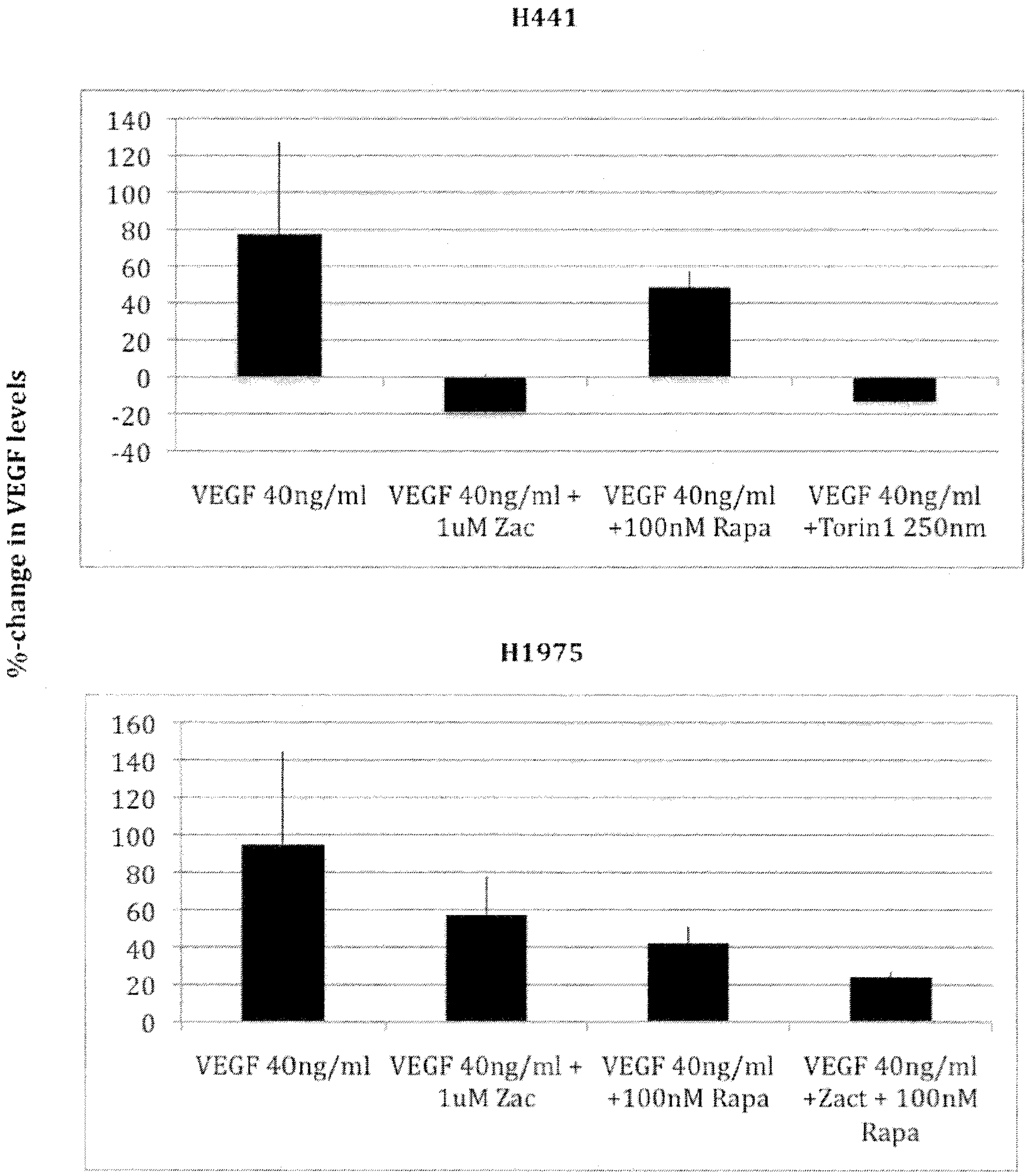

Figure 1 (cont.).

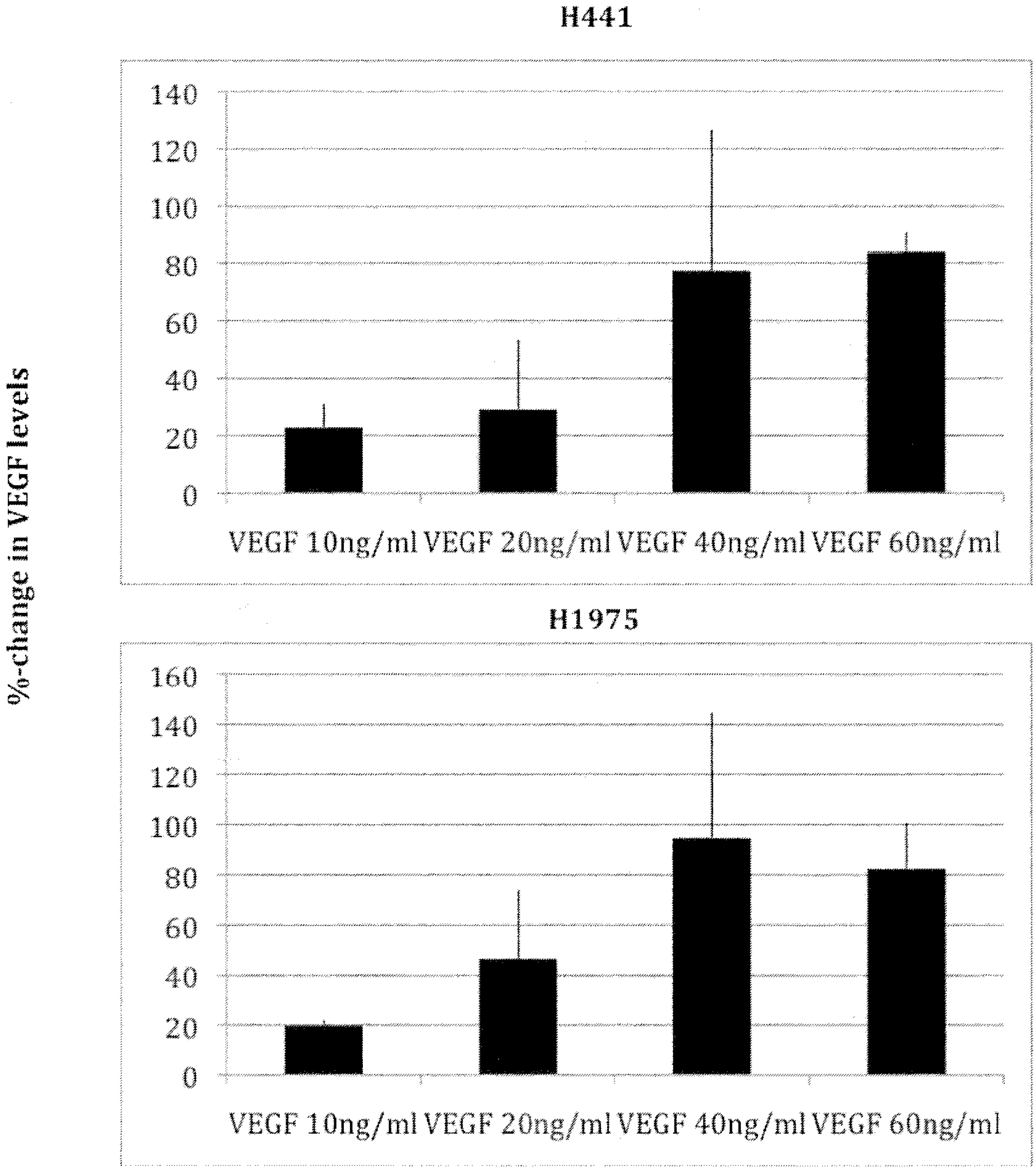

Figure 1 (cont.).

E.

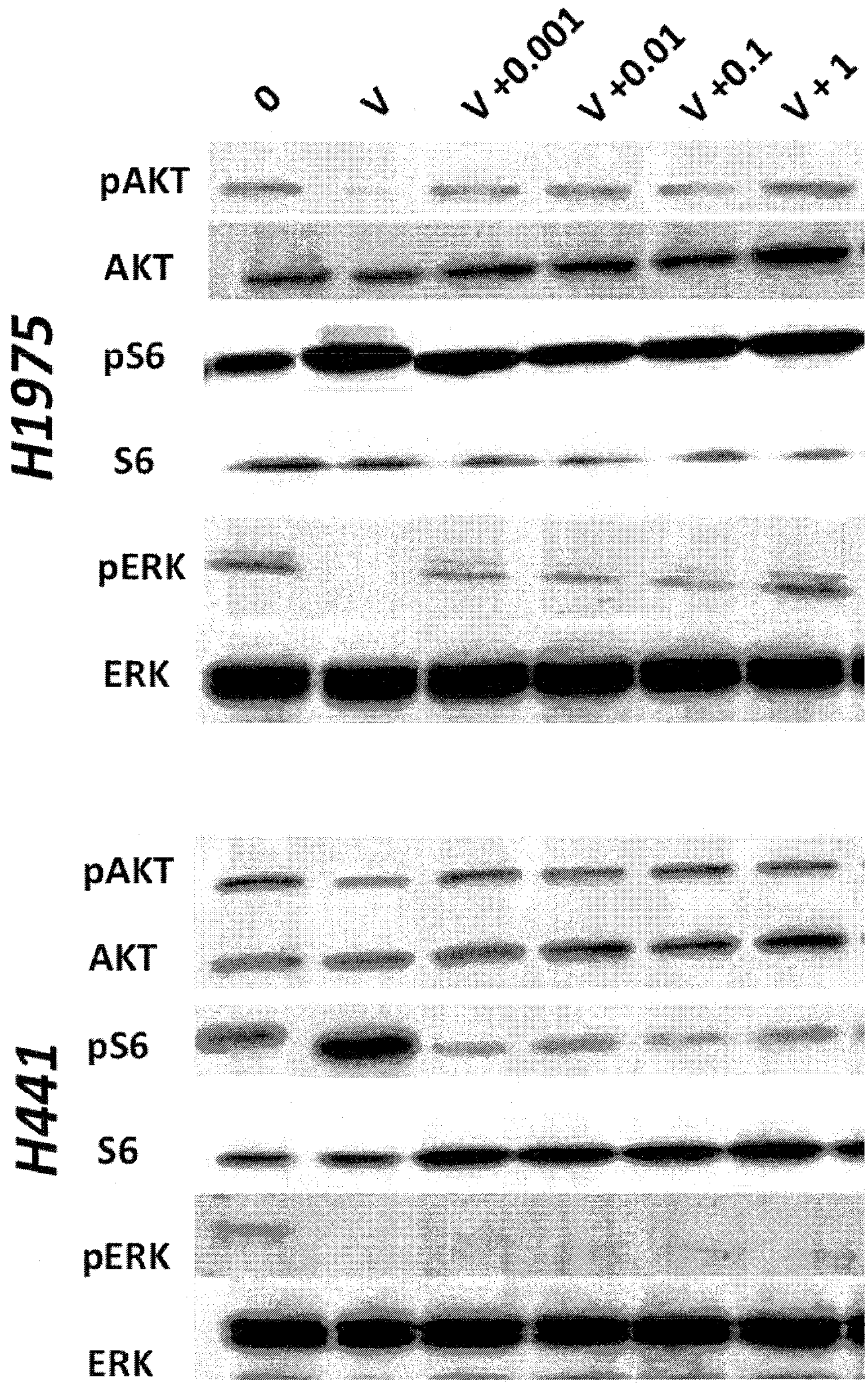

Figure 1 (cont.).

F.

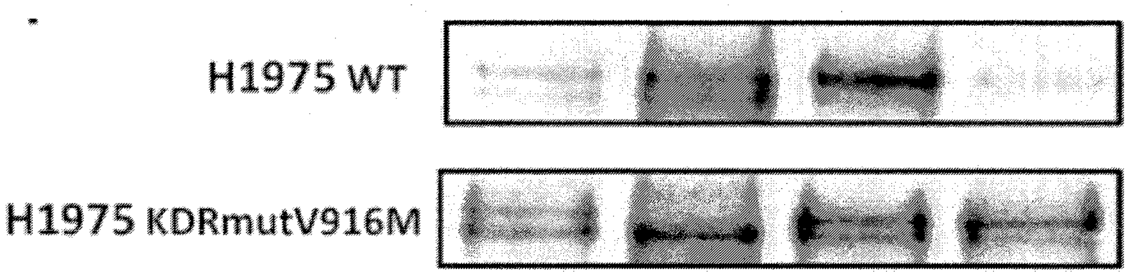


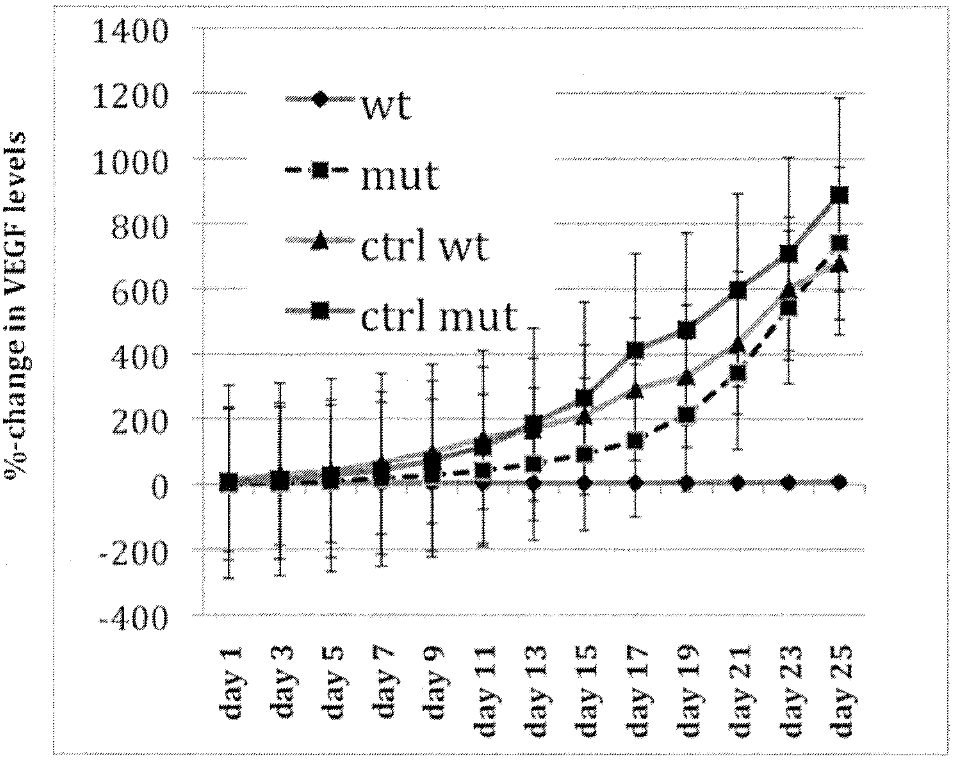

Figure 2.

A.

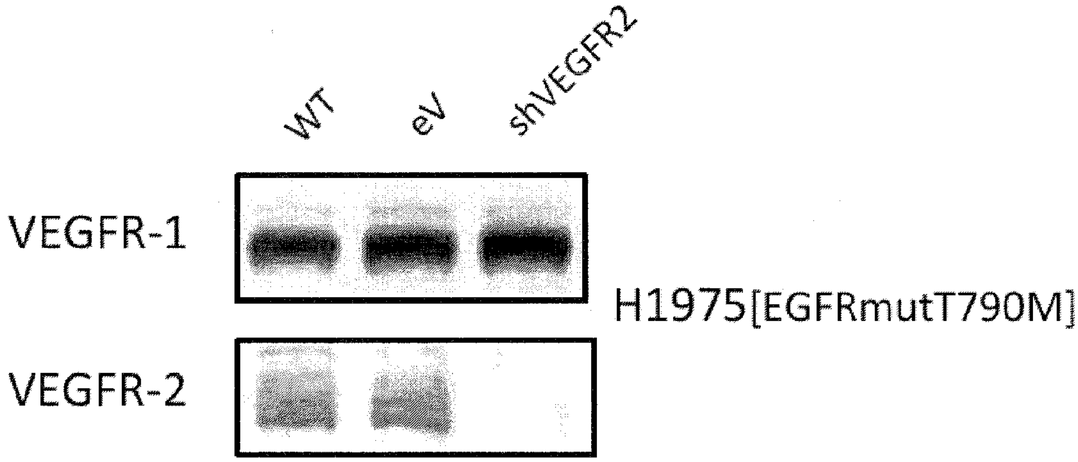
WT
eV
shVEGFR2
VEGFR-1
VEGFR-2
H1975[EGFRmutT790M]

B.

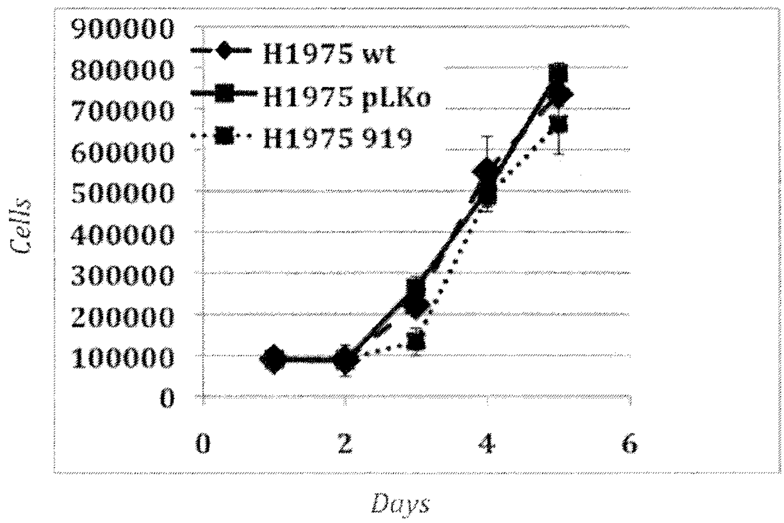
900000
800000
700000
600000
500000
400000
300000
200000
100000
0
H1975 wt
H1975 pLKo
H1975 919
Cells
0
2
4
6
Days

Figure 2 (cont.).

C.

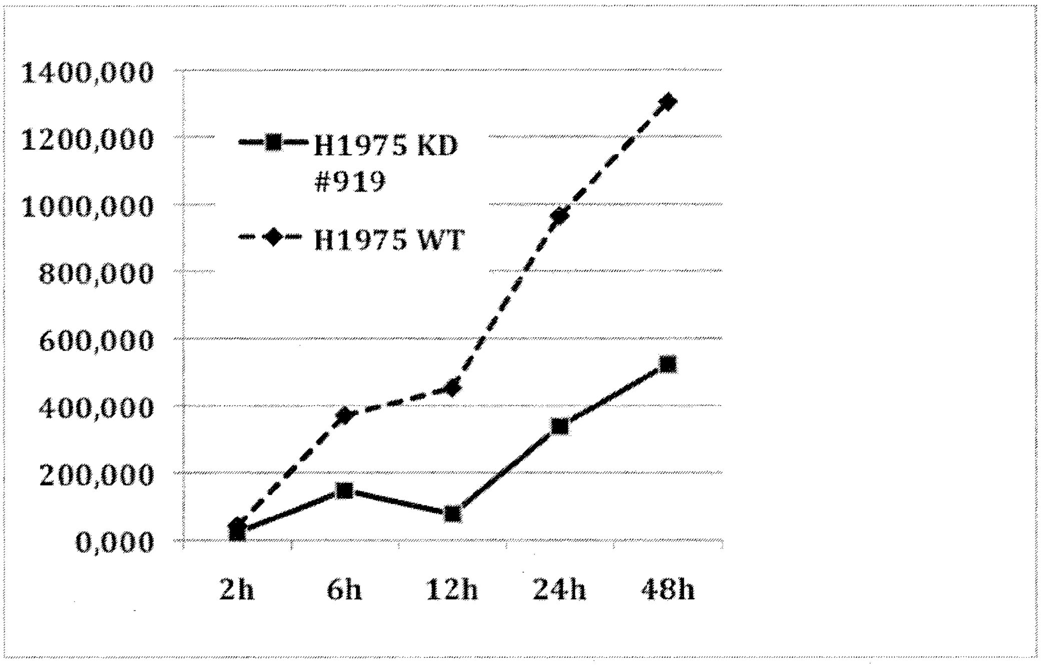


D.

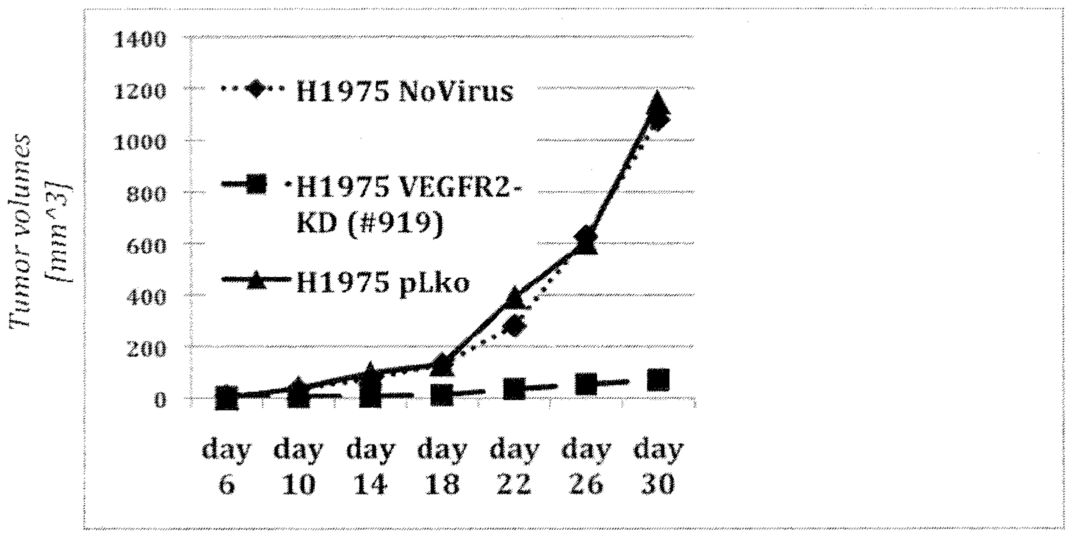

Figure 2 (cont.).

E.

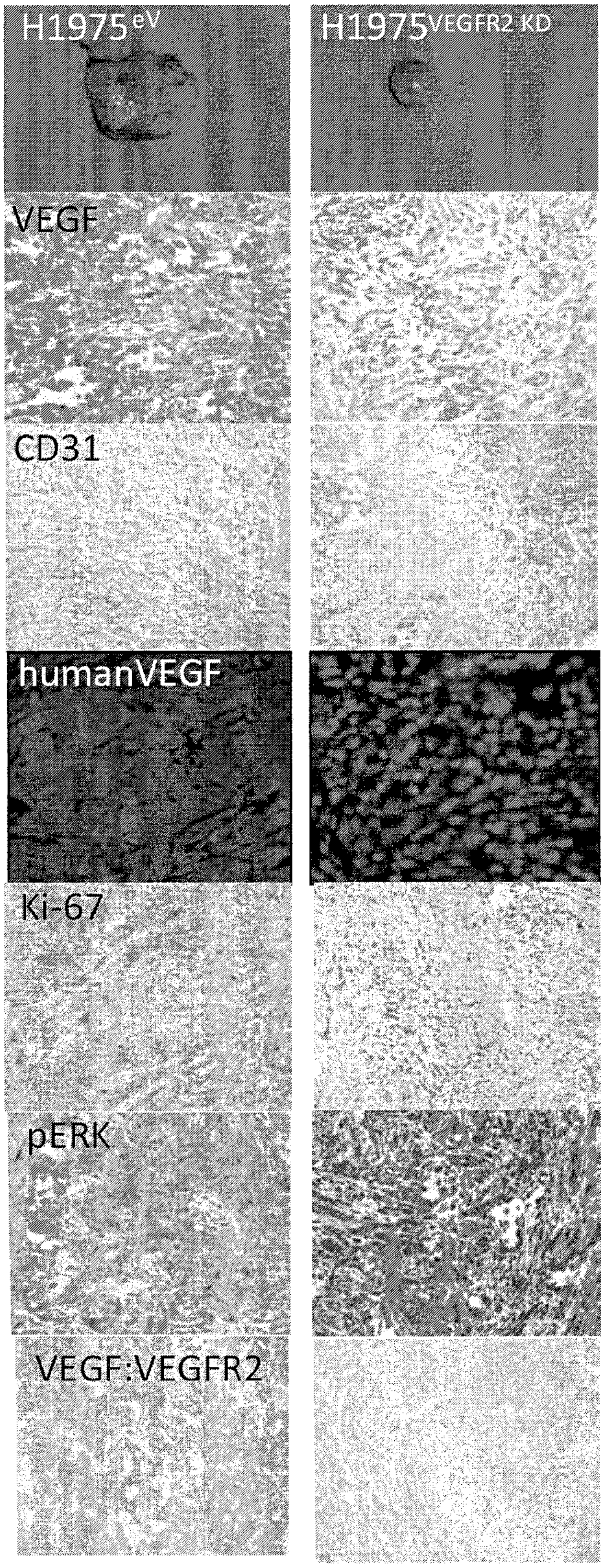

Figure 2 (cont.).

F.

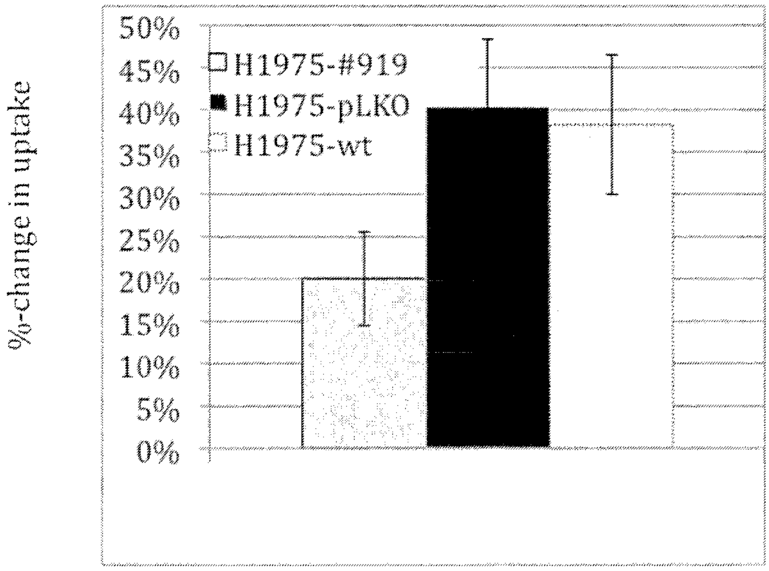

**Figure 3.**

**A.**

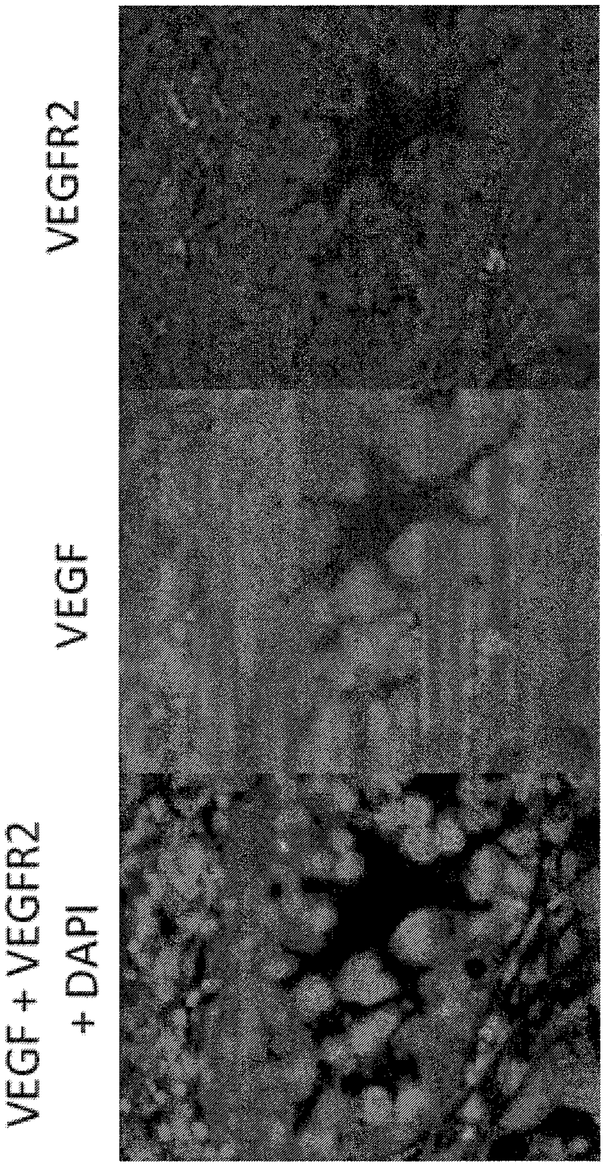

Figure 3 (cont.).

B.

VEGFR2 (p=2.612x10-5)

| VEGF | 0 | 1 | 2 | 3 |
|---|---|---|---|---|
| 0 | 0 | 1 | 0 | 1 |
| 1 | 0 | 11 | 8 | 1 |
| 2 | 0 | 10 | 42 | 16 |
| 3 | 0 | 0 | 13 | 10 |

CD31 (p=2.2x10-11)

| VEGF | 0 | 1 | 2 | 3 |
|---|---|---|---|---|
| 0 | 0 | 0 | 2 | 0 |
| 1 | 0 | 17 | 3 | 0 |
| 2 | 0 | 13 | 52 | 3 |
| 3 | 0 | 3 | 9 | 11 |

VEGFR2 (p=0.01512)

| CD31 | 0 | 1 | 2 | 3 |
|---|---|---|---|---|
| 0 | 0 | 0 | 0 | 0 |
| 1 | 0 | 11 | 19 | 3 |
| 2 | 0 | 11 | 35 | 20 |
| 3 | 0 | 0 | 9 | 5 |

C.

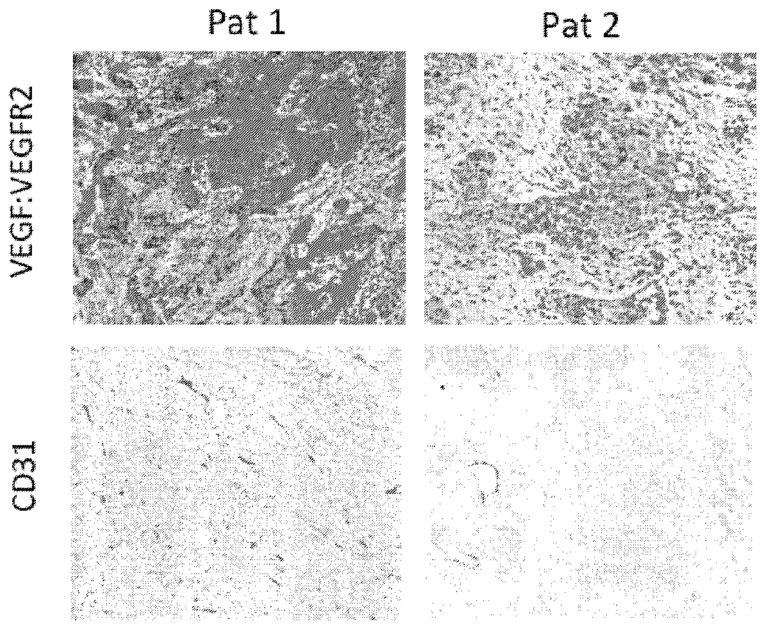

Figure 4.

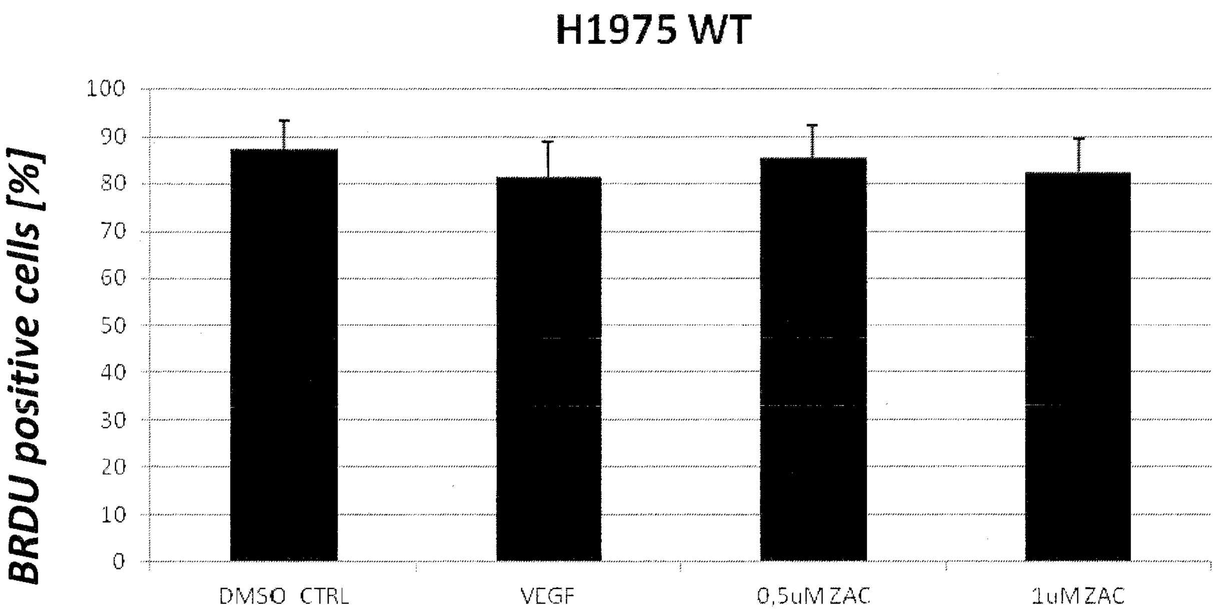
H1975 WT
BRDU positive cells [%]
100
90
80
70
60
50
40
30
20
10
0
DMSO CTRL
VEGF
0,5uM ZAC
1uM ZAC

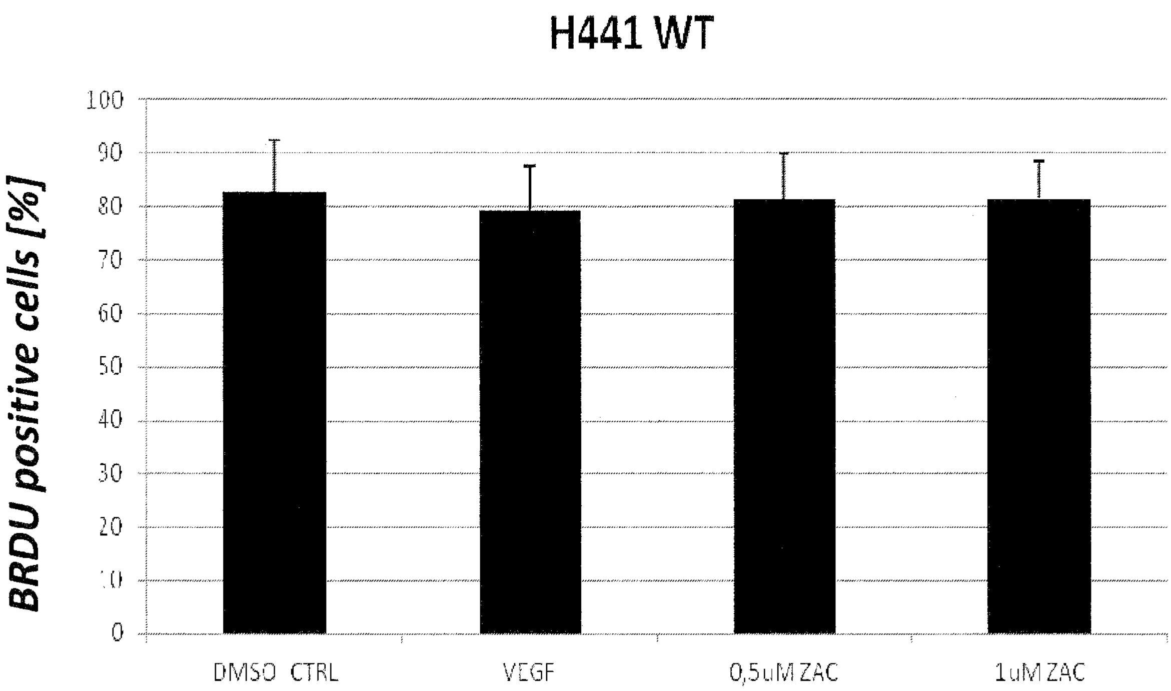
H441 WT
BRDU positive cells [%]
100
90
80
70
60
50
40
30
20
10
0
DMSO CTRL
VEGF
0,5uM ZAC
1uM ZAC

Figure 5.

A.

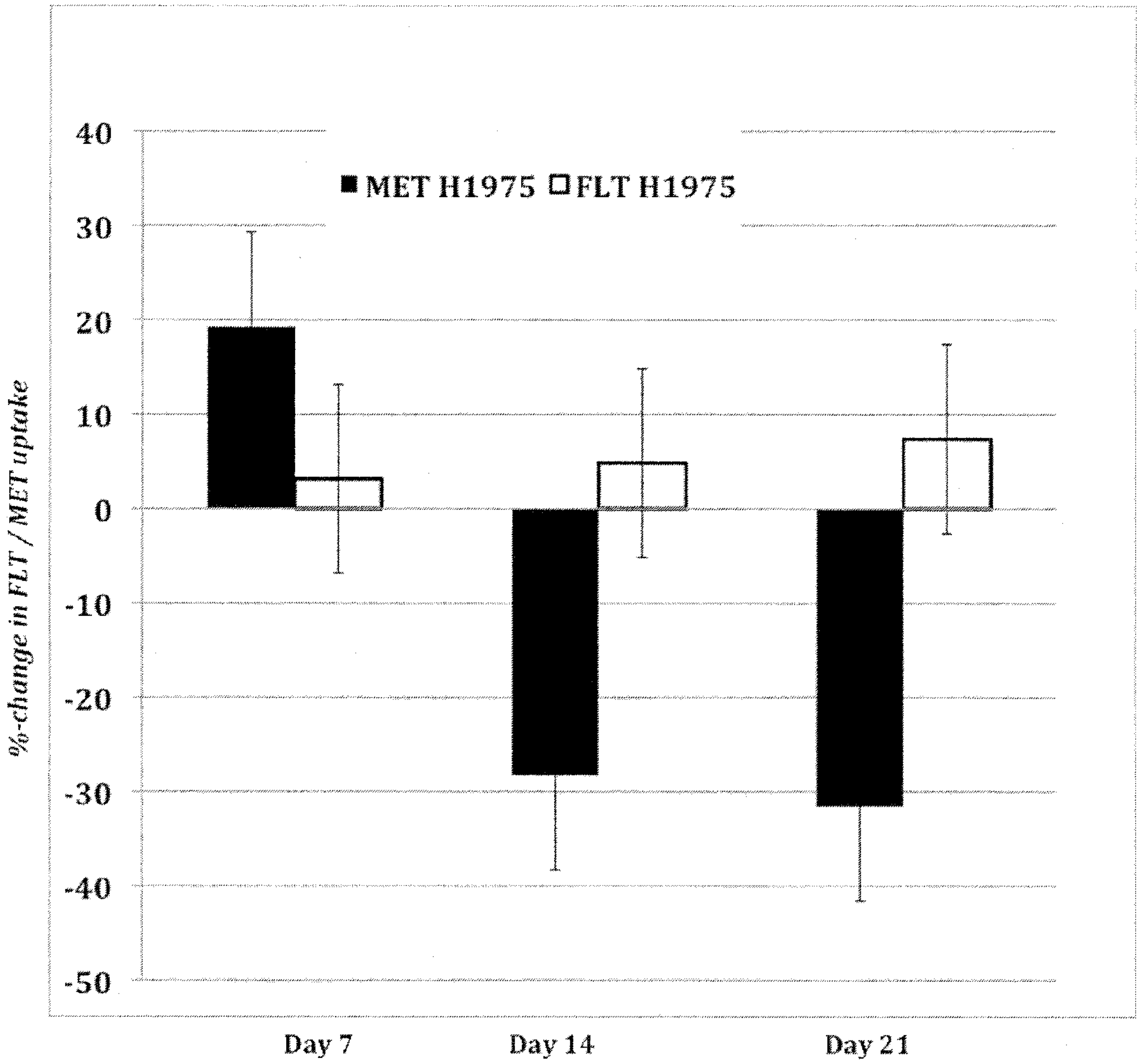
40
30
20
10
0
-10
-20
-30
-40
-50
■ MET H1975 □ FLT H1975
%-change in FLT / MET uptake
Day 7
Day 14
Day 21

B.

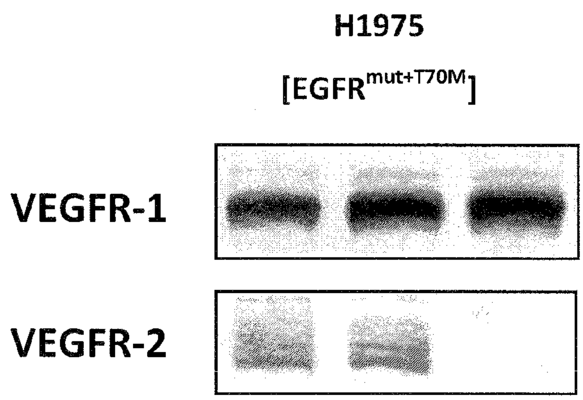
H1975
[EGFR mut+T70M]
VEGFR-1
VEGFR-2

Figure 6.

A.

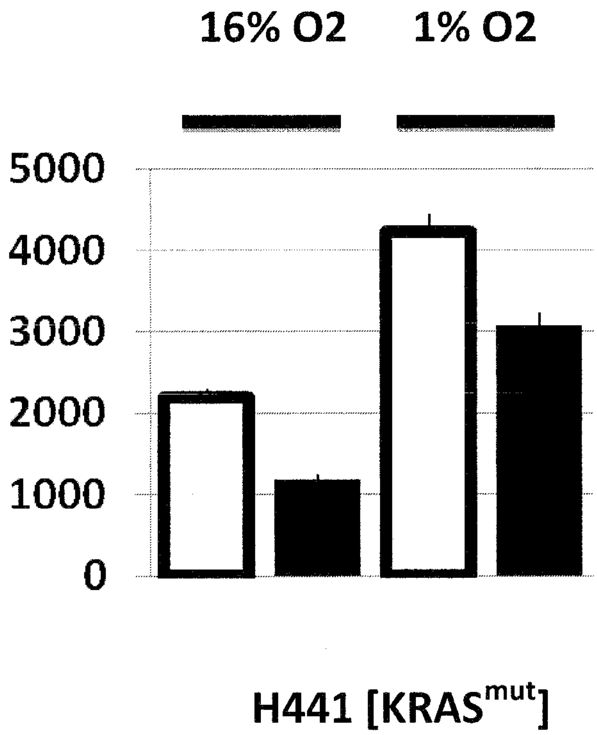
16% O2
1% O2
5000
4000
3000
2000
1000
0
H441 [KRASmut]

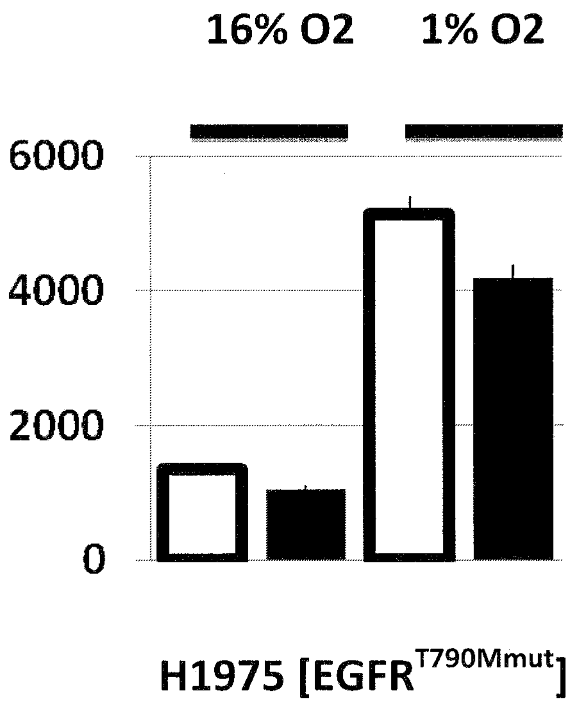
16% O2
1% O2
6000
4000
2000
0
H1975 [EGFRT790Mmut]

Figure 6 (cont.).

B.

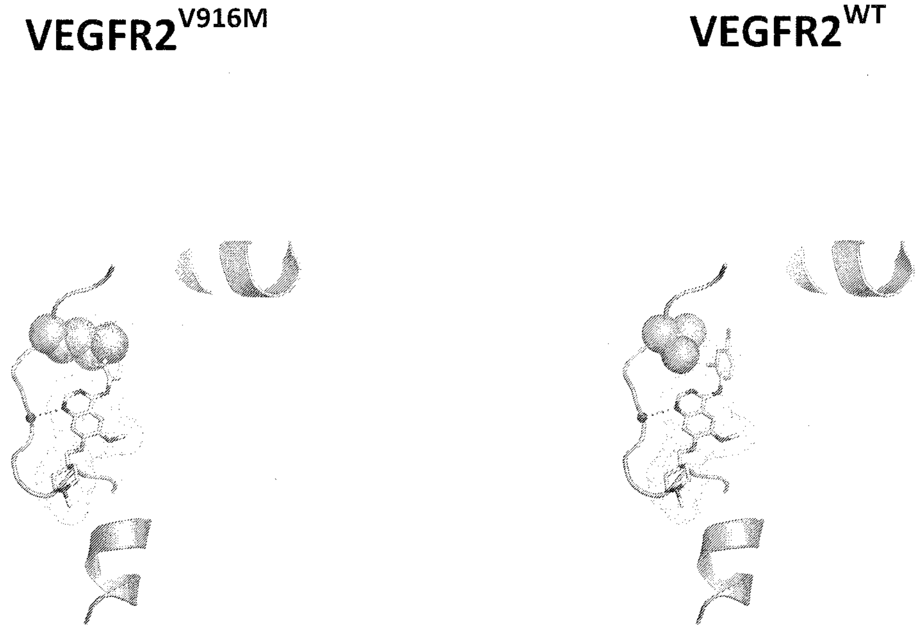

Figure 7.

A.

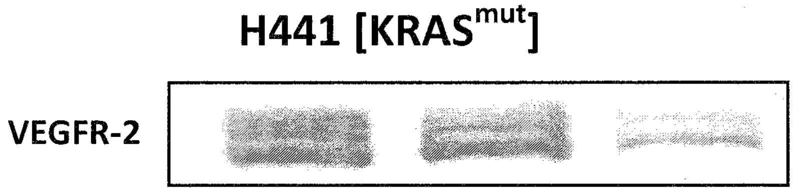
H441 [KRAS$^{mut}$]
VEGFR-2

B.

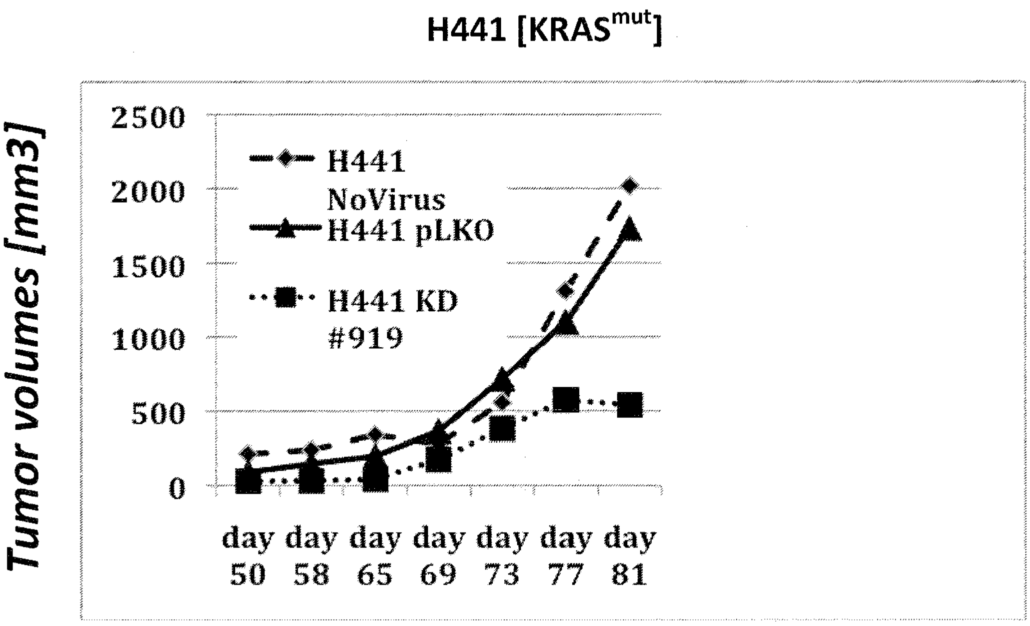
H441 [KRAS$^{mut}$]
Tumor volumes [mm3]
2500
2000
1500
1000
500
0
H441 NoVirus
H441 pLKO
H441 KD #919
day 50
day 58
day 65
day 69
day 73
day 77
day 81

Figure 8.

H1975 Vehicle

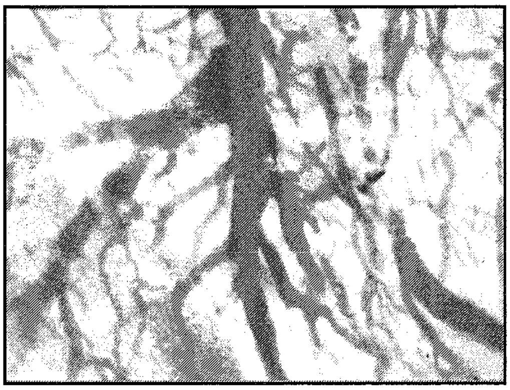

H1975 ZD6474 treated

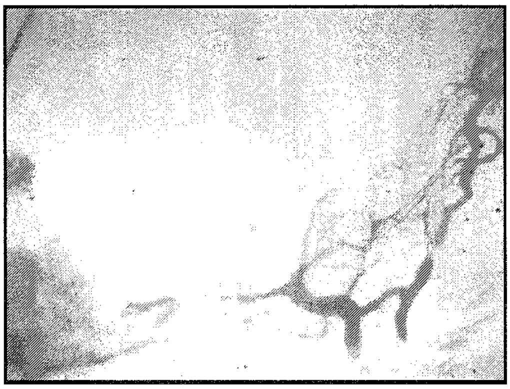

Figure 9.

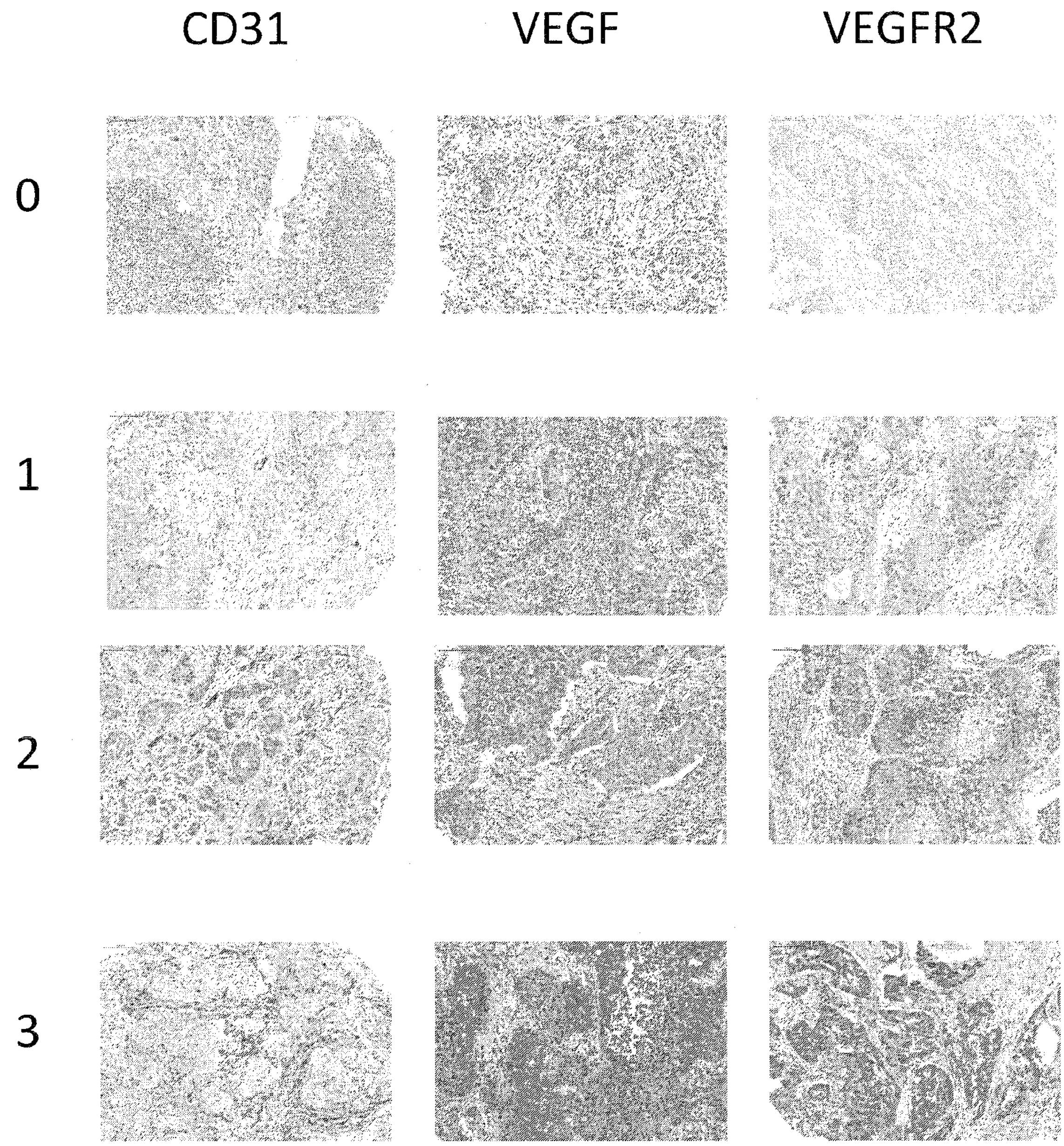

Figure 10.

A

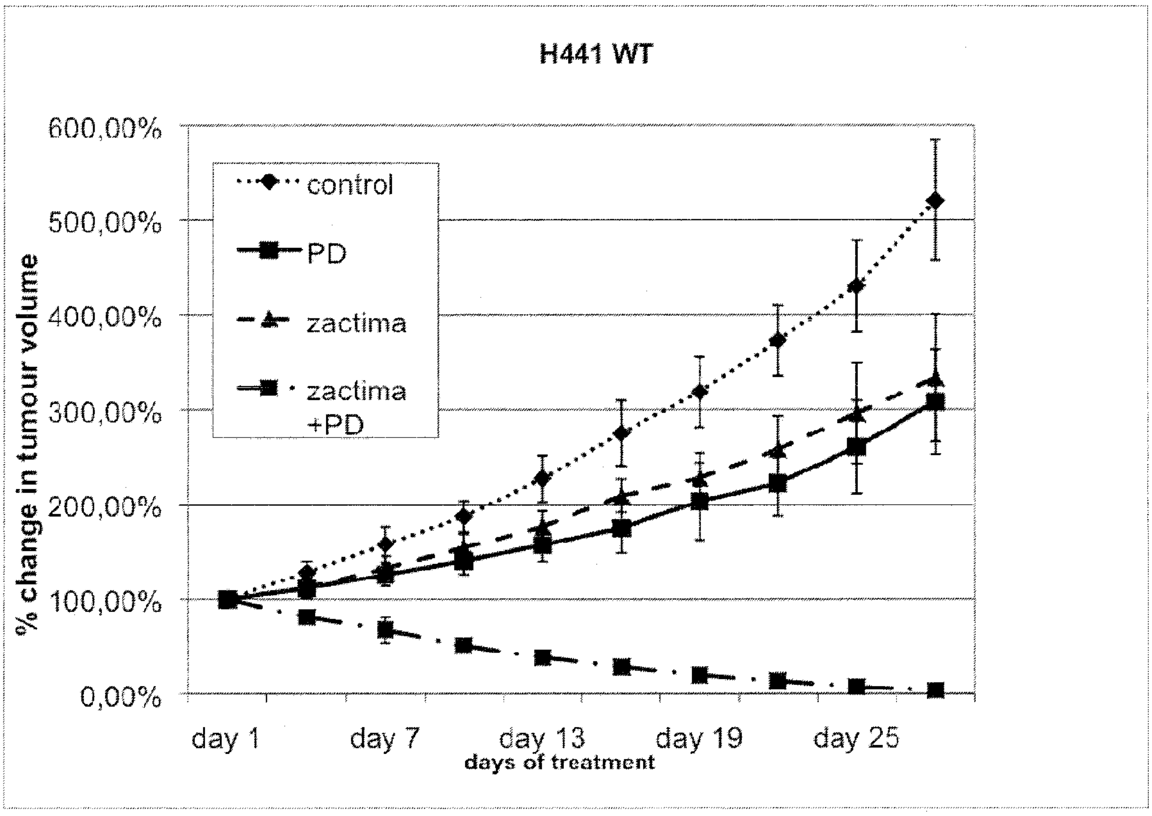
H441 WT
600,00%
500,00%
400,00%
300,00%
200,00%
100,00%
0,00%
% change in tumour volume
control
PD
zactima
zactima +PD
day 1
day 7
day 13
day 19
day 25
days of treatment

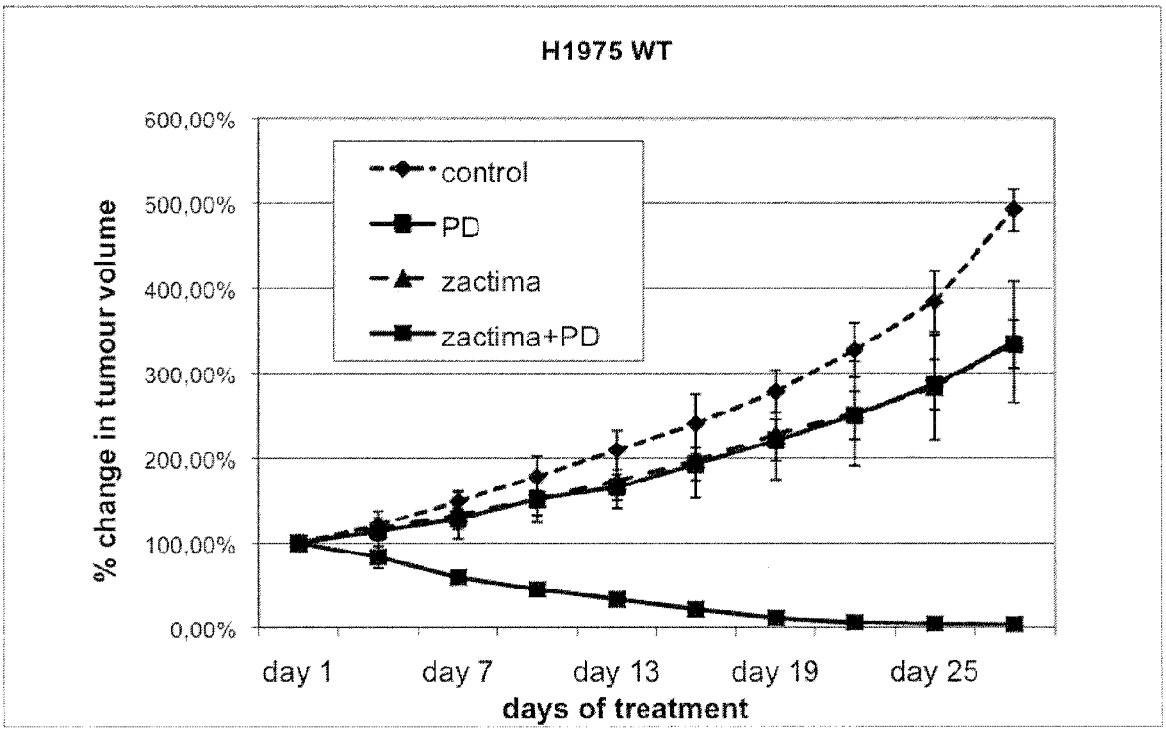
H1975 WT
600,00%
500,00%
400,00%
300,00%
200,00%
100,00%
0,00%
% change in tumour volume
control
PD
zactima
zactima+PD
day 1
day 7
day 13
day 19
day 25
days of treatment

B

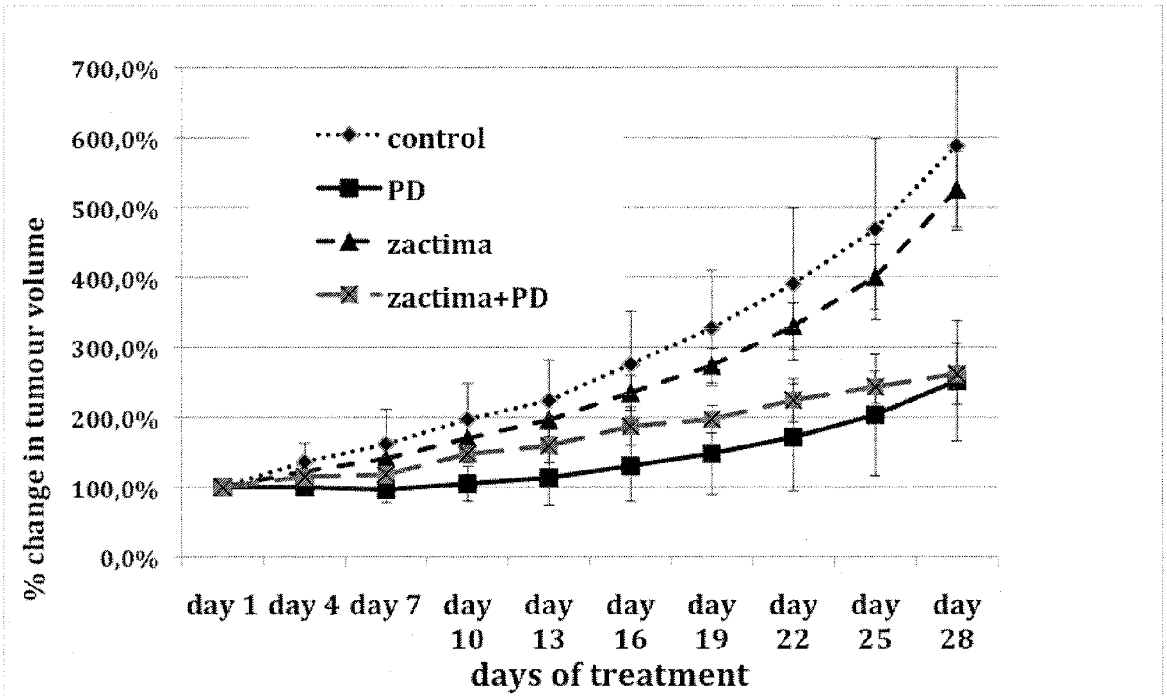
700,0%
600,0%
500,0%
400,0%
300,0%
200,0%
100,0%
0,0%
% change in tumour volume
control
PD
zactima
zactima+PD
day 1 day 4 day 7 day 10 day 13 day 16 day 19 day 22 day 25 day 28
days of treatment

C

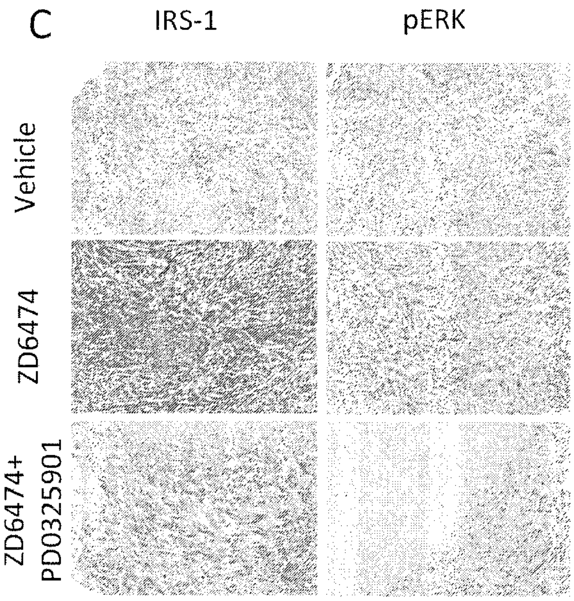
IRS-1
pERK
Vehicle
ZD6474
ZD6474+
PD0325901

D

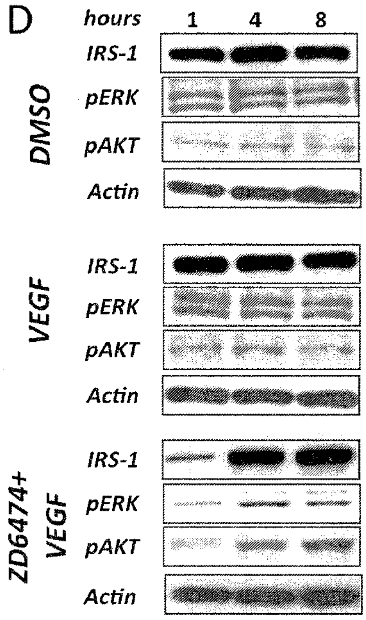
hours
1
4
8
DMSO
IRS-1
pERK
pAKT
Actin
VEGF
IRS-1
pERK
pAKT
Actin
ZD6474+
VEGF
IRS-1
pERK
pAKT
Actin

Europäisches Patentamt
European Patent Office
Office européen des brevets

# EUROPEAN SEARCH REPORT

Application Number
EP 12 15 4880

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LOCKERBIE OWEN: "Combined targeted inhibition of EGFR tyrosine kinase activity and MEK-1 in human colon cancer cells.", PROCEEDINGS OF THE AMERICAN ASSOCIATION FOR CANCER RESEARCH ANNUAL MEETING, vol. 46, April 2005 (2005-04), pages 292-293, XP009160365, & 96TH ANNUAL MEETING OF THE AMERICAN-ASSOCIATION-FOR-CANCER-RESEARCH; ANAHEIM, CA, USA; APRIL 16 -20, 2005 ISSN: 0197-016X<br>* the whole document *<br>----- | 1,2,4-15 | INV.<br>A61K31/16<br>A61K31/517<br>A61P35/00<br>A61K45/06 |
| X | C NISHIOKA ET AL: "ZD6474 induces growth arrest and apoptosis of human leukemia cells, which is enhanced by concomitant use of a novel MEK inhibitor, AZD6244", LEUKEMIA, vol. 21, no. 6, 15 March 2007 (2007-03-15), pages 1308-1310, XP055030822, ISSN: 0887-6924, DOI: 10.1038/sj.leu.2404647<br>* page 1308 - page 1309 *<br>-----<br>-/-- | 1,2,4-15 | TECHNICAL FIELDS SEARCHED (IPC)<br>A61K<br>A61P |

~~The present search report has been drawn up for all claims~~

| Place of search | Date of completion of the search | Examiner |
|---|---|---|
| Munich | 27 June 2012 | Büttner, Ulf |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 15 4880

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | E. P. JANE ET AL: "Abrogation of Mitogen-Activated Protein Kinase and Akt Signaling by Vandetanib Synergistically Potentiates Histone Deacetylase Inhibitor-Induced Apoptosis in Human Glioma Cells", JOURNAL OF PHARMACOLOGY AND EXPERIMENTAL THERAPEUTICS, vol. 331, no. 1, 1 October 2009 (2009-10-01), pages 327-337, XP055030810, ISSN: 0022-3565, DOI: 10.1124/jpet.109.155705 * page 336, paragraph 1 * ----- | 1,2,4-15 | |
| X | US 2009/246198 A1 (DONG QING [US] ET AL) 1 October 2009 (2009-10-01) * claims 99,100,104 * ----- | 1,2,5-15 | |
| X,D | O. TAKAHASHI ET AL: "Combined MEK and VEGFR Inhibition in Orthotopic Human Lung Cancer Models Results in Enhanced Inhibition of Tumor Angiogenesis, Growth, and Metastasis", CLINICAL CANCER RESEARCH, vol. 18, no. 6, 24 January 2012 (2012-01-24), pages 1641-1654, XP055030840, ISSN: 1078-0432, DOI: 10.1158/1078-0432.CCR-11-2324 * page 1647, column 2 * ----- | 1,2,4-15 | TECHNICAL FIELDS SEARCHED (IPC) |
| X,D | WO 2008/125820 A1 (ASTRAZENECA AB [SE]; ASTRAZENECA UK LTD [GB]; WEDGE STEPHEN ROBERT [GB]) 23 October 2008 (2008-10-23) * claims * ----- -/-- | 1,2,4-15 | |

~~The present search report has been drawn up for all claims~~

3

| Place of search | Date of completion of the search | Examiner |
|---|---|---|
| Munich | 27 June 2012 | Büttner, Ulf |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 15 4880

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WEDGE S R ET AL: "AZD2171: A HIGHLY POTENT, ORALLY BIOAVAILABLE, VASCULAR ENDOTHELIAL GROWTH FACTOR RECEPTOR-2 TYROSINE KINASE INHIBITOR FOR THE TREATMENT OF CANCER", CANCER RESEARCH, AMERICAN ASSOCIATION FOR CANCER RESEARCH, US, vol. 65, no. 10, 15 May 2005 (2005-05-15), pages 4389-4400, XP008066714, ISSN: 0008-5472, DOI: 10.1158/0008-5472.CAN-04-4409 * table 1 * ----- | 1,2,4-15 | |
| A | SLEMANN D ET AL: "91 POSTER Impact of tumor VEGF expression level on the in situ efficacy of the VEGFR2 associated tyrosine kinase inhibitor ZD6474", EUROPEAN JOURNAL OF CANCER. SUPPLEMENT, PERGAMON, OXFORD, GB, vol. 4, no. 12, 1 November 2006 (2006-11-01), pages 31-32, XP027888551, ISSN: 1359-6349 [retrieved on 2006-11-01] * the whole document * ----- | 1-15 | TECHNICAL FIELDS SEARCHED (IPC) |
| A,D | WEDGE STEPHEN R ET AL: "ZD6474 inhibits vascular endothelial growth factor signaling, angiogenesis, and tumor growth following oral administration", CANCER RESEARCH, AMERICAN ASSOCIATION FOR CANCER RESEARCH, US, vol. 62, no. 16, 15 August 2002 (2002-08-15), pages 4645-4655, XP002425560, ISSN: 0008-5472 * table 1 * ----- | 1-15 | |

~~The present search report has been drawn up for all claims~~

3

| Place of search | Date of completion of the search | Examiner |
|---|---|---|
| Munich | 27 June 2012 | Büttner, Ulf |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number
EP 12 15 4880

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

2(completely); 1, 4-15(partially)

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number

EP 12 15 4880

The Search Division considers that the present European patent application does not comply with the requirements of unity of invention and relates to several inventions or groups of inventions, namely:

1. claims: 2(completely); 1, 4-15(partially)

   use of Vandetanib in combination with a MEK inhibitor

   ---

2. claims: 1-15(partially)

   use of Cabozantinib in combination with a MEK inhibitor

   ---

3. claims: 1-15(partially)

   use of Apatinib in combination with a MEK inhibitor

   ---

4. claims: 1-15(partially)

   use of BMS 794833 in combination with a MEK inhibitor

   ---

5. claims: 1-15(partially)

   use of Ki875 in combination with a MEK inhibitor

   ---

6. claims: 1-15(partially)

   use of OSI-990 in combination with a MEK inhibitor

   ---

## ANNEX TO THE EUROPEAN SEARCH REPORT ON EUROPEAN PATENT APPLICATION NO. EP 12 15 4880

This annex lists the patent family members relating to the patent documents cited in the above-mentioned European search report.
The members are as contained in the European Patent Office EDP file on
The European Patent Office is in no way liable for these particulars which are merely given for the purpose of information.

27-06-2012

| Patent document cited in search report | Publication date | Patent family member(s) | Publication date |
|---|---|---|---|
| US 2009246198 A1 | 01-10-2009 | US 2009246198 A1 | 01-10-2009 |
| | | WO 2009146034 A2 | 03-12-2009 |
| WO 2008125820 A1 | 23-10-2008 | CN 101678001 A | 24-03-2010 |
| | | EP 2134340 A1 | 23-12-2009 |
| | | JP 2010523634 A | 15-07-2010 |
| | | US 2010130519 A1 | 27-05-2010 |
| | | WO 2008125820 A1 | 23-10-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008125820 A
- US 20100130519 A
- WO 0047212 A
- US 3773919 A
- EP 58481 A
- EP 133988 A
- DE 3218121
- EP 52322 A
- EP 36676 A
- EP 88046 A
- EP 143949 A
- EP 142641 A
- JP 58118008 A
- US 4485045 A
- US 4544545 A
- EP 102324 A
- US 5525711 A
- US 4711955 A
- US 5792608 A
- EP 302175 A


### Non-patent literature cited in the description


- **TAKAHASHI.** *Clin Cancer Res,* 2012
- **WEDGE.** *Cancer Res,* 2002, vol. 62, 4645-4655
- **RYAN.** *British Journal of Cancer,* 2005, vol. 92, S6-S 13
- **MOSHINSKY DJ ; RUSLIM L ; BLAKE RA ; TANG F.** *J Biomol Screen.,* August 2003, vol. 8 (4), 447-52
- **SIDMAN, U. et al.** *Biopolymers,* 1983, vol. 22, 547-556
- **R. LANGER et al.** *J. Biomed. Mater. Res.,* 1981, vol. 15, 167-277
- **R. LANGER.** *Chem. Tech.,* 1982, vol. 12, 98-105
- **EPSTEIN et al.** *Proc. Natl. Acad. Sci. (USA,* 1985, vol. 82, 3688-3692
- **HWANG et al.** *Proc. Natl. Acad. Sci. (USA,* 1980, vol. 77, 4030-4034
- Current Protocols in Neuroscience. John Wiley&Sons, 2001
- *Methods in Enzymology,* 1987, vol. 153, 385-516
- **BITTER et al.** *Methods in Enzymology,* 1987, vol. 153, 516-544
- **SAWERS et al.** *Applied Microbiology and Biotechnology,* 1996, vol. 46, 1-9
- **BILLMAN-JACOBE.** *Current Opinion in Biotechnology,* 1996, vol. 7, 500-4
- **HOCKNEY.** *Trends in Biotechnology,* 1994, vol. 12, 456-463
- **GRIFFITHS et al.** *Methods in Molecular Biology,* 1997, vol. 75, 427-440
- **SAMBROOK ; RUSSELL.** Molecular Cloning: A Laboratory Manual. CSH Press, 2001
- Methods in Yeast Genetics, A Laboratory Course Manual. Cold Spring Harbor Laboratory Press, 1990
- **SAMBROOK ; RUSSELL.** Molecular Cloning, A Laboratory Manual. Cold Spring Harbor Laboratory, 2001
- **AUSUBEL.** Current Protocols in Molecular Biology. Green Publishing Associates and Wiley Interscience, 1989
- Nucleic acid hybridization, a practical approach. IRL Press Oxford, 1985
- **THOMPSON.** *Nucl. Acids Res.,* 1994, vol. 2, 4673-4680
- **BRUTLAG.** *Comp. App. Biosci.,* 1990, vol. 6, 237-245
- **ALTSCHUL.** *Nucl. Acids Res.,* 1997, vol. 25, 3389-3402
- **ALTSCHUL.** *J. Mol. Evol.,* 1993, vol. 36, 290-300
- **ALTSCHUL.** *J. Mol. Biol.,* 1990, vol. 215, 403-410
- **HENIKOFF.** *PNAS,* 1989, vol. 89, 10915
- Wehner und Gehring. Thieme Verlag, 1995
- **SOS et al.** *J Clin Invest.,* June 2009, vol. 119 (6), 1727-40
- **BAERISWYL, V. ; CHRISTOFORI, G.** The angiogenic switch in carcinogenesis. *Semin Cancer Biol,* 2009, vol. 19, 329-337
- **CARMELIET, P.** Mechanisms of angiogenesis and arteriogenesis. *Nature medicine,* 2000, vol. 6, 389-395

- **LEE, T.H. et al.** Vascular endothelial growth factor mediates intracrine survival in human breast carcinoma cells through internally expressed VEGFR1/FLT1. *PLoS medicine,* 2007, vol. 4, 186
- **CHUNG, G.G. et al.** Vascular endothelial growth factor, FLT-1, and FLK-1 analysis in a pancreatic cancer tissue microarray. *Cancer,* 2006, vol. 106, 1677-1684
- **SILVA, S.R. et al.** VEGFR-2 expression in carcinoid cancer cells and its role in tumor growth and metastasis. *Int J Cancer,* 2010
- **LICHTENBERGER, B.M. et al.** Autocrine VEGF signaling synergizes with EGFR in tumor cells to promote epithelial cancer development. *Cell,* 2010, vol. 140, 268-279
- **PAO, W. et al.** Acquired resistance of lung adenocarcinomas to gefitinib or erlotinib is associated with a second mutation in the EGFR kinase domain. *PLoS Med,* 2005, vol. 2, 73
- **WEDGE, S.R. et al.** ZD6474 inhibits vascular endothelial growth factor signaling, angiogenesis, and tumor growth following oral administration. *Cancer Res,* 2002, vol. 62, 4645-4655
- **ULLRICH, R.T. et al.** Methyl-L-11C-methionine PET as a diagnostic marker for malignant progression in patients with glioma. *J Nucl Med,* 2009, vol. 50, 1962-1968
- **SHIELDS, A.F. et al.** Imaging proliferation in vivo with [F-18]FLT and positron emission tomography. *Nature medicine,* 1998, vol. 4, 1334-1336
- **KANAI, Y. et al.** Expression cloning and characterization of a transporter for large neutral amino acids activated by the heavy chain of 4F2 antigen (CD98. *The Journal of biological chemistry,* 1998, vol. 273, 23629-23632
- **VASUDEVAN, K.M. et al.** AKT-independent signaling downstream of oncogenic PIK3CA mutations in human cancer. *Cancer Cell,* 2009, vol. 16, 21-32
- **BLENCKE, S. et al.** Characterization of a conserved structural determinant controlling protein kinase sensitivity to selective inhibitors. *Chem Biol,* 2004, vol. 11, 691-701
- **BREKKEN, R.A. ; HUANG, X. ; KING, S.W. ; THORPE, P.E.** Vascular endothelial growth factor as a marker of tumor endothelium. *Cancer Res,* 1998, vol. 58, 1952-1959
- **LAKING, G.R. ; PRICE, P.M.** Positron emission tomographic imaging of angiogenesis and vascular function. *Br J Radiol,* 2003, vol. 76, 50-59
- **ROBERTS, D.M. et al.** The vascular endothelial growth factor (VEGF) receptor Flt-1 (VEGFR-1) modulates Flk-1 (VEGFR-2) signaling during blood vessel formation. *Am J Pathol,* 2004, vol. 164, 1531-1535
- **HAMACHER, K. ; COENEN, H.H. ; STOCKLIN, G.** Efficient stereospecific synthesis of no-carrier-added 2-[18F]-fluoro-2-deoxy-D-glucose using aminopolyether supported nucleophilic substitution. *J Nucl Med,* 1986, vol. 27, 235-238
- **MACHULLA, H. et al.** Simplified labeling approach for synthesizing 3-deoxy-3-[F-18]fluorothymidine ([F-18]FLT. *J Radiochem Nucl Chem,* 2000, vol. 243, 843-846

SEQUENCE LISTING

<110> Max-Planck-Gesellschaft zur FÃ¶rderung der Wissenschaften e.V.

<120> Combination therapy comprising selective VEGFR-2 inhibitors and MEK inhibitors

<130> T2094 EP S3

<160> 12

<170> BiSSAP 1.0

<210> 1
<211> 4071
<212> DNA
<213> Homo sapiens

<220>
<221> source
<222> 1..4071
<223> /mol_type="DNA"
/organism="Homo sapiens"

<400> 1
```
atgcagagca aggtgctgct ggccgtcgcc ctgtggctct gcgtggagac ccgggccgcc      60

tctgtgggtt tgcctagtgt ttctcttgat ctgcccaggc tcagcataca aaaagacata     120

cttacaatta aggctaatac aactcttcaa attacttgca ggggacagag ggacttggac     180

tggctttggc ccaataatca gagtggcagt gagcaaaggg tggaggtgac tgagtgcagc     240

gatggcctct tctgtaagac actcacaatt ccaaaagtga tcggaaatga cactggagcc     300

tacaagtgct tctaccggga aactgacttg gcctcggtca tttatgtcta tgttcaagat     360

tacagatctc catttattgc ttctgttagt gaccaacatg gagtcgtgta cattactgag     420

aacaaaaaca aaactgtggt gattccatgt ctcgggtcca tttcaaatct caacgtgtca     480

ctttgtgcaa gatacccaga aaagagattt gttcctgatg gtaacagaat ttcctgggac     540

agcaagaagg gctttactat tcccagctac atgatcagct atgctggcat ggtcttctgt     600

gaagcaaaaa ttaatgatga aagttaccag tctattatgt acatagttgt cgttgtaggg     660

tataggattt atgatgtggt tctgagtccg tctcatggaa ttgaactatc tgttggagaa     720

aagcttgtct taaattgtac agcaagaact gaactaaatg tggggattga cttcaactgg     780

gaataccctt cttcgaagca tcagcataag aaacttgtaa accgagacct aaaaacccag     840

tctgggagtg agatgaagaa atttttgagc accttaacta tagatggtgt aacccggagt     900

gaccaaggat tgtacacctg tgcagcatcc agtgggctga tgaccaagaa gaacagcaca     960

tttgtcaggg tccatgaaaa accttttgtt gcttttggaa gtggcatgga atctctggtg    1020

gaagccacgg tgggggagcg tgtcagaatc cctgcgaagt accttggtta cccaccccca    1080

gaaataaaat ggtataaaaa tggaataccc cttgagtcca atcacacaat taaagcgggg    1140

catgtactga cgattatgga agtgagtgaa agagacacag gaaattacac tgtcatcctt    1200
```

accaatccca tttcaaagga gaagcagagc catgtggtct ctctggttgt gtatgtccca 1260
ccccagattg gtgagaaatc tctaatctct cctgtggatt cctaccagta cggcaccact 1320
caaacgctga catgtacggt ctatgccatt cctcccccgc atcacatcca ctggtattgg 1380
cagttggagg aagagtgcgc caacgagccc agccaagctg tctcagtgac aaacccatac 1440
ccttgtgaag aatggagaag tgtggaggac ttccagggag gaaataaaat tgaagttaat 1500
aaaaatcaat ttgctctaat tgaaggaaaa aacaaaactg taagtaccct tgttatccaa 1560
gcggcaaatg tgtcagcttt gtacaaatgt gaagcggtca acaaagtcgg gagaggagag 1620
agggtgatct ccttccacgt gaccaggggt cctgaaatta ctttgcaacc tgacatgcag 1680
cccactgagc aggagagcgt gtctttgtgg tgcactgcag acagatctac gtttgagaac 1740
ctcacatggt acaagcttgg cccacagcct ctgccaatcc atgtgggaga gttgcccaca 1800
cctgtttgca agaacttgga tactctttgg aaattgaatg ccaccatgtt ctctaatagc 1860
acaaatgaca ttttgatcat ggagcttaag aatgcatcct tgcaggacca aggagactat 1920
gtctgccttg ctcaagacag gaagaccaag aaaagacatt gcgtggtcag gcagctcaca 1980
gtcctagagc gtgtggcacc cacgatcaca ggaaacctgg agaatcagac gacaagtatt 2040
ggggaaagca tcgaagtctc atgcacggca tctgggaatc cccctccaca gatcatgtgg 2100
tttaaagata atgagaccct tgtagaagac tcaggcattg tattgaagga tgggaaccgg 2160
aacctcacta tccgcagagt gaggaaggag gacgaaggcc tctacacctg ccaggcatgc 2220
agtgttcttg gctgtgcaaa agtggaggca tttttcataa tagaaggtgc ccaggaaaag 2280
acgaacttgg aaatcattat tctagtaggc acggcggtga ttgccatgtt cttctggcta 2340
cttcttgtca tcatcctacg gaccgttaag cgggccaatg gaggggaact gaagacaggc 2400
tacttgtcca tcgtcatgga tccagatgaa ctcccattgg atgaacattg tgaacgactg 2460
ccttatgatg ccagcaaatg ggaattcccc agagaccggc tgaagctagg taagcctctt 2520
ggccgtggtg cctttggcca agtgattgaa gcagatgcct ttggaattga caagacagca 2580
acttgcagga cagtagcagt caaaatgttg aaagaaggag caacacacag tgagcatcga 2640
gctctcatgt ctgaactcaa gatcctcatt catattggtc accatctcaa tgtggtcaac 2700
cttctaggtg cctgtaccaa gccaggaggg ccactcatgg tgattgtgga attctgcaaa 2760
tttggaaacc tgtccactta cctgaggagc aagagaaatg aatttgtccc ctacaagacc 2820
aaaggggcac gattccgtca agggaaagac tacgttggag caatccctgt ggatctgaaa 2880
cggcgcttgg acagcatcac cagtagccag agctcagcca gctctggatt tgtggaggag 2940
aagtccctca gtgatgtaga agaagaggaa gctcctgaag atctgtataa ggacttcctg 3000
accttggagc atctcatctg ttacagcttc caagtggcta agggcatgga gttcttggca 3060
tcgcgaaagt gtatccacag ggacctggcg gcacgaaata tcctcttatc ggagaagaac 3120
gtggttaaaa tctgtgactt tggcttggcc cgggatattt ataaagatcc agattatgtc 3180

```
agaaaaggag atgctcgcct ccctttgaaa tggatggccc cagaaacaat ttttgacaga     3240

gtgtacacaa tccagagtga cgtctggtct tttggtgttt tgctgtggga aatattttcc     3300

ttaggtgctt ctccatatcc tggggtaaag attgatgaag aattttgtag gcgattgaaa     3360

gaaggaacta gaatgagggc ccctgattat actacaccag aaatgtacca gaccatgctg     3420

gactgctggc acggggagcc cagtcagaga cccacgtttt cagagttggt ggaacatttg     3480

ggaaatctct tgcaagctaa tgctcagcag gatggcaaag actacattgt tcttccgata     3540

tcagagactt tgagcatgga agaggattct ggactctctc tgcctacctc acctgtttcc     3600

tgtatggagg aggaggaagt atgtgacccc aaattccatt atgacaacac agcaggaatc     3660

agtcagtatc tgcagaacag taagcgaaag agccggcctg tgagtgtaaa aacatttgaa     3720

gatatcccgt tagaagaacc agaagtaaaa gtaatcccag atgacaacca gacggacagt     3780

ggtatggttc ttgcctcaga agagctgaaa actttggaag acagaaccaa attatctcca     3840

tcttttggtg gaatggtgcc cagcaaaagc agggagtctg tggcatctga aggctcaaac     3900

cagacaagcg gctaccagtc cggatatcac tccgatgaca cagacaccac cgtgtactcc     3960

agtgaggaag cagaactttt aaagctgata gagattggag tgcaaaccgg tagcacagcc     4020

cagattctcc agcctgactc ggggaccaca ctgagctctc ctcctgttta a              4071
```

```
<210> 2
<211> 1356
<212> PRT
<213> Homo sapiens

<220>
<221> SOURCE
<222> 1..1356
<223> /mol_type="protein"
      /organism="Homo sapiens"

<400> 2
Met Gln Ser Lys Val Leu Leu Ala Val Ala Leu Trp Leu Cys Val Glu
1               5                   10                  15
Thr Arg Ala Ala Ser Val Gly Leu Pro Ser Val Ser Leu Asp Leu Pro
            20                  25                  30
Arg Leu Ser Ile Gln Lys Asp Ile Leu Thr Ile Lys Ala Asn Thr Thr
        35                  40                  45
Leu Gln Ile Thr Cys Arg Gly Gln Arg Asp Leu Asp Trp Leu Trp Pro
    50                  55                  60
Asn Asn Gln Ser Gly Ser Glu Gln Arg Val Glu Val Thr Glu Cys Ser
65                  70                  75                  80
Asp Gly Leu Phe Cys Lys Thr Leu Thr Ile Pro Lys Val Ile Gly Asn
                85                  90                  95
Asp Thr Gly Ala Tyr Lys Cys Phe Tyr Arg Glu Thr Asp Leu Ala Ser
            100                 105                 110
Val Ile Tyr Val Tyr Val Gln Asp Tyr Arg Ser Pro Phe Ile Ala Ser
        115                 120                 125
Val Ser Asp Gln His Gly Val Val Tyr Ile Thr Glu Asn Lys Asn Lys
    130                 135                 140
Thr Val Val Ile Pro Cys Leu Gly Ser Ile Ser Asn Leu Asn Val Ser
145                 150                 155                 160
Leu Cys Ala Arg Tyr Pro Glu Lys Arg Phe Val Pro Asp Gly Asn Arg
```

```
                165                 170                 175
Ile Ser Trp Asp Ser Lys Lys Gly Phe Thr Ile Pro Ser Tyr Met Ile
            180                 185                 190
Ser Tyr Ala Gly Met Val Phe Cys Glu Ala Lys Ile Asn Asp Glu Ser
        195                 200                 205
Tyr Gln Ser Ile Met Tyr Ile Val Val Val Val Gly Tyr Arg Ile Tyr
    210                 215                 220
Asp Val Val Leu Ser Pro Ser His Gly Ile Glu Leu Ser Val Gly Glu
225                 230                 235                 240
Lys Leu Val Leu Asn Cys Thr Ala Arg Thr Glu Leu Asn Val Gly Ile
                245                 250                 255
Asp Phe Asn Trp Glu Tyr Pro Ser Ser Lys His Gln His Lys Lys Leu
            260                 265                 270
Val Asn Arg Asp Leu Lys Thr Gln Ser Gly Ser Glu Met Lys Lys Phe
        275                 280                 285
Leu Ser Thr Leu Thr Ile Asp Gly Val Thr Arg Ser Asp Gln Gly Leu
    290                 295                 300
Tyr Thr Cys Ala Ala Ser Ser Gly Leu Met Thr Lys Lys Asn Ser Thr
305                 310                 315                 320
Phe Val Arg Val His Glu Lys Pro Phe Val Ala Phe Gly Ser Gly Met
                325                 330                 335
Glu Ser Leu Val Glu Ala Thr Val Gly Glu Arg Val Arg Ile Pro Ala
            340                 345                 350
Lys Tyr Leu Gly Tyr Pro Pro Pro Glu Ile Lys Trp Tyr Lys Asn Gly
        355                 360                 365
Ile Pro Leu Glu Ser Asn His Thr Ile Lys Ala Gly His Val Leu Thr
    370                 375                 380
Ile Met Glu Val Ser Glu Arg Asp Thr Gly Asn Tyr Thr Val Ile Leu
385                 390                 395                 400
Thr Asn Pro Ile Ser Lys Glu Lys Gln Ser His Val Val Ser Leu Val
                405                 410                 415
Val Tyr Val Pro Pro Gln Ile Gly Glu Lys Ser Leu Ile Ser Pro Val
            420                 425                 430
Asp Ser Tyr Gln Tyr Gly Thr Thr Gln Thr Leu Thr Cys Thr Val Tyr
        435                 440                 445
Ala Ile Pro Pro Pro His His Ile His Trp Tyr Trp Gln Leu Glu Glu
    450                 455                 460
Glu Cys Ala Asn Glu Pro Ser Gln Ala Val Ser Val Thr Asn Pro Tyr
465                 470                 475                 480
Pro Cys Glu Glu Trp Arg Ser Val Glu Asp Phe Gln Gly Gly Asn Lys
                485                 490                 495
Ile Glu Val Asn Lys Asn Gln Phe Ala Leu Ile Glu Gly Lys Asn Lys
            500                 505                 510
Thr Val Ser Thr Leu Val Ile Gln Ala Ala Asn Val Ser Ala Leu Tyr
        515                 520                 525
Lys Cys Glu Ala Val Asn Lys Val Gly Arg Gly Glu Arg Val Ile Ser
    530                 535                 540
Phe His Val Thr Arg Gly Pro Glu Ile Thr Leu Gln Pro Asp Met Gln
545                 550                 555                 560
Pro Thr Glu Gln Glu Ser Val Ser Leu Trp Cys Thr Ala Asp Arg Ser
                565                 570                 575
Thr Phe Glu Asn Leu Thr Trp Tyr Lys Leu Gly Pro Gln Pro Leu Pro
            580                 585                 590
Ile His Val Gly Glu Leu Pro Thr Pro Val Cys Lys Asn Leu Asp Thr
        595                 600                 605
Leu Trp Lys Leu Asn Ala Thr Met Phe Ser Asn Ser Thr Asn Asp Ile
    610                 615                 620
Leu Ile Met Glu Leu Lys Asn Ala Ser Leu Gln Asp Gln Gly Asp Tyr
625                 630                 635                 640
Val Cys Leu Ala Gln Asp Arg Lys Thr Lys Lys Arg His Cys Val Val
                645                 650                 655
Arg Gln Leu Thr Val Leu Glu Arg Val Ala Pro Thr Ile Thr Gly Asn
            660                 665                 670
Leu Glu Asn Gln Thr Thr Ser Ile Gly Glu Ser Ile Glu Val Ser Cys
        675                 680                 685
```

Thr Ala Ser Gly Asn Pro Pro Pro Gln Ile Met Trp Phe Lys Asp Asn
    690                 695                 700
Glu Thr Leu Val Glu Asp Ser Gly Ile Val Leu Lys Asp Gly Asn Arg
705                 710                 715                 720
Asn Leu Thr Ile Arg Arg Val Arg Lys Glu Asp Glu Gly Leu Tyr Thr
                725                 730                 735
Cys Gln Ala Cys Ser Val Leu Gly Cys Ala Lys Val Glu Ala Phe Phe
            740                 745                 750
Ile Ile Glu Gly Ala Gln Glu Lys Thr Asn Leu Glu Ile Ile Ile Leu
        755                 760                 765
Val Gly Thr Ala Val Ile Ala Met Phe Phe Trp Leu Leu Leu Val Ile
    770                 775                 780
Ile Leu Arg Thr Val Lys Arg Ala Asn Gly Gly Glu Leu Lys Thr Gly
785                 790                 795                 800
Tyr Leu Ser Ile Val Met Asp Pro Asp Glu Leu Pro Leu Asp Glu His
                805                 810                 815
Cys Glu Arg Leu Pro Tyr Asp Ala Ser Lys Trp Glu Phe Pro Arg Asp
            820                 825                 830
Arg Leu Lys Leu Gly Lys Pro Leu Gly Arg Gly Ala Phe Gly Gln Val
        835                 840                 845
Ile Glu Ala Asp Ala Phe Gly Ile Asp Lys Thr Ala Thr Cys Arg Thr
    850                 855                 860
Val Ala Val Lys Met Leu Lys Glu Gly Ala Thr His Ser Glu His Arg
865                 870                 875                 880
Ala Leu Met Ser Glu Leu Lys Ile Leu Ile His Ile Gly His His Leu
                885                 890                 895
Asn Val Val Asn Leu Leu Gly Ala Cys Thr Lys Pro Gly Gly Pro Leu
            900                 905                 910
Met Val Ile Val Glu Phe Cys Lys Phe Gly Asn Leu Ser Thr Tyr Leu
        915                 920                 925
Arg Ser Lys Arg Asn Glu Phe Val Pro Tyr Lys Thr Lys Gly Ala Arg
    930                 935                 940
Phe Arg Gln Gly Lys Asp Tyr Val Gly Ala Ile Pro Val Asp Leu Lys
945                 950                 955                 960
Arg Arg Leu Asp Ser Ile Thr Ser Ser Gln Ser Ser Ala Ser Ser Gly
                965                 970                 975
Phe Val Glu Glu Lys Ser Leu Ser Asp Val Glu Glu Glu Glu Ala Pro
            980                 985                 990
Glu Asp Leu Tyr Lys Asp Phe Leu Thr Leu Glu His Leu Ile Cys Tyr
        995                 1000                1005
Ser Phe Gln Val Ala Lys Gly Met Glu Phe Leu Ala Ser Arg Lys Cys
    1010                1015                1020
Ile His Arg Asp Leu Ala Ala Arg Asn Ile Leu Leu Ser Glu Lys Asn
1025                1030                1035                1040
Val Val Lys Ile Cys Asp Phe Gly Leu Ala Arg Asp Ile Tyr Lys Asp
                1045                1050                1055
Pro Asp Tyr Val Arg Lys Gly Asp Ala Arg Leu Pro Leu Lys Trp Met
            1060                1065                1070
Ala Pro Glu Thr Ile Phe Asp Arg Val Tyr Thr Ile Gln Ser Asp Val
        1075                1080                1085
Trp Ser Phe Gly Val Leu Leu Trp Glu Ile Phe Ser Leu Gly Ala Ser
    1090                1095                1100
Pro Tyr Pro Gly Val Lys Ile Asp Glu Glu Phe Cys Arg Arg Leu Lys
1105                1110                1115                1120
Glu Gly Thr Arg Met Arg Ala Pro Asp Tyr Thr Thr Pro Glu Met Tyr
                1125                1130                1135
Gln Thr Met Leu Asp Cys Trp His Gly Glu Pro Ser Gln Arg Pro Thr
            1140                1145                1150
Phe Ser Glu Leu Val Glu His Leu Gly Asn Leu Leu Gln Ala Asn Ala
        1155                1160                1165
Gln Gln Asp Gly Lys Asp Tyr Ile Val Leu Pro Ile Ser Glu Thr Leu
    1170                1175                1180
Ser Met Glu Glu Asp Ser Gly Leu Ser Leu Pro Thr Ser Pro Val Ser
1185                1190                1195                1200
Cys Met Glu Glu Glu Glu Val Cys Asp Pro Lys Phe His Tyr Asp Asn

```
                1205                1210                1215
Thr Ala Gly Ile Ser Gln Tyr Leu Gln Asn Ser Lys Arg Lys Ser Arg
            1220                1225                1230
Pro Val Ser Val Lys Thr Phe Glu Asp Ile Pro Leu Glu Glu Pro Glu
        1235                1240                1245
Val Lys Val Ile Pro Asp Asp Asn Gln Thr Asp Ser Gly Met Val Leu
    1250                1255                1260
Ala Ser Glu Glu Leu Lys Thr Leu Glu Asp Arg Thr Lys Leu Ser Pro
1265                1270                1275                1280
Ser Phe Gly Gly Met Val Pro Ser Lys Ser Arg Glu Ser Val Ala Ser
                1285                1290                1295
Glu Gly Ser Asn Gln Thr Ser Gly Tyr Gln Ser Gly Tyr His Ser Asp
            1300                1305                1310
Asp Thr Asp Thr Thr Val Tyr Ser Ser Glu Glu Ala Glu Leu Leu Lys
        1315                1320                1325
Leu Ile Glu Ile Gly Val Gln Thr Gly Ser Thr Ala Gln Ile Leu Gln
    1330                1335                1340
Pro Asp Ser Gly Thr Thr Leu Ser Ser Pro Pro Val
1345                1350                1355

<210> 3
<211> 1083
<212> DNA
<213> Homo sapiens

<220>
<221> source
<222> 1..1083
<223> /mol_type="DNA"
      /organism="Homo sapiens"

<400> 3
atggcggcgg cggcggcggc gggcgcgggc ccggagatgg tccgcgggca ggtgttcgac      60

gtggggccgc gctacaccaa cctctcgtac atcggcgagg gcgcctacgg catggtgtgc     120

tctgcttatg ataatgtcaa caaagttcga gtagctatca agaaaatcag cccctttgag     180

caccagacct actgccagag aaccctgagg gagataaaaa tcttactgcg cttcagacat     240

gagaacatca ttggaatcaa tgacattatt cgagcaccaa ccatcgagca aatgaaagat     300

gtatatatag tacaggacct catggaaaca gatctttaca agctcttgaa gacacaacac     360

ctcagcaatg accatatctg ctattttctc taccagatcc tcagagggtt aaaatatatc     420

cattcagcta acgttctgca ccgtgacctc aagccttcca acctgctgct caacaccacc     480

tgtgatctca agatctgtga ctttggcctg gcccgtgttg cagatccaga ccatgatcac     540

acagggttcc tgacagaata tgtggccaca cgttggtaca gggctccaga aattatgttg     600

aattccaagg gctacaccaa gtccattgat atttggtctg taggctgcat tctggcagaa     660

atgctttcta acaggcccat ctttccaggg aagcattatc ttgaccagct gaaccacatt     720

ttgggtattc ttggatcccc atcacaagaa gacctgaatt gtataataaa tttaaaagct     780

aggaactatt tgctttctct tccacacaaa aataaggtgc catggaacag gctgttccca     840

aatgctgact ccaaagctct ggacttattg gacaaaatgt tgacattcaa cccacacaag     900

aggattgaag tagaacaggc tctggcccac ccatatctgg agcagtatta cgacccgagt     960

gacgagccca tcgccgaagc accattcaag ttcgacatgg aattggatga cttgcctaag    1020
```

gaaaagctca aagaactaat ttttgaagag actgctagat tccagccagg atacagatct 1080

taa 1083

<210> 4
<211> 360
<212> PRT
<213> Homo sapiens

<220>
<221> SOURCE
<222> 1..360
<223> /mol_type="protein"
/organism="Homo sapiens"

<400> 4
```
Met Ala Ala Ala Ala Ala Ala Gly Ala Gly Pro Glu Met Val Arg Gly
1               5                   10                  15
Gln Val Phe Asp Val Gly Pro Arg Tyr Thr Asn Leu Ser Tyr Ile Gly
            20                  25                  30
Glu Gly Ala Tyr Gly Met Val Cys Ser Ala Tyr Asp Asn Val Asn Lys
        35                  40                  45
Val Arg Val Ala Ile Lys Lys Ile Ser Pro Phe Glu His Gln Thr Tyr
    50                  55                  60
Cys Gln Arg Thr Leu Arg Glu Ile Lys Ile Leu Leu Arg Phe Arg His
65                  70                  75                  80
Glu Asn Ile Ile Gly Ile Asn Asp Ile Ile Arg Ala Pro Thr Ile Glu
                85                  90                  95
Gln Met Lys Asp Val Tyr Ile Val Gln Asp Leu Met Glu Thr Asp Leu
            100                 105                 110
Tyr Lys Leu Leu Lys Thr Gln His Leu Ser Asn Asp His Ile Cys Tyr
        115                 120                 125
Phe Leu Tyr Gln Ile Leu Arg Gly Leu Lys Tyr Ile His Ser Ala Asn
    130                 135                 140
Val Leu His Arg Asp Leu Lys Pro Ser Asn Leu Leu Leu Asn Thr Thr
145                 150                 155                 160
Cys Asp Leu Lys Ile Cys Asp Phe Gly Leu Ala Arg Val Ala Asp Pro
                165                 170                 175
Asp His Asp His Thr Gly Phe Leu Thr Glu Tyr Val Ala Thr Arg Trp
            180                 185                 190
Tyr Arg Ala Pro Glu Ile Met Leu Asn Ser Lys Gly Tyr Thr Lys Ser
        195                 200                 205
Ile Asp Ile Trp Ser Val Gly Cys Ile Leu Ala Glu Met Leu Ser Asn
    210                 215                 220
Arg Pro Ile Phe Pro Gly Lys His Tyr Leu Asp Gln Leu Asn His Ile
225                 230                 235                 240
Leu Gly Ile Leu Gly Ser Pro Ser Gln Glu Asp Leu Asn Cys Ile Ile
                245                 250                 255
Asn Leu Lys Ala Arg Asn Tyr Leu Leu Ser Leu Pro His Lys Asn Lys
            260                 265                 270
Val Pro Trp Asn Arg Leu Phe Pro Asn Ala Asp Ser Lys Ala Leu Asp
        275                 280                 285
Leu Leu Asp Lys Met Leu Thr Phe Asn Pro His Lys Arg Ile Glu Val
    290                 295                 300
Glu Gln Ala Leu Ala His Pro Tyr Leu Glu Gln Tyr Tyr Asp Pro Ser
305                 310                 315                 320
Asp Glu Pro Ile Ala Glu Ala Pro Phe Lys Phe Asp Met Glu Leu Asp
                325                 330                 335
Asp Leu Pro Lys Glu Lys Leu Lys Glu Leu Ile Phe Glu Glu Thr Ala
            340                 345                 350
Arg Phe Gln Pro Gly Tyr Arg Ser
        355                 360
```

<210> 5
<211> 1203
<212> DNA
<213> Homo sapiens

<220>
<221> source
<222> 1..1203
<223> /mol_type="DNA"
/organism="Homo sapiens"

<400> 5
atgctggccc ggaggaagcc ggtgctgccg gcgctcacca tcaaccctac catcgccgag 60

ggcccatccc ctaccagcga gggcgcctcc gaggcaaacc tggtggacct gcagaagaag 120

ctggaggagc tggaacttga cgagcagcag aagaagcggc tggaagcctt tctcacccag 180

aaagccaagg tcggcgaact caaagacgat gacttcgaaa ggatctcaga gctgggcgcg 240

ggcaacggcg gggtggtcac caaagtccag cacagaccct cgggcctcat catggccagg 300

aagctgatcc accttgagat caagccggcc atccggaacc agatcatccg cgagctgcag 360

gtcctgcacg aatgcaactc gccgtacatc gtgggcttct acggggcctt ctacagtgac 420

ggggagatca gcatttgcat ggaacacatg gacggcggct ccctggacca ggtgctgaaa 480

gaggccaaga ggattcccga ggagatcctg gggaaagtca gcatcgcggt tctccggggc 540

ttggcgtacc tccgagagaa gcaccagatc atgcaccgag atgtgaagcc ctccaacatc 600

ctcgtgaact ctagagggga gatcaagctg tgtgacttcg ggtgagcgg ccagctcatc 660

gactccatgg ccaactcctt cgtgggcacg cgctcctaca tggctccgga gcggttgcag 720

ggcacacatt actcggtgca gtcggacatc tggagcatgg gcctgtccct ggtggagctg 780

gccgtcggaa ggtaccccat cccccgccc gacgccaaag agctggaggc catctttggc 840

cggcccgtgg tcgacgggga agaaggagag cctcacagca tctcgcctcg gccgaggccc 900

cccgggcgcc ccgtcagcgg tcacgggatg gatagccggc ctgccatggc catctttgaa 960

ctcctggact atattgtgaa cgagccacct cctaagctgc ccaacggtgt gttcaccccc 1020

gacttccagg agtttgtcaa taaatgcctc atcaagaacc cagcggagcg ggcggacctg 1080

aagatgctca caaaccacac cttcatcaag cggtccgagg tggaagaagt ggattttgcc 1140

ggctggttgt gtaaaaccct gcggctgaac cagcccggca cacccacgcg caccgccgtg 1200

tga 1203

<210> 6
<211> 400
<212> PRT
<213> Homo sapiens

<220>
<221> SOURCE
<222> 1..400
<223> /mol_type="protein"
/organism="Homo sapiens"

<400> 6
Met Leu Ala Arg Arg Lys Pro Val Leu Pro Ala Leu Thr Ile Asn Pro
1 5 10 15
Thr Ile Ala Glu Gly Pro Ser Pro Thr Ser Glu Gly Ala Ser Glu Ala
20 25 30
Asn Leu Val Asp Leu Gln Lys Lys Leu Glu Glu Leu Glu Leu Asp Glu
35 40 45
Gln Gln Lys Lys Arg Leu Glu Ala Phe Leu Thr Gln Lys Ala Lys Val
50 55 60
Gly Glu Leu Lys Asp Asp Asp Phe Glu Arg Ile Ser Glu Leu Gly Ala
65 70 75 80
Gly Asn Gly Gly Val Val Thr Lys Val Gln His Arg Pro Ser Gly Leu
85 90 95
Ile Met Ala Arg Lys Leu Ile His Leu Glu Ile Lys Pro Ala Ile Arg
100 105 110
Asn Gln Ile Ile Arg Glu Leu Gln Val Leu His Glu Cys Asn Ser Pro
115 120 125
Tyr Ile Val Gly Phe Tyr Gly Ala Phe Tyr Ser Asp Gly Glu Ile Ser
130 135 140
Ile Cys Met Glu His Met Asp Gly Gly Ser Leu Asp Gln Val Leu Lys
145 150 155 160
Glu Ala Lys Arg Ile Pro Glu Glu Ile Leu Gly Lys Val Ser Ile Ala
165 170 175
Val Leu Arg Gly Leu Ala Tyr Leu Arg Glu Lys His Gln Ile Met His
180 185 190
Arg Asp Val Lys Pro Ser Asn Ile Leu Val Asn Ser Arg Gly Glu Ile
195 200 205
Lys Leu Cys Asp Phe Gly Val Ser Gly Gln Leu Ile Asp Ser Met Ala
210 215 220
Asn Ser Phe Val Gly Thr Arg Ser Tyr Met Ala Pro Glu Arg Leu Gln
225 230 235 240
Gly Thr His Tyr Ser Val Gln Ser Asp Ile Trp Ser Met Gly Leu Ser
245 250 255
Leu Val Glu Leu Ala Val Gly Arg Tyr Pro Ile Pro Pro Pro Asp Ala
260 265 270
Lys Glu Leu Glu Ala Ile Phe Gly Arg Pro Val Val Asp Gly Glu Glu
275 280 285
Gly Glu Pro His Ser Ile Ser Pro Arg Pro Arg Pro Pro Gly Arg Pro
290 295 300
Val Ser Gly His Gly Met Asp Ser Arg Pro Ala Met Ala Ile Phe Glu
305 310 315 320
Leu Leu Asp Tyr Ile Val Asn Glu Pro Pro Pro Lys Leu Pro Asn Gly
325 330 335
Val Phe Thr Pro Asp Phe Gln Glu Phe Val Asn Lys Cys Leu Ile Lys
340 345 350
Asn Pro Ala Glu Arg Ala Asp Leu Lys Met Leu Thr Asn His Thr Phe
355 360 365
Ile Lys Arg Ser Glu Val Glu Glu Val Asp Phe Ala Gly Trp Leu Cys
370 375 380
Lys Thr Leu Arg Leu Asn Gln Pro Gly Thr Pro Thr Arg Thr Ala Val
385 390 395 400

<210> 7
<211> 1188
<212> DNA
<213> Homo sapiens

<220>
<221> source
<222> 1..1188
<223> /mol_type="DNA"
/organism="Homo sapiens"

<400> 7

```
ctgacggaca gacagacaga caccgccccc agccccagct accacctcct ccccggccgg      60
cggcggacag tggacgcggc ggcgagccgc gggcaggggc cggagcccgc gcccggaggc     120
ggggtggagg gggtcggggc tcgcggcgtc gcactgaaac ttttcgtcca acttctgggc     180
tgttctcgct tcggaggagc cgtggtccgc gcgggggaag ccgagccgag cggagccgcg     240
agaagtgcta gctcgggccg ggaggagccg cagccggagg aggggggagga ggaagaagag    300
aaggaagagg agagggggcc gcagtggcga ctcggcgctc ggaagccggg ctcatggacg     360
ggtgaggcgg cggtgtgcgc agacagtgct ccagccgcgc gcgctcccca ggccctggcc     420
cgggcctcgg gccggggagg aagagtagct cgccgaggcg ccgaggagag cgggccgccc     480
cacagcccga gccggagagg gagcgcgagc cgcgccggcc ccggtcgggc ctccgaaacc     540
atgaactttc tgctgtcttg ggtgcattgg agccttgcct tgctgctcta cctccaccat     600
gccaagtggt cccaggctgc acccatggca gaaggaggag ggcagaatca tcacgaagtg     660
gtgaagttca tggatgtcta tcagcgcagc tactgccatc caatcgagac cctggtggac     720
atcttccagg agtaccctga tgagatcgag tacatcttca agccatcctg tgtgcccctg     780
atgcgatgcg gggctgctg caatgacgag ggcctggagt gtgtgcccac tgaggagtcc      840
aacatcacca tgcagattat gcggatcaaa cctcaccaag gccagcacat aggagagatg     900
agcttcctac agcacaacaa atgtgaatgc agaccaaaga aagatagagc aagacaagaa     960
aaaaaatcag ttcgaggaaa gggaaagggg caaaaacgaa agcgcaagaa atcccggtat    1020
aagtcctgga gcgttccctg tgggccttgc tcagagcgga gaaagcattt gtttgtacaa    1080
gatccgcaga cgtgtaaatg ttcctgcaaa aacacagact cgcgttgcaa ggcgaggcag    1140
cttgagttaa acgaacgtac ttgcagatgt gacaagccga ggcggtga                 1188
```

<210> 8
<211> 395
<212> PRT
<213> Homo sapiens

<220>
<221> SOURCE
<222> 1..395
<223> /mol_type="protein"
/organism="Homo sapiens"

<400> 8

```
Met Thr Asp Arg Gln Thr Asp Thr Ala Pro Ser Pro Ser Tyr His Leu
1               5                   10                  15
Leu Pro Gly Arg Arg Arg Thr Val Asp Ala Ala Ala Ser Arg Gly Gln
            20                  25                  30
Gly Pro Glu Pro Ala Pro Gly Gly Gly Val Glu Gly Val Gly Ala Arg
        35                  40                  45
Gly Val Ala Leu Lys Leu Phe Val Gln Leu Leu Gly Cys Ser Arg Phe
    50                  55                  60
Gly Gly Ala Val Val Arg Ala Gly Glu Ala Glu Pro Ser Gly Ala Ala
65                  70                  75                  80
Arg Ser Ala Ser Ser Gly Arg Glu Glu Pro Gln Pro Glu Glu Gly Glu
                85                  90                  95
```

```
Glu Glu Glu Glu Lys Glu Glu Glu Arg Gly Pro Gln Trp Arg Leu Gly
            100                 105                 110
Ala Arg Lys Pro Gly Ser Trp Thr Gly Glu Ala Ala Val Cys Ala Asp
        115                 120                 125
Ser Ala Pro Ala Ala Arg Ala Pro Gln Ala Leu Ala Arg Ala Ser Gly
    130                 135                 140
Arg Gly Gly Arg Val Ala Arg Arg Gly Ala Glu Glu Ser Gly Pro Pro
145                 150                 155                 160
His Ser Pro Ser Arg Arg Gly Ser Ala Ser Arg Ala Gly Pro Gly Arg
                165                 170                 175
Ala Ser Glu Thr Met Asn Phe Leu Leu Ser Trp Val His Trp Ser Leu
            180                 185                 190
Ala Leu Leu Leu Tyr Leu His His Ala Lys Trp Ser Gln Ala Ala Pro
        195                 200                 205
Met Ala Glu Gly Gly Gly Gln Asn His His Glu Val Val Lys Phe Met
    210                 215                 220
Asp Val Tyr Gln Arg Ser Tyr Cys His Pro Ile Glu Thr Leu Val Asp
225                 230                 235                 240
Ile Phe Gln Glu Tyr Pro Asp Glu Ile Glu Tyr Ile Phe Lys Pro Ser
                245                 250                 255
Cys Val Pro Leu Met Arg Cys Gly Gly Cys Cys Asn Asp Glu Gly Leu
            260                 265                 270
Glu Cys Val Pro Thr Glu Glu Ser Asn Ile Thr Met Gln Ile Met Arg
        275                 280                 285
Ile Lys Pro His Gln Gly Gln His Ile Gly Glu Met Ser Phe Leu Gln
    290                 295                 300
His Asn Lys Cys Glu Cys Arg Pro Lys Lys Asp Arg Ala Arg Gln Glu
305                 310                 315                 320
Lys Lys Ser Val Arg Gly Lys Gly Lys Gly Gln Lys Arg Lys Arg Lys
                325                 330                 335
Lys Ser Arg Tyr Lys Ser Trp Ser Val Pro Cys Gly Pro Cys Ser Glu
            340                 345                 350
Arg Arg Lys His Leu Phe Val Gln Asp Pro Gln Thr Cys Lys Cys Ser
        355                 360                 365
Cys Lys Asn Thr Asp Ser Arg Cys Lys Ala Arg Gln Leu Glu Leu Asn
    370                 375                 380
Glu Arg Thr Cys Arg Cys Asp Lys Pro Arg Arg
385                 390                 395
```

<210> 9
<211> 3633
<212> DNA
<213> Homo sapiens

<220>
<221> source
<222> 1..3633
<223> /mol_type="DNA"
/organism="Homo sapiens"

<400> 9

```
atgcgaccct ccgggacggc cggggcagcg ctcctggcgc tgctggctgc gctctgcccg      60

gcgagtcggg ctctggagga aaagaaagtt tgccaaggca cgagtaacaa gctcacgcag     120

ttgggcactt ttgaagatca ttttctcagc ctccagagga tgttcaataa ctgtgaggtg     180

gtccttggga atttggaaat tacctatgtg cagaggaatt atgatctttc cttcttaaag     240

accatccagg aggtggctgg ttatgtcctc attgccctca acacagtgga gcgaattcct     300

ttggaaaacc tgcagatcat cagaggaaat atgtactacg aaaattccta tgccttagca     360

gtcttatcta actatgatgc aaataaaacc ggactgaagg agctgcccat gagaaattta     420
```

caggaaatcc tgcatggcgc cgtgcggttc agcaacaacc ctgccctgtg caacgtggag 480
agcatccagt ggcgggacat agtcagcagt gactttctca gcaacatgtc gatggacttc 540
cagaaccacc tgggcagctg ccaaaagtgt gatccaagct gtcccaatgg gagctgctgg 600
ggtgcaggag aggagaactg ccagaaactg accaaaatca tctgtgccca gcagtgctcc 660
gggcgctgcc gtggcaagtc ccccagtgac tgctgccaca accagtgtgc tgcaggctgc 720
acaggccccc gggagagcga ctgcctggtc tgccgcaaat tccgagacga agccacgtgc 780
aaggacacct gcccccccact catgctctac aaccccacca cgtaccagat ggatgtgaac 840
cccgagggca aatacagctt tggtgccacc tgcgtgaaga agtgtccccg taattatgtg 900
gtgacagatc acggctcgtg cgtccgagcc tgtggggccg acagctatga gatggaggaa 960
gacggcgtcc gcaagtgtaa gaagtgcgaa gggccttgcc gcaaagtgtg taacggaata 1020
ggtattggtg aatttaaaga ctcactctcc ataaatgcta cgaatattaa acacttcaaa 1080
aactgcacct ccatcagtgg cgatctccac atcctgccgg tggcatttag ggggtgactcc 1140
ttcacacata ctcctcctct ggatccacag gaactggata ttctgaaaac cgtaaaggaa 1200
atcacagggt ttttgctgat tcaggcttgg cctgaaaaca ggacggacct ccatgccttt 1260
gagaacctag aaatcatacg cggcaggacc aagcaacatg gtcagttttc tcttgcagtc 1320
gtcagcctga acataacatc cttgggatta cgctccctca aggagataag tgatggagat 1380
gtgataattt caggaaacaa aaatttgtgc tatgcaaata caataaactg gaaaaaactg 1440
tttgggacct ccggtcagaa aaccaaaatt ataagcaaca gaggtgaaaa cagctgcaag 1500
gccacaggcc aggtctgcca tgccttgtgc tcccccgagg gctgctgggg cccggagccc 1560
agggactgcg tctcttgccg gaatgtcagc cgaggcaggg aatgcgtgga caagtgcaac 1620
cttctggagg gtgagccaag ggagtttgtg gagaactctg agtgcataca gtgccaccca 1680
gagtgcctgc ctcaggccat gaacatcacc tgcacaggac ggggaccaga caactgtatc 1740
cagtgtgccc actacattga cggcccccac tgcgtcaaga cctgcccggc aggagtcatg 1800
ggagaaaaca acaccctggt ctggaagtac gcagacgccg gccatgtgtg ccacctgtgc 1860
catccaaact gcacctacgg atgcactggg ccaggtcttg aaggctgtcc aacgaatggg 1920
cctaagatcc cgtccatcgc cactgggatg gtgggggccc tcctcttgct gctggtggtg 1980
gccctgggga tcggcctctt catgcgaagg cgccacatcg ttcggaagcg cacgctgcgg 2040
aggctgctgc aggagaggga gcttgtggag cctcttacac ccagtggaga agctcccaac 2100
caagctctct tgaggatctt gaaggaaact gaattcaaaa agatcaaagt gctgggctcc 2160
ggtgcgttcg gcacggtgta taagggactc tggatcccag aaggtgagaa agttaaaatt 2220
cccgtcgcta tcaaggaatt aagagaagca acatctccga aagccaacaa ggaaatcctc 2280
gatgaagcct acgtgatggc cagcgtggac aacccccacg tgtgccgcct gctgggcatc 2340
tgcctcacct ccaccgtgca gctcatcacg cagctcatgc ccttcggctg cctcctggac 2400

```
tatgtccggg aacacaaaga caatattggc tcccagtacc tgctcaactg gtgtgtgcag     2460
atcgcaaagg gcatgaacta cttggaggac cgtcgcttgg tgcaccgcga cctggcagcc     2520
aggaacgtac tggtgaaaac accgcagcat gtcaagatca cagattttgg gctggccaaa     2580
ctgctgggtg cggaagagaa agaataccat gcagaaggag gcaaagtgcc tatcaagtgg     2640
atggcattgg aatcaatttt acacagaatc tatacccacc agagtgatgt ctggagctac     2700
ggggtgactg tttgggagtt gatgaccttt ggatccaagc catatgacgg aatccctgcc     2760
agcgagatct cctccatcct ggagaaagga gaacgcctcc ctcagccacc catatgtacc     2820
atcgatgtct acatgatcat ggtcaagtgc tggatgatag acgcagatag tcgcccaaag     2880
ttccgtgagt tgatcatcga attctccaaa atggcccgag acccccagcg ctaccttgtc     2940
attcaggggg atgaaagaat gcatttgcca agtcctacag actccaactt ctaccgtgcc     3000
ctgatggatg aagaagacat ggacgacgtg gtggatgccg acgagtacct catcccacag     3060
cagggcttct tcagcagccc ctccacgtca cggactcccc tcctgagctc tctgagtgca     3120
accagcaaca attccaccgt ggcttgcatt gatagaaatg ggctgcaaag ctgtcccatc     3180
aaggaagaca gcttcttgca gcgatacagc tcagacccca caggcgcctt gactgaggac     3240
agcatagacg acaccttcct cccagtgcct gaatacataa accagtccgt tcccaaaagg     3300
cccgctggct ctgtgcagaa tcctgtctat cacaatcagc ctctgaaccc cgcgcccagc     3360
agagacccac actaccagga cccccacagc actgcagtgg gcaaccccga gtatctcaac     3420
actgtccagc ccacctgtgt caacagcaca ttcgacagcc ctgcccactg ggcccagaaa     3480
ggcagccacc aaattagcct ggacaaccct gactaccagc aggacttctt tcccaaggaa     3540
gccaagccaa atggcatctt taagggctcc acagctgaaa atgcagaata cctaagggtc     3600
gcgccacaaa gcagtgaatt tattggagca tga                                  3633
```

```
<210> 10
<211> 1210
<212> PRT
<213> Homo sapiens

<220>
<221> SOURCE
<222> 1..1210
<223> /mol_type="protein"
      /organism="Homo sapiens"

<400> 10
Met Arg Pro Ser Gly Thr Ala Gly Ala Ala Leu Leu Ala Leu Leu Ala
1               5                   10                  15
Ala Leu Cys Pro Ala Ser Arg Ala Leu Glu Glu Lys Lys Val Cys Gln
            20                  25                  30
Gly Thr Ser Asn Lys Leu Thr Gln Leu Gly Thr Phe Glu Asp His Phe
        35                  40                  45
Leu Ser Leu Gln Arg Met Phe Asn Asn Cys Glu Val Val Leu Gly Asn
    50                  55                  60
Leu Glu Ile Thr Tyr Val Gln Arg Asn Tyr Asp Leu Ser Phe Leu Lys
```

```
65                  70                  75                  80
Thr Ile Gln Glu Val Ala Gly Tyr Val Leu Ile Ala Leu Asn Thr Val
                85                  90                  95
Glu Arg Ile Pro Leu Glu Asn Leu Gln Ile Ile Arg Gly Asn Met Tyr
            100                 105                 110
Tyr Glu Asn Ser Tyr Ala Leu Ala Val Leu Ser Asn Tyr Asp Ala Asn
        115                 120                 125
Lys Thr Gly Leu Lys Glu Leu Pro Met Arg Asn Leu Gln Glu Ile Leu
    130                 135                 140
His Gly Ala Val Arg Phe Ser Asn Asn Pro Ala Leu Cys Asn Val Glu
145                 150                 155                 160
Ser Ile Gln Trp Arg Asp Ile Val Ser Ser Asp Phe Leu Ser Asn Met
                165                 170                 175
Ser Met Asp Phe Gln Asn His Leu Gly Ser Cys Gln Lys Cys Asp Pro
            180                 185                 190
Ser Cys Pro Asn Gly Ser Cys Trp Gly Ala Gly Glu Glu Asn Cys Gln
        195                 200                 205
Lys Leu Thr Lys Ile Ile Cys Ala Gln Gln Cys Ser Gly Arg Cys Arg
    210                 215                 220
Gly Lys Ser Pro Ser Asp Cys Cys His Asn Gln Cys Ala Ala Gly Cys
225                 230                 235                 240
Thr Gly Pro Arg Glu Ser Asp Cys Leu Val Cys Arg Lys Phe Arg Asp
                245                 250                 255
Glu Ala Thr Cys Lys Asp Thr Cys Pro Pro Leu Met Leu Tyr Asn Pro
            260                 265                 270
Thr Thr Tyr Gln Met Asp Val Asn Pro Glu Gly Lys Tyr Ser Phe Gly
        275                 280                 285
Ala Thr Cys Val Lys Lys Cys Pro Arg Asn Tyr Val Val Thr Asp His
    290                 295                 300
Gly Ser Cys Val Arg Ala Cys Gly Ala Asp Ser Tyr Glu Met Glu Glu
305                 310                 315                 320
Asp Gly Val Arg Lys Cys Lys Lys Cys Glu Gly Pro Cys Arg Lys Val
                325                 330                 335
Cys Asn Gly Ile Gly Ile Gly Glu Phe Lys Asp Ser Leu Ser Ile Asn
            340                 345                 350
Ala Thr Asn Ile Lys His Phe Lys Asn Cys Thr Ser Ile Ser Gly Asp
        355                 360                 365
Leu His Ile Leu Pro Val Ala Phe Arg Gly Asp Ser Phe Thr His Thr
    370                 375                 380
Pro Pro Leu Asp Pro Gln Glu Leu Asp Ile Leu Lys Thr Val Lys Glu
385                 390                 395                 400
Ile Thr Gly Phe Leu Leu Ile Gln Ala Trp Pro Glu Asn Arg Thr Asp
                405                 410                 415
Leu His Ala Phe Glu Asn Leu Glu Ile Ile Arg Gly Arg Thr Lys Gln
            420                 425                 430
His Gly Gln Phe Ser Leu Ala Val Val Ser Leu Asn Ile Thr Ser Leu
        435                 440                 445
Gly Leu Arg Ser Leu Lys Glu Ile Ser Asp Gly Asp Val Ile Ile Ser
    450                 455                 460
Gly Asn Lys Asn Leu Cys Tyr Ala Asn Thr Ile Asn Trp Lys Lys Leu
465                 470                 475                 480
Phe Gly Thr Ser Gly Gln Lys Thr Lys Ile Ile Ser Asn Arg Gly Glu
                485                 490                 495
Asn Ser Cys Lys Ala Thr Gly Gln Val Cys His Ala Leu Cys Ser Pro
            500                 505                 510
Glu Gly Cys Trp Gly Pro Glu Pro Arg Asp Cys Val Ser Cys Arg Asn
        515                 520                 525
Val Ser Arg Gly Arg Glu Cys Val Asp Lys Cys Asn Leu Leu Glu Gly
    530                 535                 540
Glu Pro Arg Glu Phe Val Glu Asn Ser Glu Cys Ile Gln Cys His Pro
545                 550                 555                 560
Glu Cys Leu Pro Gln Ala Met Asn Ile Thr Cys Thr Gly Arg Gly Pro
                565                 570                 575
Asp Asn Cys Ile Gln Cys Ala His Tyr Ile Asp Gly Pro His Cys Val
            580                 585                 590
```

```
Lys Thr Cys Pro Ala Gly Val Met Gly Glu Asn Asn Thr Leu Val Trp
        595                 600                 605
Lys Tyr Ala Asp Ala Gly His Val Cys His Leu Cys His Pro Asn Cys
    610                 615                 620
Thr Tyr Gly Cys Thr Gly Pro Gly Leu Glu Gly Cys Pro Thr Asn Gly
625                 630                 635                 640
Pro Lys Ile Pro Ser Ile Ala Thr Gly Met Val Gly Ala Leu Leu Leu
                645                 650                 655
Leu Leu Val Val Ala Leu Gly Ile Gly Leu Phe Met Arg Arg Arg His
            660                 665                 670
Ile Val Arg Lys Arg Thr Leu Arg Arg Leu Leu Gln Glu Arg Glu Leu
        675                 680                 685
Val Glu Pro Leu Thr Pro Ser Gly Glu Ala Pro Asn Gln Ala Leu Leu
    690                 695                 700
Arg Ile Leu Lys Glu Thr Glu Phe Lys Lys Ile Lys Val Leu Gly Ser
705                 710                 715                 720
Gly Ala Phe Gly Thr Val Tyr Lys Gly Leu Trp Ile Pro Glu Gly Glu
                725                 730                 735
Lys Val Lys Ile Pro Val Ala Ile Lys Glu Leu Arg Glu Ala Thr Ser
            740                 745                 750
Pro Lys Ala Asn Lys Glu Ile Leu Asp Glu Ala Tyr Val Met Ala Ser
        755                 760                 765
Val Asp Asn Pro His Val Cys Arg Leu Leu Gly Ile Cys Leu Thr Ser
    770                 775                 780
Thr Val Gln Leu Ile Thr Gln Leu Met Pro Phe Gly Cys Leu Leu Asp
785                 790                 795                 800
Tyr Val Arg Glu His Lys Asp Asn Ile Gly Ser Gln Tyr Leu Leu Asn
                805                 810                 815
Trp Cys Val Gln Ile Ala Lys Gly Met Asn Tyr Leu Glu Asp Arg Arg
            820                 825                 830
Leu Val His Arg Asp Leu Ala Ala Arg Asn Val Leu Val Lys Thr Pro
        835                 840                 845
Gln His Val Lys Ile Thr Asp Phe Gly Leu Ala Lys Leu Leu Gly Ala
    850                 855                 860
Glu Glu Lys Glu Tyr His Ala Glu Gly Gly Lys Val Pro Ile Lys Trp
865                 870                 875                 880
Met Ala Leu Glu Ser Ile Leu His Arg Ile Tyr Thr His Gln Ser Asp
                885                 890                 895
Val Trp Ser Tyr Gly Val Thr Val Trp Glu Leu Met Thr Phe Gly Ser
            900                 905                 910
Lys Pro Tyr Asp Gly Ile Pro Ala Ser Glu Ile Ser Ser Ile Leu Glu
        915                 920                 925
Lys Gly Glu Arg Leu Pro Gln Pro Pro Ile Cys Thr Ile Asp Val Tyr
    930                 935                 940
Met Ile Met Val Lys Cys Trp Met Ile Asp Ala Asp Ser Arg Pro Lys
945                 950                 955                 960
Phe Arg Glu Leu Ile Ile Glu Phe Ser Lys Met Ala Arg Asp Pro Gln
                965                 970                 975
Arg Tyr Leu Val Ile Gln Gly Asp Glu Arg Met His Leu Pro Ser Pro
            980                 985                 990
Thr Asp Ser Asn Phe Tyr Arg Ala Leu Met Asp Glu Glu Asp Met Asp
        995                 1000                1005
Asp Val Val Asp Ala Asp Glu Tyr Leu Ile Pro Gln Gln Gly Phe Phe
    1010                1015                1020
Ser Ser Pro Ser Thr Ser Arg Thr Pro Leu Leu Ser Ser Leu Ser Ala
1025                1030                1035                1040
Thr Ser Asn Asn Ser Thr Val Ala Cys Ile Asp Arg Asn Gly Leu Gln
                1045                1050                1055
Ser Cys Pro Ile Lys Glu Asp Ser Phe Leu Gln Arg Tyr Ser Ser Asp
            1060                1065                1070
Pro Thr Gly Ala Leu Thr Glu Asp Ser Ile Asp Asp Thr Phe Leu Pro
        1075                1080                1085
Val Pro Glu Tyr Ile Asn Gln Ser Val Pro Lys Arg Pro Ala Gly Ser
    1090                1095                1100
Val Gln Asn Pro Val Tyr His Asn Gln Pro Leu Asn Pro Ala Pro Ser
```

```
1105                1110                1115                1120
Arg Asp Pro His Tyr Gln Asp Pro His Ser Thr Ala Val Gly Asn Pro
                1125                1130                1135
Glu Tyr Leu Asn Thr Val Gln Pro Thr Cys Val Asn Ser Thr Phe Asp
            1140                1145                1150
Ser Pro Ala His Trp Ala Gln Lys Gly Ser His Gln Ile Ser Leu Asp
        1155                1160                1165
Asn Pro Asp Tyr Gln Gln Asp Phe Phe Pro Lys Glu Ala Lys Pro Asn
    1170                1175                1180
Gly Ile Phe Lys Gly Ser Thr Ala Glu Asn Ala Glu Tyr Leu Arg Val
1185                1190                1195                1200
Ala Pro Gln Ser Ser Glu Phe Ile Gly Ala
                1205                1210
```

<210> 11
<211> 1239
<212> DNA
<213> Homo sapiens

<220>
<221> source
<222> 1..1239
<223> /mol_type="DNA"
/organism="Homo sapiens"

<400> 11

```
atgatggagg gtcactttcc ccagagcgat gtgataggcc aggttctgcc tgaagcaaca      60

actacagcat ttgaatatga agatgaagat ggtgatcgaa ttacagtgag aagtgatgag     120

gaaatgaagg caatgctgtc atattattat tccacagtaa tggaacagca agtaaatgga     180

cagttaatag agcctctgca gatatttcca agagcctgca agcctcctgg ggaacggaac     240

atacatggcc tgaaggtgaa tactcgggcc ggaccctctc aacacagcag cccagcagtc     300

tcagattcac ttccaagcaa tagcttaaag aagtcttctg ctgaactgaa aaaaatacta     360

gccaatggcc agatgaatga acaagacata cgatatcggg acactcttgg tcatggcaac     420

ggaggcacag tctacaaagc atatcatgtc ccgagtggga aaatattagc tgtaaaggtc     480

atactactag atattacact ggaacttcag aagcaaatta tgtctgaatt ggaaattctt     540

tataagtgcg attcatcata tatcattgga ttttatggag catttttgt agaaaacagg      600

atttcaatat gtacagaatt catggatggg ggatctttgg atgtatatag gaaaatgcca     660

gaacatgtcc ttggaagaat tgcagtagca gttgttaaag gccttactta tttgtggagt     720

ttaaagattt tacatagaga cgtgaagccc tccaatatgc tagtaaacac aagaggacag     780

gttaagctgt gtgattttgg agttagcact cagctggtga attctatagc caagacgtat     840

gttggaacaa atgcttatat ggcgcctgaa aggatttcag gggagcagta tggaattcat     900

tctgatgtct ggagcttagg aatctctttt atggagcttg ctcttgggag gtttccatat     960

cctcagattc agaaaaacca gggatcttta atgcctctcc agcttctgca gtgcattgtt    1020

gatgaggatt cgcccgtcct tccagttgga gagttctcgg agccatttgt acatttcatc    1080

actcagtgta tgcgaaaaca gccaaaagaa aggccagcac ctgaagaatt gatgggccac    1140

ccgttcatcg tgcagttcaa tgatggaaat gccgccgtgg tgtccatgtg ggtgtgccgg    1200
```

gcgctggagg agaggcggag ccagcagggg cccccgtga 1239

<210> 12
<211> 412
<212> PRT
<213> Homo sapiens

<220>
<221> SOURCE
<222> 1..412
<223> /mol_type="protein"
/organism="Homo sapiens"

<400> 12
```
Met Met Glu Gly His Phe Pro Gln Ser Asp Val Ile Gly Gln Val Leu
1               5                   10                  15
Pro Glu Ala Thr Thr Thr Ala Phe Glu Tyr Glu Asp Glu Asp Gly Asp
            20                  25                  30
Arg Ile Thr Val Arg Ser Asp Glu Glu Met Lys Ala Met Leu Ser Tyr
        35                  40                  45
Tyr Tyr Ser Thr Val Met Glu Gln Gln Val Asn Gly Gln Leu Ile Glu
    50                  55                  60
Pro Leu Gln Ile Phe Pro Arg Ala Cys Lys Pro Pro Gly Glu Arg Asn
65                  70                  75                  80
Ile His Gly Leu Lys Val Asn Thr Arg Ala Gly Pro Ser Gln His Ser
                85                  90                  95
Ser Pro Ala Val Ser Asp Ser Leu Pro Ser Asn Ser Leu Lys Lys Ser
            100                 105                 110
Ser Ala Glu Leu Lys Lys Ile Leu Ala Asn Gly Gln Met Asn Glu Gln
        115                 120                 125
Asp Ile Arg Tyr Arg Asp Thr Leu Gly His Gly Asn Gly Gly Thr Val
    130                 135                 140
Tyr Lys Ala Tyr His Val Pro Ser Gly Lys Ile Leu Ala Val Lys Val
145                 150                 155                 160
Ile Leu Leu Asp Ile Thr Leu Glu Leu Gln Lys Gln Ile Met Ser Glu
                165                 170                 175
Leu Glu Ile Leu Tyr Lys Cys Asp Ser Ser Tyr Ile Ile Gly Phe Tyr
            180                 185                 190
Gly Ala Phe Phe Val Glu Asn Arg Ile Ser Ile Cys Thr Glu Phe Met
        195                 200                 205
Asp Gly Gly Ser Leu Asp Val Tyr Arg Lys Met Pro Glu His Val Leu
    210                 215                 220
Gly Arg Ile Ala Val Ala Val Val Lys Gly Leu Thr Tyr Leu Trp Ser
225                 230                 235                 240
Leu Lys Ile Leu His Arg Asp Val Lys Pro Ser Asn Met Leu Val Asn
                245                 250                 255
Thr Arg Gly Gln Val Lys Leu Cys Asp Phe Gly Val Ser Thr Gln Leu
            260                 265                 270
Val Asn Ser Ile Ala Lys Thr Tyr Val Gly Thr Asn Ala Tyr Met Ala
        275                 280                 285
Pro Glu Arg Ile Ser Gly Glu Gln Tyr Gly Ile His Ser Asp Val Trp
    290                 295                 300
Ser Leu Gly Ile Ser Phe Met Glu Leu Ala Leu Gly Arg Phe Pro Tyr
305                 310                 315                 320
Pro Gln Ile Gln Lys Asn Gln Gly Ser Leu Met Pro Leu Gln Leu Leu
                325                 330                 335
Gln Cys Ile Val Asp Glu Asp Ser Pro Val Leu Pro Val Gly Glu Phe
            340                 345                 350
Ser Glu Pro Phe Val His Phe Ile Thr Gln Cys Met Arg Lys Gln Pro
        355                 360                 365
Lys Glu Arg Pro Ala Pro Glu Glu Leu Met Gly His Pro Phe Ile Val
    370                 375                 380
Gln Phe Asn Asp Gly Asn Ala Ala Val Val Ser Met Trp Val Cys Arg
```

```
385                     390                     395                     400
Ala Leu Glu Glu Arg Arg Ser Gln Gln Gly Pro Pro
                    405                     410
```